(19) Europäisches Patentamt
European Patent Office
Office européen des brevets

(11) EP 1 814 894 B1

(12) **EUROPÄISCHE PATENTSCHRIFT**

(45) Veröffentlichungstag und Bekanntmachung des Hinweises auf die Patenterteilung:
**08.08.2012 Patentblatt 2012/32**

(21) Anmeldenummer: **05808134.0**

(22) Anmeldetag: **04.11.2005**

(51) Int Cl.:
***C07H 17/07*** *(2006.01)*

(86) Internationale Anmeldenummer:
**PCT/EP2005/011830**

(87) Internationale Veröffentlichungsnummer:
**WO 2006/056308 (01.06.2006 Gazette 2006/22)**

(54) **FLAVONOID-KOMPLEXE MIT CYCLODEXTRINEN**

FLAVONOID COMPLEXES WITH CYCLODEXTRINS

COMPLEXES FLAVONOIDES AVEC CYCLODEXTRINES

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **25.11.2004 DE 102004056899**

(43) Veröffentlichungstag der Anmeldung:
**08.08.2007 Patentblatt 2007/32**

(73) Patentinhaber: **Merck Patent GmbH
64293 Darmstadt (DE)**

(72) Erfinder:
- **CAROLA, Christophe
69120 Heidelberg (DE)**
- **TOULLEC, Anne
92160 Antony (FR)**
- **BUCHHOLZ, Herwig
60599 Frankfurt (DE)**

(56) Entgegenhaltungen:
**EP-A- 1 400 579** **WO-A-02/069926**
**WO-A-2005/068484**

- **BUSCHMANN H-J ET AL: "APPLICATIONS OF CYCLODEXTRINS IN COSMETIC PRODUCTS: A REVIEW" JOURNAL OF COSMETIC SCIENCE, SOCIETY OF COSMETIC CHEMISTS, NEW YORK, NY,, US, Bd. 53, Nr. 3, Mai 2002 (2002-05), Seiten 185-191, XP008013888 ISSN: 1525-7886**
- **LOFTSSON T ET AL: "CYLODEXTRINS IN TOPICAL DRUG FORMULATONS: THEORY AND PRACTICE" INTERNATIONAL JOURNAL OF PHARMACEUTICS, AMSTERDAM, NL, Bd. 225, Nr. 1/2, August 2001 (2001-08), Seiten 15-30, XP001023910 ISSN: 0378-5173**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 814 894 B1

## Beschreibung

Die Erfindung betrifft Komplexe bestimmter Flavonoid-Derivate, Zubereitungen, die solche Komplexe enthalten, entsprechende Verfahren zur Herstellung der Komplexe bzw. der diese enthaltenden Zubereitungen und deren Verwendung, insbesondere zur Pflege, Konservierung oder Verbesserung des allgemeinen Zustandes der Haut oder Haare.

Aufgabe pflegender Kosmetik ist es, nach Möglichkeit den Eindruck einer jugendlichen Haut zu erhalten. Prinzipiell stehen verschiedene Wege offen, um diesen Weg zu erreichen. So können bereits vorhandene Schädigungen der Haut, wie unregelmäßige Pigmentierung oder Faltenbildung, durch abdeckende Puder oder Cremes ausgeglichen werden. Ein anderer Ansatzpunkt ist, die Haut vor Umwelteinflüssen zu schützen, die zu einer dauerhaften Schädigung und damit Alterung der Haut führen. Die Idee ist also, vorbeugend einzugreifen und dadurch den Alterungsprozess hinauszuzögern. Ein Beispiel sind hierfür die bereits erwähnten UV-Filter, welche durch Absorption bestimmter Wellenlängenbereiche eine Schädigung der Haut vermeiden oder zumindest vermindern. Während bei UV-Filtern das schädigende Ereignis, die UV-Strahlung, von der Haut abgeschirmt wird, versucht man bei einem weiteren Weg, die natürlichen Abwehr- bzw. Reparaturmechanismen der Haut gegen das schädigende Ereignis zu unterstützen. Schließlich verfolgt man als weiteren Ansatzpunkt die mit zunehmendem Alter sich abschwächenden Abwehrfunktionen der Haut gegen schädigende Einflüsse auszugleichen, indem Substanzen von außen zugeführt werden, die diese nachlassende Abwehr- bzw. Reparaturfunktion ersetzen können. Beispielsweise besitzt die Haut die Fähigkeit, Radikale, die durch äußere oder innere Stressfaktoren erzeugt werden, abzufangen. Diese Fähigkeit schwächt sich mit zunehmendem Alter ab, wodurch sich der Alterungsprozess mit zunehmendem Alter beschleunigt.

Eine mehr oder minder stark ausgeprägte Sonnenbräune der Haut gilt in der modernen Gesellschaft als attraktiv und als Ausdruck von Dynamik und Sportlichkeit. Neben dieser erwünschten Wirkung der Sonne auf die Haut treten eine Reihe von unerwünschten Nebenwirkungen auf, wie Sonnenbrand oder vorzeitige Hautalterung und Faltenbildung. Von besonderer Bedeutung ist dabei der Wellenlängenbereich von 280 bis 400 nm. Dieser Bereich umfaßt UV-B-Strahlen mit einer Wellenlänge zwischen 280 und 320 nm, die bei der Bildung eines Sonnenerythems eine entscheidende Rollen spielen, wie auch UV-A-Strahlen, mit einer Wellenlänge zwischen 320 und 400 nm, welche die Haut bräunen aber auch altern lassen, die Auslösung einer erythematösen Reaktion begünstigen oder diese Reaktion bei bestimmten Menschen vergrößern oder sogar phytotoxische oder photoallergische und irritative Reaktionen auslösen können.

Hautschädigungen werden nicht nur durch Sonnenlicht verursacht, sondern auch durch andere äußere Einflüsse, wie Kälte oder Wärme. Ferner unterliegt die Haut einer natürlichen Alterung, wodurch Falten entstehen und die Spannkraft der Haut nachläßt.

Eine weitere Schwierigkeit bei der Herstellung von Kosmetika besteht darin, dass Wirkstoffe, die in kosmetische Zubereitungen eingearbeitet werden sollen, oftmals nicht stabil sind und in der Zubereitung geschädigt werden können. Die Schädigungen können beispielsweise durch eine Reaktion mit Luftsauerstoff oder durch die Absorption von UV-Strahlen verursacht werden. Die so geschädigten Moleküle können durch ihre Strukturänderung z.B. ihre Farbe ändern und/oder ihre Wirksamkeit verlieren.

Ein bekannter Weg, die beschriebenen Probleme zu behandeln besteht im Zusatz von Antioxidantien zu den Zubereitungen.

Laut CD Römpp Chemie Lexikon - Version 1.0, Stuttgart/New York: Georg Thieme Verlag 1995 handelt es sich bei Antioxidantien um Verbindungen, die unerwünschte, durch Sauerstoff-Einwirkungen u.a. oxidative Prozesse bedingte Veränderungen in den zu schützenden Stoffen hemmen oder verhindern. Einsatzgebiete sind z.B. in Kunststoffen und Kautschuk zum Schutz gegen Alterung; in Fetten zum Schutz vor Ranzigkeit, in Ölen, Viehfutter, Autobenzin und Düsentreibstoffen zum Schutz gegen Verharzung, in Transformatoren- und Turbinenöl gegen Schlammbildung, in Aromastoffen gegen Geruchsverschlechterung. Als Antioxidantien wirksam sind u.a. durch sterisch hindernde Gruppen substituierte Phenole, Hydrochinone, Brenzcatechine und Aromat. Amine sowie deren MetallKomplexe. Die Wirkung der Antioxidantien besteht laut Römpp meist darin, daß sie als Radikalfänger für die bei der Autoxidation auftretenden freien Radikale wirken.

Unter den Phenolen mit antioxidativer Wirkung sind die teilweise als Naturstoffe vorkommenden Polyphenole für Anwendungen im pharmazeutischen, kosmetischen oder Ernährungsbereich besonders interessant. Beispielsweise weisen die hauptsächlich als Pflanzenfarbstoffe bekannten Flavonoide oder Bioflavonoide häufig ein antioxidantes Potential auf. Mit Effekten des Substitutionsmusters von Mono- und Dihydoxyflavonen beschäftigen sich K. Lemanska, H. Szymusiak, B. Tyrakowska, R. Zielinski, I.M.C.M. Rietjens; Current Topics in Biophysics 2000, 24(2), 101-108. Es wird dort beobachtet, dass Dihydroxyflavone mit einer OH-Gruppe benachbart zur Ketofunktion oder OH-Gruppen in 3'4'- oder 6,7- oder 7,8-Position antioxidative Eigenschaften aufweisen, während andere Mono- und Dihydroxyflavone teilweise keine antioxidativen Eigenschaften aufweisen.

Häufig wird Quercetin (Cyanidanol, Cyanidenolon 1522, Meletin, Sophoretin, Ericin, 3,3',4',5,7-Pentahydroxyflavon) als besonders wirksames Antioxidans genannt (z.B. C.A. Rice-Evans, N.J. Miller, G. Paganga, Trends in Plant Science 1997, 2(4), 152-159). K. Lemanska, H. Szymusiak, B. Tyrakowska, R. Zielinski, A.E.M.F. Soffers, I.M.C.M. Rietjens; Free Radical Biology&Medicine 2001, 31(7), 869-881 untersuchen die pH-Abhängigkeit der antioxidanten

Wirkung von Hydoxyflavonen. Über den gesamten pH-Bereich zeigt Quercetin die höchste Aktivität der untersuchten Strukturen.

In der DE 197 55 504 A1 wird die Verwendung von Flavonen und Flavonoiden gegen die UV-induzierte Zersetzung von Dibenzoylmethan und dessen Derivaten beschrieben.

In der WO 02/00214 für die Verwendung von bestimmten Flavon-Derivaten zur Herstellung von oralen Arzneimitteln zur systemischen Behandlung und Prophylaxe von UV-induzierten Dermatosen, insbesondere der polymorphen Lichtdermatosen und ihren Unterformen und/oder unerwünschten Langzeitfolgen von UV-Bestrahlung, besonders der Lichtalterung, beschrieben. Bevorzugte Flavon-Derivate sind dabei insbesondere natürlich vorkommende Bioflavonoide, wie Rutin, Naringin, Naringenin, Hesperidin, Hesperetin, Taxifolin etc. sowie Derivate davon, wie Troxerutin oder Monoxerutin.

In der internationalen Patentanmeldung WO 00/61095 werden Mischungen von Polyphenolen mit Vitaminen beschrieben. Diese Mischungen eignen sich zum Einsatz in kosmetischen oder dermatologischen Zusammensetzungen und sind optimiert zum Fangen freier Radikale wie Hydroxy-Radikale oder Peroxiden. Insbesondere bevorzugt ist dabei die Kombination von Troxerutin mit $\alpha$-Tocopherolsuccinat und Ascorbyl-palmitat.

In der EP-A-1400579 wird beschrieben, dass bestimmte Flavonoide sich hervorragend als Antioxdantien eignen. Es werden Zubereitung mit antioxidanten Eigenschaften, enthaltend zumindest eine Verbindung der Formel

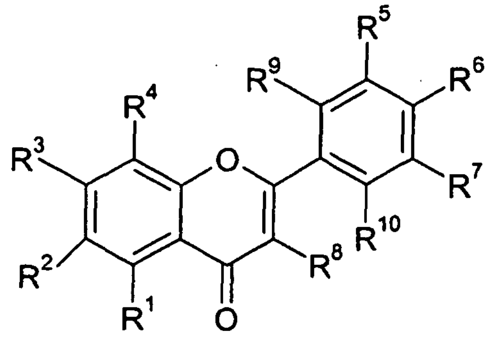

wobei $R^1$ bis $R^{10}$ gleich oder verschieden sein können und ausgewählt sind aus

- H
- $OR^{11}$
- geradkettigen oder verzweigten $C_1$- bis $C_{20}$-Alkylgruppen,
- geradkettigen oder verzweigten $C_3$- bis $C_{20}$-Alkenylgruppen,
- geradkettigen oder verzweigten $C_1$- bis $C_{20}$-Hydroxyalkylgruppen, wobei die Hydroxygruppe an ein primäres oder sekundäres Kohlenstoffatom der Kette gebunden sein kann und weiter die Alkylkette auch durch Sauerstoff unterbrochen sein kann, und/oder
- $C_3$- bis $C_{10}$-Cycloalkylgruppen und/oder $C_3$- bis $C_{12}$-Cycloalkenylgruppen, wobei die Ringe jeweils auch durch $-(CH_2)_n$-Gruppen mit n = 1 bis 3 überbrückt sein können,
- wobei alle $OR^{11}$ unabhängig voneinander stehen für
  - OH
  - geradkettige oder verzweigte $C_1$- bis $C_{20}$-Alkyloxygruppen,
  - geradkettigen oder verzweigten $C_3$- bis $C_{20}$-Alkenyloxygruppen,
  - geradkettigen oder verzweigten $C_1$- bis $C_{20}$-Hydroxyalkoxygruppen, wobei die Hydroxygruppe(n) an ein primäre oder sekundäre Kohlenstoffatome der Kette gebunden sein können und weiter die Alkylkette auch durch Sauerstoff unterbrochen sein kann, und/oder
  - $C_3$- bis $C_{10}$-Cycloalkyloxygruppen und/oder $C_3$- bis $C_{12}$-Cycloalkenyloxygruppen, wobei die Ringe jeweils auch durch $-(CH_2)_n$-Gruppen mit n = 1 bis 3 überbrückt sein können und/oder,
  - Mono- und/oder Oligoglycosylreste,

  mit der Maßgabe, dass mindestens 4 Reste aus $R^1$ bis $R^7$ stehen für OH und dass im Molekül mindestens 2 Paare benachbarter Gruppen -OH vorliegen,
- oder $R^2$, $R^5$ und $R^6$ für OH und die Reste $R^1$, $R^3$, $R^4$ und $R^{7-10}$ für H stehen, angegeben.

Es besteht jedoch weiterhin Bedarf nach hautverträglichen Antioxidantien, die sich auch zum Einsatz in hautpflegenden Zubereitungen eignen. Insbesondere besteht Bedarf nach wasserlöslichen Verbindungen, welche die genannten Anforderungen erfüllen.

Aufgabe der Erfindung ist es daher, eine Verbindungen bzw. Zusammensetzung zur Verfügung zu stellen, welche eine schützende Wirkung gegen UV-Strahlen aufweist und/oder eine schützende Wirkung gegen oxidativen Stress auf Körperzellen ausübt und/oder einer Alterung der Haut entgegenwirkt.

Ein erster Gegenstand der vorliegenden Erfindung sind daher Komplex-Verbindungen der Formel I

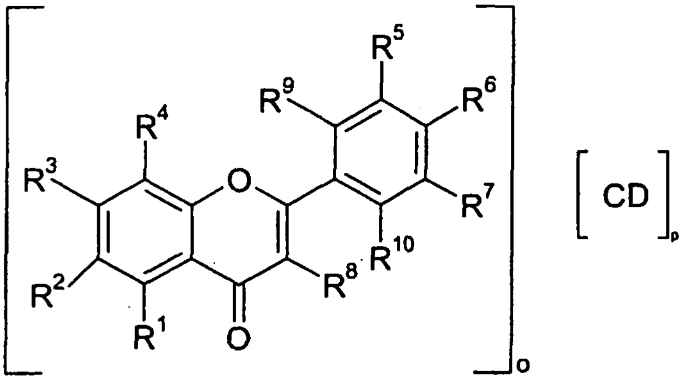

I

wobei $R^1$ bis $R^{10}$ gleich oder verschieden sein können und ausgewählt sind aus

- H
- $OR^{11}$
- geradkettigen oder verzweigten $C_1$- bis $C_{20}$-Alkylgruppen,
- geradkettigen oder verzweigten $C_3$- bis $C_{20}$-Alkenylgruppen,
- geradkettigen oder verzweigten $C_1$- bis $C_{20}$-Hydroxyalkylgruppen, wobei die Hydroxygruppe an ein primäres oder sekundäres Kohlenstoffatom der Kette gebunden sein kann und weiter die Alkylkette auch durch Sauerstoff unterbrochen sein kann, und/oder
- $C_3$- bis $C_{10}$-Cycloalkylgruppen und/oder $C_3$- bis $C_{12}$-Cycloalkenylgruppen, wobei die Ringe jeweils auch durch $-(CH_2)_n$-Gruppen mit n = 1 bis 3 überbrückt sein können,
- wobei alle $OR^{11}$ unabhängig voneinander stehen für

  - OH
  - geradkettige oder verzweigte $C_1$- bis $C_{20}$-Alkyloxygruppen,
  - geradkettigen oder verzweigten $C_3$- bis $C_{20}$-Alkenyloxygruppen,
  - geradkettigen oder verzweigten $C_1$- bis $C_{20}$-Hydroxyalkoxygruppen, wobei die Hydroxygruppe(n) an ein primäre oder sekundäre Kohlenstoffatome der Kette gebunden sein können und weiter die Alkylkette auch durch Sauerstoff unterbrochen sein kann, und/oder
  - $C_3$- bis $C_{10}$-Cycloalkyloxygruppen und/oder $C_3$- bis $C_{12}$-Cycloalkenyloxygruppen, wobei die Ringe jeweils auch durch $-(CH_2)_n$-Gruppen mit n = 1 bis 3 überbrückt sein können und/oder,
  - Mono- und/oder Oligoglycosylreste, wobei Mono - und Oligoglycosylreste für $R^8$ ausgeschlossen sind,

CD steht für ein Cyclodextrin-Molekül
o steht für die Zahl 1 und
p steht für eine Zahl aus dem Bereich 0,5 bis 50.

mit der Maßgabe, dass mindestens 2 Reste aus $R^1$ bis $R^7$ stehen für OH und dass im Molekül mindestens 1 Paar benachbarter Gruppen -OH vorliegt.

Ein zweiter Gegenstand der vorliegenden Erfindung sind Zubereitungen enthaltend einen geeigneten Träger, die dadurch gekennzeichnet, sind dass die Zubereitung

- 0,005 bis 99 Gew.-% einer Komplex-Verbindung nach Formel I enthält oder die Zubereitung
- 0,002 bis 70 Gew.-% Cyclodextrin und
  0,001 bis 60 Gew.-% mindestens einer Verbindung der Formel II

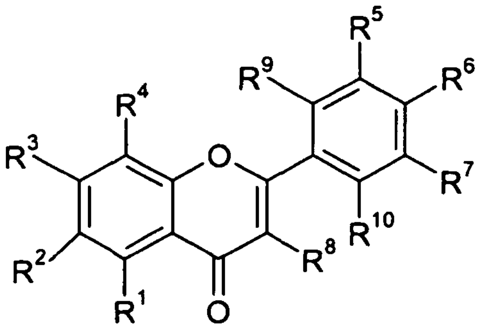

II

wobei $R^1$ bis $R^{10}$ gleich oder verschieden sein können und ausgewählt sind aus

- H
- $OR^{11}$
- geradkettigen oder verzweigten $C_1$- bis $C_{20}$-Alkylgruppen,
- geradkettigen oder verzweigten $C_3$- bis $C_{20}$-Alkenylgruppen,
- geradkettigen oder verzweigten $C_1$- bis $C_{20}$-Hydroxyalkylgruppen, wobei die Hydroxygruppe an ein primäres oder sekundäres Kohlenstoffatom der Kette gebunden sein kann und weiter die Alkylkette auch durch Sauerstoff unterbrochen sein kann, und/oder
- $C_3$- bis $C_{10}$-Cycloalkylgruppen und/oder $C_3$- bis $C_{12}$-Cycloalkenylgruppen, wobei die Ringe jeweils auch durch $-(CH_2)_n$-Gruppen mit n = 1 bis 3 überbrückt sein können,
- wobei alle $OR^{11}$ unabhängig voneinander stehen für

  - OH
  - geradkettige oder verzweigte $C_1$- bis $C_{20}$-Alkyloxygruppen,
  - geradkettigen oder verzweigten $C_3$- bis $C_{20}$-Alkenyloxygruppen,
  - geradkettigen oder verzweigten $C_1$- bis $C_{20}$-Hydroxyalkoxygruppen, wobei die Hydroxygruppe(n) an ein primäre oder sekundäre Kohlenstoffatome der Kette gebunden sein können und weiter die Alkylkette auch durch Sauerstoff unterbrochen sein kann, und/oder
  - $C_3$- bis $C_{10}$-Cycloalkyloxygruppen und/oder $C_3$- bis $C_{12}$-Cycloalkenyloxygruppen, wobei die Ringe jeweils auch durch $-(CH_2)_n$-Gruppen mit n = 1 bis 3 überbrückt sein können und/oder,
  - Mono- und/oder Oligoglycosylreste, wobei Mono - und Oligoglycosylreste für $R^8$ aus- geschlossen sind,

mit der Maßgabe, dass mindestens 2 Reste aus $R^1$ bis $R^7$ stehen für OH und dass im Molekül mindestens 1 Paar benachbarter Gruppen -OH vorliegt, oder ihrer topisch verträglicher Salze und/oder Derivate enthält.

Vorteile der erfindungsgemäßen Verbindungen bzw. Zusammensetzungen sind dabei insbesondere die antioxidante Wirkung und die gute Hautverträglichkeit. Zusätzlich sind bevorzugte der hier beschriebenen Verbindungen farblos oder nur schwach gefärbt und führen so nicht oder nur in geringer Weise zu Verfärbungen der Zubereitungen. Von Vorteil ist insbesondere das besondere Wirkprofil der erfindungsgemäßen Verbindungen, welches sich im DPPH-Assay (siehe unten) in einer hohen Kapazität Radikale zu fangen ($EC_{50}$), einer zeitverzögerten Wirkung ($T_{EC50}$ > 120 min) und damit einer mittleren bis hohen antiradikalischen Effizienz (AE) äußert. Zudem vereinigen die Verbindungen nach Formel I im Molekül antioxidative Eigenschaften mit UV-Absorption im UV-A- und/oder -B-Bereich bei gleichzeitig hervorragender Wasserlöslichkeit.

Ein Gegenstand der vorliegenden Erfindung ist daher auch die Verwendung der Verbindungen gemäß Formel I, wie oben angegeben, als Antioxidationsmittel mit langanhaltender Wirkung bzw. zur Herstellung einer Zubereitung mit antioxidanten Eigenschaften.

Bei den erfindungsgemäßen Zubereitungen handelt es sich dabei üblicherweise entweder um topisch anwendbare Zubereitungen, beispielsweise kosmetische oder dermatologische Formulierungen, oder um Arzneimittel bzw. Nahrungsmittel bzw. Nahrungsergänzungsmittel oder oral anzuwendende Kosmetika. Die Zubereitungen enthalten einen kosmetisch oder dermatologisch oder pharmazeutisch oder Nahrungsmittel-geeigneten Träger und je nach gewünschtem Eigenschaftsprofil optional weitere geeignete Inhaltsstoffe.

In einer bevorzugten Ausführungsform der vorliegenden Erfindung handelt es sich bei den Zubereitungen um sprühbare Zubereitungen. Insbesondere vorteilhaft kann es dabei sein, wenn diese Zubereitungen auf wässriger oder wässrig alkoholischer Trägerbasis aufgebaut sind.

Bevorzugt kommen dabei Aerosole zum Einsatz. Ein Aerosol ist ein disperses System, bei dem ein Feststoff oder eine Flüssigkeit in einem Gas äußerst fein verteilt vorliegt. Das Aerosol wird in der Regel erst bei der Anwendung

mit Hilfe eines geeigneten Sprühsystems durch Versprühen von Lösungen, Emulsionen oder Suspensionen selbst erzeugt, wozu beispielsweise Sprühdosen verwendet werden können, in denen ein verflüssigtes Druckgas als Treibgas dient. Beim Öffnen des Druckventils entweicht das Treibmittel- Zubereitungsgemisch durch eine feine Düse, das Treibmittel verdampft und hinterläßt das fein verteilte Sprühgut als Aerosol.
Wirkstoffe können in Aerosolformulierungen sowohl gelöst als auch in fester Form vorliegen; liegen sie in fester Form vor, müssen sie allerdings im Treibmittelsystem entsprechend suspendiert werden.
Als Aerosol versprühbare kosmetische und dermatologische Hautpflegezubereitungen auf Emulsionsbasis sind in der Regel O/W-Systeme, in denen hydrophile Wirkstoffe in der externen Wasserphase gelöst sind. Die Ölphase enthält häufig silikonhaltige Öle, welche zu einem angenehmen Hautgefühl nach dem Versprühen beitragen.

Als Treibgase können dabei hydrophile Treibgase - wie z. B. Kohlendioxid - oder lipophile Treibmittel wie beispielsweise Kohlenwasserstoffe eingesetzt, werden. Andere bevorzugte Zubereitungen sind Pumpsprays, bei denen das Produkt in eine Zerstäuberflasche abgefüllt wird und durch mechanisches Ausbringen zerstäubt wird.

Geeignete sprühbare W/O-Emulsionen sind beispielsweise die in den Schriften DE-10162844-A1, DE-10162842-A1, DE-10162841-A1, DE-10162840-A1 oder DE-10048683-A1 offenbart.

Geeignet sind auch bei Raumtemperatur als Aerosole versprühbare W/O-Emulsionen, wie sie in WO2004030641 beschrieben sind, Derartige Emulsionen enthalten eine Fettphase, welche mindestens 90 Gew.-% bei Raumtemperatur flüssige Ölkomponenten und höchstens 4 Gew.% an Substanzen, gewählt aus der Gruppe der C3- bis C4-Ester von C12- bis C18-Alkansäuren, C8- bis C12-Alkanole und Silikonöle, enthält, sowie 20 bis 85 Gew.-% - bezogen auf das Gesamtgewicht der Zubereitung - Wasser, und einen oder mehrere W/O-Emulgatoren und ein oder mehrere lipophile Treibgase.

Besonders bevorzugt ist es, wenn der W/O-Emulgator oder die W/O-Emulgatoren gewählt werden aus der Gruppe PEG-30 Dipolyhydroxystearat, Decaglycerylheptaoleat, PolyOlyceryl-3-Diisostearat, PEG-8 Distearat, Diglycerin Dipolyhydroxystearat, Glycerinisostearat, Sorbitanisostearat, Polyglyceryl-3 Methylglucosedistearat, Fettalkohole mit 8 bis 30 Kohlenstoffatomen, Oligo- oder Polyglycerinether, Cetyl Dimethicon Copolyole, Alkyl Methicon Copolyole, Alkyl Dimethicon Ethoxy Glucoside, W/O- Emulgatoren, die zusätzlich (poly 5)ethoxyliert und/oder (poly-) propoxyliert sind, beispielsweise polyethoxyliertes hydrogeniertes oder nichthydrogeniertes Ricinusöl, ethoxyliertes Cholesterin, ethoxlierte Fettalkohole wie Steareth-2, ethoxlierte Fettsäuren wie PEG-2 Stearat, PEG-40 Sorbitanperisostearat. Bevorzugt ist es, wenn der oder die W/O-Emulgatoren gewählt werden aus der Gruppe PEG-30 Dipolyhydroxystearat, Polyglyceryl-3-Diisostearat (= Polyglycerin-3-Diisostearat), Diglycerin Dipolyhydroxystearat, Glycerinisostearat, Cetyl PEG/PPG-10/1 Dimethicon, Sorbitanisostearat, Polyglyceryl-3 Methylglucosedistearat, Steareth-2, PEG-2 Stearat, Sorbitanstearat, Cetylalkotol, Stearylalkohol und/oder Cetearylalkohol. Ganz besonders bevorzugt ist es, wenn Kombinationen der oben genannten W/O- Emulgatoren, insbesondere eine Kombination aus zwei Emulgatoren, eingesetzt werden

Der verwendete W/O-Emulgator bzw. die erfindungsgemäß verwendeten W/O-Emulgatoren liegt bzw. liegen vorteilhaft in Konzentrationen von 0,5 bis 8 Gew.-% (bezogen auf das Gesamtgewicht der Zubereitung) vor, wobei es allerdings möglich und vorteilhaft ist, den Gehalt an Emulgatoren niedrig zu halten, etwa bis 5 Gew.-%, jeweils bezogen auf das Gesamtgewicht der Zusammensetzung. Ferner kann es vorteilhaft sein, die Emulgatoren so zu wählen, dass Kombinationen aus W/O- und O/W-Emulgatoren zum Einsatz kommen.
Es kann vorteilhaft sein, wenn die Zubereitungen darüber hinaus Stabilisatoren enthalten. Bevorzugt wird als Stabilisator das PEG-45 /Dodecylglycolcopolymer undioder das PEG 22 / Dodecylglycolcopolymer und/oder das Methoxy PEG-22/Dodecyl Glycol Copolymer 10 verwendet. Ferner können auch Poloxamere des Pluronic-Typs bevorzugt werden. Der Stabilisator bzw. die Stabilisatoren liegen vorteilhaft in Konzentrationen von O bis 8 Gew.-% vor, wobei es allerdings möglich und vorteilhaft ist, den Gehalt an Stabilis toren niedrig zu halten, etwa bis 5 Gew.-%, jeweils bezogen auf das Gesamtgewicht der Zusammensetzung. Es ist ganz besonders vorteilhaft, Stabilisatoren zu verwenden, wenn der pH-Wert der Zubereitungen im sauren Bereich liegt. Ganz besonders bevorzugt ist es, wenn Kombinationen aus den oben genannten W/O Emulgatoren und eines Stabilisators eingesetzt werden.
Wenn die erfindungsgemäßen Zubereitungen UV-Filtersubstanzen enthalten, ist es vorteilhaft, wenn die Ölphase Butylenglykol-Derivate (wie beispielsweise Butylenglykol Di caprylat), Triglyceride (wie beispielsweise Capryl-Caprinsäure-Triglycerid [caprylic/capric triglycerid]), C2-C5 Alkylbenzoat und/oder Silikonöle enthält bzw. gänzlich aus solchen Ölen besteht.

Die Wassermenge kann bis zu etwa 85 Gew.-% betragen, bezogen auf das Gesamtgewicht der Zubereitungen, wobei üblicherweise optimale Wassergehalte im Bereich zwischen 50 und 80 Gew.-% gewählt werden.

Die erfindungsgemäßen sprühbaren Zubereitungen zeigen sehr gute sensorische Eigenschaften, wie beispielsweise die Verteilbarkeit auf der Haut oder das Einzugsvermögen in die Haut, und zeichnen sich darüberhinaus durch eine überdurchschnittlich gute Hautpflege und einen angenehmen Kühleffekt aus.

Cyclodextrine sind aus 6, 7, 8 oder noch mehr $\alpha$-1,4-verknüpften Glucoseeinheiten aufgebaut, wobei die Cyclohexaamylose (alpha- oder $\alpha$-Cyclodextrin) sich durch die Struktur

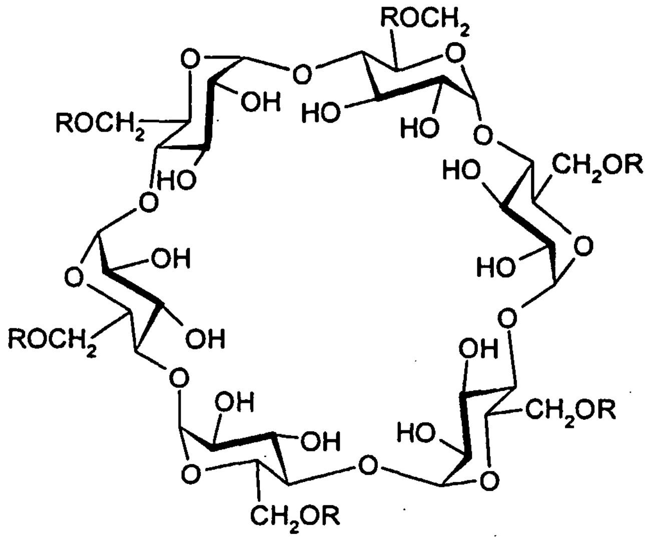

auszeichnet. Die Cycloheptaamylose (beta- oder β-Cyclodextrin) zeichnet sich durch die Struktur

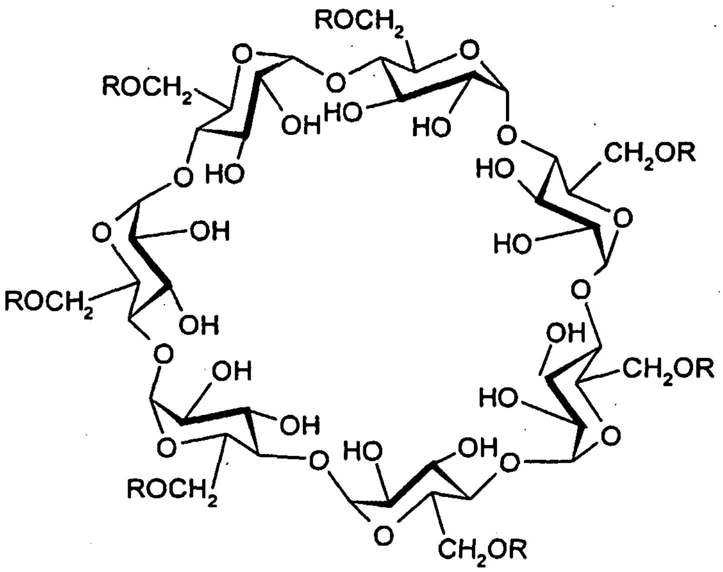

aus. Die Cyclooctaamylose (gamma- oder γ-Cyclodextrin) zeichnet sich durch die Struktur

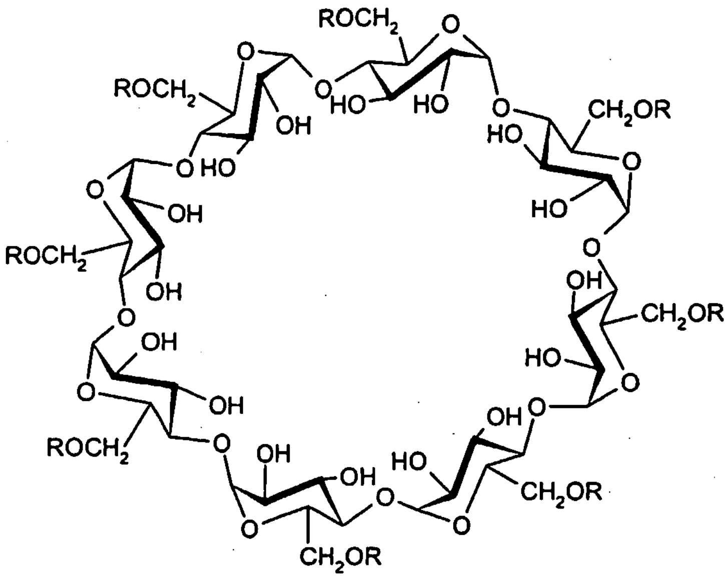

aus. Die Cycloenneaamylose (delta- oder δ-Cyclodextrin) zeichnet sich durch die Struktur

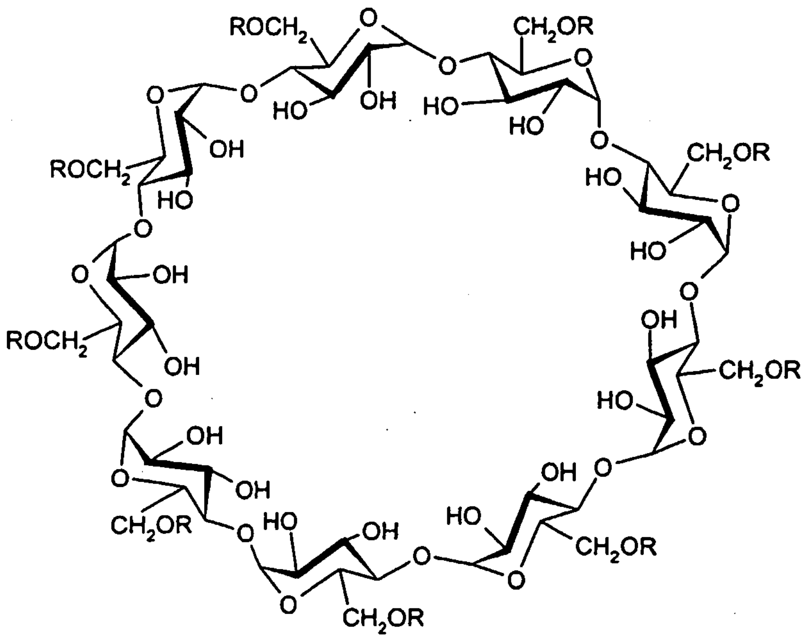

aus. Cyclodextrine können in der underivatisierten Form (R = H) oder auch in derivatisierter Form, z. B. in der Position R alkoxyliert, Hydroxyalkyliert oder alkyliert, insbesondere propoxyliert oder methyliert vorliegen.

Bioflavonoid-Cyclodextrin-Komplexe sind dabei im Prinzip bekannt:

- K. Miyake, H. Arima et al. (Pharm. Dev. Techn. 5(3) 2000, 399-407 beschreiben 1:1 - Komplexe von Rutin mit beta-Cyclodextrinen und deren Löslichkeits- bzw. Freisetzungsverhalten. Dabei zeigte sich, dass der beta-Cyclodextrin-Komplex und der 2-Hydroxypropyl-beta-cyclodextrin-Komplex besonders stabil sind. Alpha- und gamma-Cyclodextrin-Komplexe sind nach dieser Publikation generell schlechter zur Komplexierung von Rutin geeignet als beta-cyclodextrin-Komplexe.
- T. K. Nguyen, H. Galons, C. Chemtob, Congr. Int. Technol. Pharm., 6th (1992), Vol 5, 408-16408-416 beschreiben ebenfalls verschiedene Cyclodextrin-Komplexe von Rutin. Als besonders gut löslich erweist sich hier der 2,6-Di-O-methyl-beta-cyclodextrin-Komplex., wobei Komplexe mit Molverhältnissen Rutin:Cyclodextrin von 1:1 und 1:2 beschrieben werden.
- Komplexe von Isoflavonen in Sojabohnen bzw. fermentierten Sojabohnen mit beta- und gamma-Cyclodextrinen

werden in der europäischen Patentanmeldung EP-A- 796 624 beschrieben. Die Komplexbildung erhöht die Löslichkeit der Isoflavone und reduziert deren Bitterkeit.

- Rutin- Komplexe mit beta- und gamma-Cyclodextrinen und ihre Verwendung als Antioxidans werden in der Japanischen Patentanmeldung JP 05/9137499 beschrieben.
- Getränke enthaltend Cyclodextrin-Komplexe von Quercetin-Glycosiden werden in der Japanischen Patentanmeldung JP 07/075536 beschrieben.
- In der Japanischen Patentanmeldung JP 05/186344 werden Zusammensetzungen enthaltend Vitamin C und Cyclodextrin-Komplexe von Vitamin P, welche die Bioabsorption von Vitamin C verbessern, beschrieben. Es werden Komplexe von Rutin, Hesperidin und Eriocitrin, wie beispielsweise ein Rutin-beta-Cyclodextrin-Komplex mit Molverhältnis 1.2 beschrieben.
- Die Wirkung eines Komplexes von 3-Methoxyquercetin mit Hydroxypropyl-beta-cyclodextrin auf nasale Epithelzellen wird in S. Dimova, R. Mugabowindekwe et al. Int. J. Pharm. 26381-2) 2003, 95-103 untersucht.
- Beta-Cyclodextrin-Komplexe verschiedener Flavonoide (Hesperetin, Hesperidin, Naringenin, Naringin) werden in R. Ficarra, S. Tommasini et al.; J. Pharm. Biomed. Analysis 29(6) 2002, 1005-1014 charakterisiert.
- In R.-L. Wang, Yu Yang et al.; Yingyong Huaxue 19(7) 2002 702-704 wird die Stabilität und Löslichkeit verschiedener Beta-Cyclodextrin-Komplexe verschiedener Flavonoide (Rutin, Quercetin, Morin) verglichen. Methyl-beta-cyclodextrin-Komplexe erwiesen sich dabei als besonders gut löslich.
- K. Hostettmann, M. Lederer und A. Marston; Phytochemical Analysis 1186) 2000, 380-382 untersuchen die eluierende Wirkung von 2-Hydroxypropyl-beta-cyclodextrin auf Flavonoide, die auf Cellulose absorbiert sind.

Es hat sich für den Fachmann nicht vorhersehbar herausgestellt, dass Zubereitungen zur topischen Anwendung enthaltend die oben genannten Komplex-Verbindungen der Formel I bzw. Verbindungen der Formel II und Cyclodextrine den Nachteilen des Standes der Technik abhelfen.

Dabei ist es insbesondere von Vorteil, wenn als Cyclodextrine an ein oder mehreren Hydroxygruppen $C_{1-24}$-Alkyl- oder $C_{1-24}$-Hydroxyalkyl-substituierte Cyclodextrine, wie insbesondere Hydroxypropyl-Cyclodextrin, oder Gemische aus Cyclodextrinen, welche mindestens zu 30 Gew.%, bezogen auf das Gesamtgewicht des Cyclodextringemisches an den o.g. Cyclodextrinen enthalten, verwendet werden. Dabei ist es insbesondere bevorzugt, wenn β- oder γ-Cyclodextrine eingesetzt werden.

Weiterhin vorteilhaft ist es, wenn der Gehalt an Cyclodextrinen 0,01-20,0 Gew.%, bevorzugt 0,05-10,0 Gew. %, besonders bevorzugt 0,1-5,0 Gew.%, jeweils bezogen auf das Gesamtgewicht der Zusammensetzung beträgt. Der Anteil der Verbindungen der Formel II in der Zubereitung beträgt dabei vorzugsweise von 0,01 bis 20 Gew.%, besonders bevorzugt von 0,05 bis 10 Gew.% und insbesondere bevorzugt von 0,1 bis 5 Gew.% bezogen auf die gesamte Zubereitung. Ganz außerordentlich bevorzugt beträgt der Anteil der Verbindungen der Formel II in der Zubereitung von 0,1 bis 2 Gew.% bezogen auf die gesamte Zubereitung.

Erfindungsgemäß vorteilhaft ist die Verwendung von Cyclodextrinen und/oder Cyclodextrinderivaten zur Erhöhung der Löslichkeit von Verbindungen der Formel II. Weiterhin vorteilhaft ist die Verwendung von Cyclodextrinen und/oder Cyclodextrinderivaten zur Verbesserung der biologischen Wirksamkeit von Verbindungen der Formel II.

Die Wirkstoffkombinationen gemäß der Erfindung bzw. kosmetische oder dermatologische Zubereitungen, solche Wirkstoffkombinationen enthaltend, sind in jeglicher Hinsicht überaus befriedigende Präparate. Es war für den Fachmann nicht vorauszusehen, dass die Zubereitungen gemäß der Erfindung

- Verbindungen der Formel II in erhöhter Verfügbarkeit bereitstellen,
- die Haut besser vor Umwelteinflüssen schützen als die Zubereitungen des Standes der Technik.

Die Verbindungen der Formel I werden erfindungsgemäß typisch in Mengen von 0,01 bis 20 Gew.-%, vorzugsweise in Mengen von 0,1 Gew.- % bis 10 Gew.-% und insbesondere bevorzugt in Mengen von 1 bis 8 Gew.-% eingesetzt. Dabei bereitet es dem Fachmann keinerlei Schwierigkeiten die Mengen abhängig von der beabsichtigten Wirkung der Zubereitung entsprechend auszuwählen.

Bevorzugt sind dabei Verbindungen der Formel I bzw. Zubereitungen enthaltend Verbindungen gemäß Formel I oder Formel II bei denen mindestens 3 Reste aus $R^1$ bis $R^7$ stehen für OH und im Molekül mindestens 2 Paare benachbarter Gruppen -OH vorliegen, oder bei denen $R^2$, $R^5$ und $R^6$ für OH und die Reste $R^1$, $R^3$, $R^4$ und $R^{7-10}$ für H stehen. Bevorzugt sind auch Verbindungen der Formel I bzw. Zubereitungen enthaltend zumindest eine Verbindung der Formel I, die dadurch gekennzeichnet ist, dass mindestens zwei benachbarte Reste der Reste $R^1$ bis $R^4$ stehen für OH und mindestens zwei benachbarte Reste der Reste $R^5$ bis $R^7$ stehen für OH.

Insbesondere bevorzugte Zubereitungen enthalten zumindest eine Verbindung der Formel I, die dadurch gekennzeichnet ist, dass mindestens drei benachbarte Reste der Reste $R^1$ bis $R^4$ stehen für OH, wobei vorzugsweise die Reste $R^1$ bis $R^3$ für OH stehen.

Damit die Verbindungen der Formel I ihre positive Wirkung als Radikalfänger auf die Haut besonders gut

entwickeln können, kann es bevorzugt sein die Verbindungen der Formel I in tiefere Hautschichten eindringen zu lassen. Dazu stehen mehrere Möglichkeiten zur Verfügung. Zum einen können die Verbindungen der Formel I eine ausreichende Lipophilie aufweisen, um durch die äußere Hautschicht in epidermale Schichten vordringen zu können. Als weitere Möglichkeit können in der Zubereitung auch entsprechende Transportmittel, beispielsweise Liposomen, vorgesehen sein, die einen Transport der Verbindungen der Formel I durch die äußeren Hautschichten ermöglichen. Schließlich ist auch ein systemischer Transport der Verbindungen der Formel I denkbar. Die Zubereitung wird dann beispielsweise so gestaltet, dass sie für eine orale Gabe geeignet ist.

Allgemein wirken die Substanzen der Formel I als Radikalfänger. Solche Radikale werden nicht nur durch Sonnenlicht erzeugt, sondern werden unter verschiedenen Bedingungen gebildet. Beispiele sind Anoxie, die den Elektronenfluß stromauf der Cytochromoxidasen blockiert und die Bildung von Superoxidradikalarionen bedingt; Entzündungen, die unter anderem mit der Bildung von Superoxidanionen durch die Membran-NADPH-Oxidase der Leukozyten einhergehen, die jedoch auch mit der Bildung (durch Disproportionierung in Gegenwart von Eisen (II)-ionen) der Hydroxyradikale und anderer reaktiver Spezies, die normalerweise beim Phänomen einer Phagocytose beteiligt sind, einhergehen; sowie Lipidautooxidation die im Allgemeinen durch ein Hydroxylradikal initiiert wird und lipidische Alkoxyradikale und Hydroperoxide liefert.

Es wird vermutet, dass bevorzugte Verbindungen der Formel I auch als Enzymhemmer wirken. Sie hemmen vermutlich Histidindecarboxylase, Proteinkinasen, Elastase, Aldosereduktase sowie Hyaluronidase, und ermöglichen daher, die Unversehrtheit der Grundsubstanz vaskulärer Hüllen aufrecht zu erhalten. Ferner hemmen sie vermutlich nicht spezifisch Katechol-O-methyltransferase, wodurch die Menge der verfügbaren Katecholamine und dadurch die Gefäßfestigkeit erhöht wird. Weiter hemmen sie AMP-Phosphodiesterase, wodurch die Substanzen ein Potential zur Hemmung der Thrombozytenaggregation aufweisen. Aufgrund dieser Eigenschaften eignen sich die erfindungsgemäßen Zubereitungen allgemein zur Immunprotektion und zum Schutz der DNA und RNA. Insbesondere eignen sich die Zubereitungen dabei zum Schutz von DNA und RNA vor oxidativen Angriffen, vor Radikalen und vor Schädigung durch Strahlung, insbesondere UV-Strahlung. Ein weiterer Vorteil der erfindungsgemäßen Zubereitungen ist der Zellschutz, insbesondere der Schutz von Langerhans-Zellen vor Schäden durch die oben genannten Einflüsse. Alle diese Verwendungen bzw. die Verwendung der Verbindungen der formel I zur Herstellung entsprechend einsetzbarer Zubereitungen sind ausdrücklich auch Gegenstand der vorliegenden Erfindung.

Insbesondere eignen sich bevorzugte erfindungsgemäße Zusammensetzungen auch zur Behandlung von Hautkrankheiten, die mit einer Störung der Keratinisierung verbunden sind, die die Differenzierung und Zellprolif-eration betrifft, insbe-sondere zur Behandlung der Akne vulgaris, Akne comedonicá, der polymorphen Akne, der Akne rosaceae, der nodulären Akne, der Akne conglobata, der alters-bedingten Aknen, der als Neben-wirkung auftretenden Aknen, wie der Akne solaris, der medikamenten-bedingten Akne oder der Akne professionalis, zur Behandlung anderer Störungen der Keratinisierung, insbesondere der Ichtyosen, der ichtyosi-formen Zustände, der Darrier-Krankheit, der Keratosis palmoplantaris, der Leukoplasien, der leukoplasiformen Zustände, der Haut- und Schleimhaut-flechten (Buccal) (Lichen), zur Behandlung anderer Hauterkrankungen, die mit einer Störung der Keratinisierung zusammenhängen und eine entzünd-liche und/oder immunoallergische Komponente haben und insbesondere aller Formen der Psoriasis, die die Haut, die Schleimhäute und die Finger und Zehennägel betreffen, und des psoriatischen Rheumas und der Hautatopien, wie Ekzemen oder der respiratorischen Atopie oder auch der Hypertrophie des Zahnfleisches, wobei die Verbindungen ferner bei einigen Entzündungen verwendet werden können, die nicht mit einer Störung der Keratinisierung zusammenhängen, zur Behandlung aller gutartigen oder bösartigen Wucherungen der Dermis oder Epidermis, die gegebenenfalls viralen Ursprungs sind, wie Verruca vulgaris. Veruca plana, Epidermodysplasia verruciformis, orale Papillomatose, Papillomatosis florida, und der Wucherungen, die durch UV-Strahlung hervorgerufen werden können, insbesondere des Epithelioma baso-cellulare und Epithelioma spinocellulare, zur Behandlung anderer Hautkrankheiten, wie der Dermatitis bullosa und der das Kollagen betreffenden Krankheiten, zur Behandlung bestimmter Augenkrankheiten, insbesondere der Hornhauterkran-kungen, zur Behebung oder Bekämpfung der lichtbedingten und der mit dem Älterwerden zusammenhängenden Hautalterung, zur Verminderung der Pigmentierungen und der Keratosis actinica und zur Behandlung aller Krankheiten, die mit der normalen Alterung oder der lichtbedingten Alterung zusammenhängen, zur Vorbeugung vor oder der Heilung von Wunden/Narben der Atrophien der Epidermis und/oder Dermis, die durch lokal oder systemisch angewendete Corticosteroide hervorgerufen werden und aller sonstigen Arten der Hautatrophie, zur Vorbeugung vor oder Behandlung von Störungen der Wundheilung, zur Vermeidung oder Behebung von Schwanger-schaftsstreifen oder auch zur Förderung der Wundheilung, zur Bekämpfung von Störungen der Talgproduktion, wie Hypersebhorrhö bei Akne oder der einfachen Seborrhö, zur Bekämpfung von oder Vorbeugung von krebsartigen Zuständen oder vor präkanzerogenen Zuständen, insbesondere der promyelozytären Leukämien, zur Behandlung von Entzündungserkrankungen, wie Arthritis, zur Behandlung aller virusbedingten Erkrankungen der Haut oder anderer Bereiche des Körpers, zur Vorbeugung vor oder Behandlung der Alopecie, zur Behandlung von Hautkrankheiten oder Krankheiten anderer Körperbereiche mit einer immuno-logischen Komponente, zur Behandlung von Herz-/Kreislauf-Erkran-kungen, wie Arteriosklerose oder Bluthochdruck, sowie des Insulin-unabhängigen Diabetes, zur Behandlung von Hautproblemen, die durch UV-Strahlung hervorgerufen werden.

Die Antioxidanten Wirkungen der Verbindungen gemäß Formel I können beispielsweise mit dem 2,2-Diphenyl-1-picrylhydrazyl(DPPH)-Assay gezeigt werden. 2,2-Diphenyl-1-picrylhydrazyl ist ein in Lösung stabiles freies Radikal. Das ungepaarte Elektron führt zu einer starken Absorptionsbande bei 515 nm, die Lösung ist dunkel-violett gefärbt. In Gegenwart eines Radikalfängers wird das Elektron gepaart, die Absorption verschwindet und die Entfärbung verläuft stöchiometrisch unter Berücksichtigung der aufgenommenen Elektronen. Gemessen wird die Extinktion im Photometer. Die antiradikalische Eigenschaft der zu testenden Substanz wird bestimmt, indem man die Konzentration ermittelt, bei der 50 % des eingesetzten 2,2-Diphenyl-1-picrylhydrazyls mit dem Radikalfänger reagiert haben. Ausgedrückt wird diese Konzentration als $EC_{50}$, ein Wert, der unter den gegebenen Messbedingungen als Substanzeigenschaft zu betrachten ist. Verglichen wird die untersuchte Substanz mit einem Standard (z.B. Tocopherol). Der $EC_{50}$-Wert ist dabei ein Maß für die Kapazität der jeweiligen Verbindung Radikale zu fangen. Je niedriger der $EC_{50}$-Wert ist, desto höher ist die Kapazität Radikale zu fangen. Im Sinne dieser Erfindung wird von einer großen oder hohen Kapazität Radikale zu fangen gesprochen, wenn der $EC_{50}$-Wert niedriger als der von Tocopherol.

Ein weiterer wichtiger Aspekt für die Wirkung der Antioxidantien ist die Zeit in der dieser $EC_{50}$-Wert erreicht wird. Diese Zeit gemessen in Minuten ergibt den $T_{EC50}$-Wert, der eine Aussage über die Geschwindigkeit zulässt, mit der diese Antioxidantien Radikale fangen. Im Sinne dieser Erfindungen gelten Antioxidantien, die diesen Wert innerhalb von weniger als 60 Minuten erreichen als schnell, solche die den $EC_{50}$-Wert erst nach mehr als 120 Minuten erreichen als zeitverzögert wirkend.

Die antiradikalische Effizienz (AE) (beschrieben bei C. Sanchez-Moreno, J.A. Larrauri und F. Saura-Calixto in J. Sci. Food Agric. 1998, 76(2), 270-276.) ergibt sich aus den oben genannten Größen nach folgender Beziehung:

$$AE = \frac{1}{EC_{50} T_{EC50}}$$

Eine niedrige AE ($x10^{-3}$) liegt im Bereich bis etwa 10, von einer mittleren AE wird im Bereich von 10 bis 20 gesprochen und eine hohe AE liegt erfindungsgemäß bei Werten oberhalb 20 vor.

Dabei kann es erfindungsgemäß insbesondere bevorzugt sein, schnell wirkende Antioxidantien mit solchen mit langsamer oder zeitverzögerter Wirkung zu kombinieren. Dabei sind typische Gewichtsverhältnisse der schnell wirkenden Antioxidantien zu zeitverzögert wirkenden Antioxidantien im Bereich 10:1 bis 1:10, vorzugsweise im Bereich 10:1 bis 1:1 und für hautschützende Zubereitungen insbesondere bevorzugt im Bereich 5:1 bis 2:1. In anderen erfindungsgemäß ebenfalls bevorzugten Zubereitungen kann es im Sinne einer Wirkungsoptimierung allerdings von Vorteil sein, mehr zeitverzögert wirkende Antioxidantien als schnell wirkende Antioxidanten vorliegen. Typische Zusammensetzungen zeigen dann Gewichtsverhältnisse der schnell wirkenden Antioxidantien zu zeitverzögert wirkenden Antioxidantien im Bereich 1:1 bis 1:10, vorzugsweise im Bereich 1:2 bis 1:8.

Die schützende Wirkung gegen oxidativen Stress bzw. gegen die Einwirkung von Radikalen kann also weiter verbessert werden, wenn die Zubereitungen ein oder mehrere weitere Antioxidantien enthalten, wobei es dem Fachmann keinerlei Schwierigkeiten bereitet geeignet schnell oder zeitverzögert wirkende Antioxidantien auszuwählen..

In einer bevorzugten Ausführungsform der vorliegenden Erfindungen handelt es sich bei der Zubereitung daher um eine Zubereitung zum Schutz von Körperzellen gegen oxidativen Stress, insbesondere zur Verringerung der Hautalterung, dadurch gekennzeichnet, dass sie neben den ein oder mehreren Verbindungen nach Formel I vorzugsweise ein oder mehrere weitere Antioxidantien enthält.

Es gibt viele aus der Fachliteratur bekannte und bewährte Substanzen, die als Antioxidantien verwendet werden können, z.B. Aminosäuren (z.B. Glycin, Histidin, Tyrosin, Tryptophan) und deren Derivate, Imidazole, (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis μmol/kg), ferner (Metall-) Chelatoren, (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate, Vitamin C und Derivate (z.B. Ascorbylpalmitat, Magnesium-Ascorbylphosphat, Ascorbylacetat), Tocopherole und Derivate (z.B. Vitamin-E-acetat), Vitamin A und Derivate (z.B. Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordohydroguajaretsäure, Trihydroxybutyrophenon, Quercitin, Harnsäure und deren Derivate, Mannose und deren Derivate, Zink und dessen Derivate (z.B. ZnO, $ZnSO_4$), Selen und dessen Derivate (z.B. Selenmethionin), Stilbene und deren Derivate (z.B. Stilbenoxid, trans-Stilbenoxid).

Mischungen von Antioxidantien sind ebenfalls zur Verwendung in den erfindungsgemäßen kosmetischen Zubereitungen geeignet. Bekannte und käufliche Mischungen sind beispielsweise Mischungen enthaltend als aktive Inhaltsstoffe Lecithin, L-(+)-Ascorbylpalmitat und Zitronensäure (z.B. (z.B. Oxynex® AP), natürliche Tocopherole, L-(+)-Ascorbylpalmitat, L-(+)-Ascorbinsäure und Zitronensäure (z.B. Oxynex® K LIQUID), Tocopherolextrakte aus natürlichen Quellen, L-(+)-Ascorbylpalmitat, L-(+)-Ascorbinsäure und Zitronensäure (z.B. Oxynex® L LIQUID), DL-α-Tocopherol, L-(+)-Ascorbylpalmitat, Zitronensäure und Lecithin (z.B. Oxynex® LM) oder Butylhydroxytoluol (BHT), L-(+)-Ascorbylpalmitat und Zitronensäure (z.B. Oxynex® 2004). Derartige Antioxidantien werden mit Verbindungen der Formel I in solchen Zusammensetzungen überlicherweise in Verhältnissen im Bereich von 1000:1 bis 1:1000, bevorzugt in Mengen von 100:1 bis 1:100 eingesetzt.

Die erfindungsgemäßen Zubereitungen können als weitere Inhaltsstoffe Vitamine enthalten. Bevorzugt sind Vitamine und Vitamin-Derivate ausgewählt aus Vitamin A, Vitamin-A-Propionat, Vitamin-A-Palmitat, Vitamin-A-Acetat, Retinol, Vitamin B, Thiaminchloridhydrochlorid (Vitamin $B_1$), Riboflavin (Vitamin $B_2$), Nicotinsäureamid, Vitamin C (Ascorbinsäure), Vitamin D, Ergocalciferol (Vitamin $D_2$), Vitamin E, DL-α-Tocopherol, Tocopherol-E-Acetat, Tocopherolhydrogensuccinat, Vitamin $K_1$, Esculin (Vitamin P-Wirkstoff), Thiamin (Vitamin $B_1$), Nicotinsäure (Niacin), Pyridoxin, Pyridoxal, Pyridoxamin, (Vitamin $B_6$), Panthothensäure, Biotin, Folsäure und Cobalamin (Vitamin $B_{12}$) in den erfindungsgemäßen kosmetischen Zubereitungen enthalten, insbesondere bevorzugt Vitamin-A-Palmitat, Vitamin C und dessen Derivaten, DL-α-Tocopherol, Tocopherol-E-Acetat, Nicotinsäure, Pantothensäure und Biotin. Vitamine werden dabei mit Verbindungen der Formel I überlicherweise in Verhältnissen im Bereich von 1000:1 bis 1:1000, bevorzugt in Mengen von 100: 1 bis 1:100 eingesetzt.

Die erfindungsgemäßen Verbindungen der Formel I weisen in der Regel auch ein UV-Absorption im UV-A- und oder UV-B-ereich auf. Unter den erfindungsgemäß einzusetzenden Flavonoiden der Formel I finden sich dabei Breitband-UV-Filter, die alleine oder in Kombination mit weiteren UV-Filtern eingesetzt werden können. Andere ebenfalls bevorzugte Verbindungen der Formel I zeigen ein Absorptionsmaximum im Grenzbereich zwischen der UV-B- und der UV-A-Strahlung. Als UV-A-II-Filter ergänzen sie daher vorteilhaft das Absorptionsspektrum von handelsüblichen UV-B- bzw. UV-A-I-Filtern.

Als Mono- oder Oligosaccharid-reste bevorzugt sind dabei Hexosylreste, insbesondere Ramnosylreste und Glucosylreste. Aber auch andere Hexosylreste, beispielsweise Allosyl, Altrosyl, Galactosyl, Gulosyl, Idosyl, Mannosyl und Talosyl sind gegebenenfalls vorteilhaft zu verwenden. Es kann auch vorteilhaft sein, Pentosylreste zu verwenden. Die Glycosylreste können α- oder β-glycosidisch mit dem Grundkörper verbunden sein. Ein bevorzugtes Disaccharid ist beispielsweise das 6-O-(6-deoxy-α-L-mannopyranosyl)-β-D-glucopyranosid.

Erfindungsgemäß insbesondere bevorzugte Zubereitungen enthalten neben den Verbindungen der Formel I auch reine UV-Filter.

Bei Einsatz der als UV-A-Filter insbesondere bevorzugten Dibenzoylmethanderivate in Kombination mit den Verbindungen der Formel I ergibt sich ein zusätzlicher Vorteil: Die UV-empfindlichen Dibenzoylmethanderivate werden durch die Anwesenheit der Verbindungen der Formel I zusätzlich stabilisiert. Ein weiterer Gegenstand der vorliegenden Erfindung ist daher die Verwendung der Verbindungen gemäß Formel I zur Stabilisierung von Dibenzoylmethanderivaten in Zubereitungen.

Prinzipiell kommen alle UV-Filter für eine Kombination mit den erfindungsgemäßen Verbindungen der Formel I in Frage. Besonders bevorzugt sind solche UV-Filter, deren physiologische Unbedenklichkeit bereits nachgewiesen ist. Sowohl für UVA wie auch UVB-Filter gibt es viele aus der Fachliteratur bekannte und bewährte Substanzen, z.B.

Benzylidenkampferderivate wie 3-(4'-Methylbenzyliden)-dl-kampfer (z.B. Eusolex® 6300), 3-Benzylidenkampfer (z.B. Mexoryl® SD), Polymere von N-{(2 und 4)-[(2-oxoborn-3-yliden)methyl]benzyl}-acrylamid (z.B. Mexoryl® SW), N,N,N-Trimethyl-4-(2-oxoborn-3-ylidenmethyl)anilinium methylsulfat (z.B. Mexoryl® SK) oder (2-Oxoborn-3-yliden)toluol-4-sulfonsäure (z.B. Mexoryl® SL),

Benzoyl- oder Dibenzoylmethane wie 1-(4-tert-Butylphenyl)-3-(4-methoxyphenyl)propan-1,3-dion (z.B. Eusolex® 9020) oder 4-Isopropyldibenzoylmethan (z.B. Eusolex® 8020),

Benzophenone wie 2-Hydroxy-4-methoxybenzophenon (z.B. Eusolex® 4360) oder 2-Hydroxy-4-methoxybenzophenon-5-sulfonsäure und ihr Natriumsalz (z.B. Uvinul® MS-40),

Methoxyzimtsäureester wie Methoxyzimtsäureoctylester (z.B. Eusolex® 2292), 4-Methoxyzimtsäureisopentylester, z.B. als Gemisch der Isomere (z.B. Neo Heliopan® E 1000),

Salicylatderivate wie 2-Ethylhexylsalicylat (z.B. Eusolex® OS), 4-Isopropylbenzylsalicylat (z.B. Megasol®) oder 3,3,5-Trimethylcyclohexylsalicylat (z.B. Eusolex® HMS),

4-Aminobenzoesäure und Derivate wie 4-Aminobenzoesäure, 4-(Dimethylamino)benzoesäure-2-ethylhexylester (z.B. Eusolex® 6007), ethoxylierter 4-Aminobenzoesäureethylester (z.B. Uvinul® P25),

Phenylbenzimidazolsulfonsäuren, wie 2-Phenylbenzimidazol-5-sulfonsäure sowie ihre Kalium-, Natrium- und

Triethanolaminsalze (z.B. Eusolex® 232), 2,2-(1,4-Phenylen)-bisbenzimidazol-4,6-disulfonsäure bzw. deren Salze (z.B. Neoheliopan® AP) oder 2,2-(1,4-Phenylen)-bisbenzimidazol-6-sulfonsäure;
und weitere Substanzen wie

- 2-Cyano-3,3-diphenylacrylsäure-2-ethylhexylester (z.B. Eusolex® OCR),
- 3,3'-(1,4-Phenylendimethylen)-bis-(7,7-dimethyl-2-oxobicyclo-[2.2.1]hept-1-ylmethansulfonsäure sowie ihre Salze (z.B. Mexoryl® SX) und
- 2,4,6-Trianilino-(p-carbo-2'-ethylhexyl-1'-oxi)-1,3,5-triazin (z.B. Uvinul® T 150)
- 2-(4-Diethylamino-2-hydroxy-benzoyl)-benzoesäure hexylester (z.B. Uvinul®UVA Plus, Fa. BASF).

Die in der Liste aufgeführten Verbindungen sind nur als Beispiele aufzufassen. Selbstverständlich können auch andere UV-Filter verwendet werden.

Diese organischen UV-Filter werden in der Regel in einer Menge von 0,5 bis 10 Gewichtsprozent, vorzugsweise 1 - 8 %, in kosmetische Formulierungen eingearbeitet.

Weitere geeignete organische UV-Filter sind z.B.

- 2-(2H-Benzotriazol-2-yl)-4-methyl-6-(2-methyl-3-(1,3,3,3-tetramethyl-1-(trimethylsilyloxy)disiloxanyl)propyl)phenol (z.B. Silatrizole®),
- 4,4'-[(6-[4-((1,1-Dimethylethyl)aminocarbonyl)phenylamino]-1,3,5-triazin-2,4-diyl)diimino]bis(benzoesäure-2-ethylhexylester) (z.B. Uvasorb® HEB),
- $\alpha$-(Trimethylsilyl)-$\omega$-[trimethylsilyl)oxy]poly[oxy(dimethyl [und ca. 6% methyl[2-[p-[2,2-bis(ethoxycarbonyl]vinyl] phenoxy]-1-methylenethyl] und ca. 1,5 % methyl[3-[p-[2,2-bis(ethoxycarbonyl)vinyl)phenoxy propenyl) und 0,1 bis 0,4% (methylhydrogen]silylen]] (n $\approx$ 60) (CAS-Nr. 207 574-74-1)
- 2,2'-Methylen-bis-(6-(2H-benzotriazol-2-yl)-4-(1,1,3,3-tetramethylbutyl)phenol) (CAS-Nr. 103 597-45-1)
- 2,2'-(1,4-Phenylen)bis-(1H-benzimidazol-4,6-disulfonsäure, Mononatriumsalz) (CAS-Nr. 180 898-37-7) und
- 2,4-bis-{[4-(2-Ethyl-hexyloxy)-2-hydroxyl]-phenyl}-6-(4-methoxyphenyl)-1,3,5-triazin (CAS-Nr. 103 597-45-, 187 393-00-6).
- 4,4'-[(6-[4-((1,1-Dimethylethyl)aminocarbonyl)phenylamino]-1,3,5-triazin-2,4-diyl)diimino]bis(benzoesäure-2-ethylhexylester) (z.B. Uvasorb® HEB),

Weitere geeignete UV-Filter sind auch Methoxyflavone ensprechend der älteren Deutschen Patentanmeldung DE 10232595.2.

Organische UV-Filter werden in der Regel in einer Menge von 0,5 bis 20 Gewichtsprozent, vorzugsweise 1 - 15 %, in kosmetische Formulie-rungen eingearbeitet.

Als anorganische UV-Filter sind solche aus der Gruppe der Titandioxide wie z.B. gecoatetes Titandioxid (z.B. Eusolex® T-2000, Eusolex®T-AQUA), Zinkoxide (z.B. Sachtotec®), Eisenoxide oder auch Ceroxide denkbar. Diese anorganischen UV-Filter werden in der Regel in einer Menge von 0,5 bis 20 Gewichtsprozent, vorzugsweise 2 - 10 %, in kosmetische Zubereitungen eingearbeitet.

Bevorzugte Verbindungen mit UV-filternden Eigenschaften sind 3-(4'-Methylbenzyliden)-dl-kampfer, 1-(4-tert-Butylphenyl)-3-(4-methoxy-phenyl)-pro-pan-1,3-dion, 4-Isopropyldibenzoylmethan, 2-Hydroxy-4-meth-oxy-ben-zo--phenon, Methoxyzimtsäureoctylester, 3,3,5-Trimethyl-cyclo-hexylsali-cylat, 4-(Dimethylamino)benzoesäure-2-ethyl-hexylester, 2-Cyano-3,3-di-phenyl-acrylsäure-2-ethylhexylester, 2-Phenyl-benzimidazol-5-sulfonsäure sowie ihre Kalium-, Natrium- und Triethanol-aminsalze.

Durch Kombination von einer oder mehrerer Verbindungen der Formel I mit weiteren UV-Filtern kann die Schutzwirkung gegen schädliche Einwirkungen der UV-Strahlung optimiert werden.

Optimierte Zusammensetzungen können beispielsweise die Kombination der organischen UV-Filter 4'-Methoxy-6-hydroxyflavon-CD mit 1-(4-tert-Butylphenyl)-3-(4-methoxyphenyl)propan-1,3-dion und 3-(4'-Methylbenzyliden)-dl-kampfer enthalten. Mit dieser Kombination ergibt sich ein Breitbandschutz, der durch Zusatz von anorganischen UV-Filtern, wie Titandioxid-Mikropartikeln noch ergänzt werden kann.

Alle genannten UV-Filter können auch in verkapselter Form eingesetzt werden. Insbesondere ist es von Vorteil organische UV-Filter in verkapselter Form einzusetzen. Im Einzelnen ergeben sich die folgende Vorteile:

- Die Hydrophilie der Kapselwand kann unabhängig von der Löslichkeit des UV-Filters eingestellt werden. So können beispielsweise auch hydrophobe UV-Filter in rein wässrige Zubereitungen eingearbeitet werden. Zudem wird der häufig als unangenehm empfundene ölige Eindruck beim Auftragen der hydrophobe UV-Filter enthaltenden Zubereitung unterbunden.
- Bestimmte UV-Filter, insbesondere Dibenzoylmethanderivate, zeigen in kosmetischen Zubereitungen nur eine verminderte Photostabilität. Durch Verkapselung dieser Filter oder von Verbindungen, die die Photostabilität dieser

Filter beeinträchtigen, wie beispielsweise Zimtsäurederivate, kann die Photostabilität der gesamten Zubereitung erhöht werden.

- In der Literatur wird immer wieder die Hautpenetration durch organische UV-Filter und das damit verbundene Reizpotential beim direkten Auftragen auf die menschliche Haut diskutiert. Durch die hier vorgeschlagene Verkapselung der entsprechenden Substanzen wird dieser Effekt unterbunden.
- Allgemein können durch Verkapselung einzelner UV-Filter oder anderer Inhaltstoffe Zubereitungsprobleme, die durch Wechselwirkung einzelner Zubereitungsbestandteile untereinander entstehen, wie Kristallisationsvorgänge, Ausfällungen und Agglomeratbildung vermieden werden, da die Wechselwirkung unterbunden wird.

Daher ist es erfindungsgemäß bevorzugt, wenn ein oder mehrere der oben genannten UV-Filter in verkapselter Form vorliegen. Vorteilhaft ist es dabei, wenn die Kapseln so klein sind, dass sie mit dem bloßen Auge nicht beobachtet werden können. Zur Erzielung der o.g. Effekte ist es weiterhin erforderlich, dass die Kapseln hinreichend stabil sind und den verkapselten Wirkstoff (UV-Filter) nicht oder nur in geringem Umfang an die Umgebung abgeben.

Geeignete Kapseln können Wände aus anorganischen oder organischen Polymeren aufweisen. Beispielsweise wird in US 6,242,099 B1 die Herstellung geeigneter Kapseln mit Wänden aus Chitin, Chitin-Derivaten oder polyhydroxylierten Polyaminen beschrieben. Erfindungsgemäß besonders bevorzugt einzusetzende Kapseln weisen Wände auf, die durch einen SolGel-Prozeß, wie er in den Anmeldungen WO 00/09652, WO 00/72806 und WO 00/71084 beschrieben ist, erhalten werden können. Bevorzugt sind hier wiederum Kapseln, deren Wände aus Kieselgel (Silica; undefiniertes Silicium-oxid-hydroxid) aufgebaut sind. Die Herstellung entsprechender Kapseln ist dem Fachmann beispielsweise aus den zitierten Patentanmeldungen bekannt, deren Inhalt ausdrücklich auch zum Gegenstand der vorliegenden Anmeldung gehört.

Dabei sind die Kapseln in erfindungsgemäßen Zubereitungen vorzugsweise in solchen Mengen enthalten, die gewährleisten, dass die verkapselten UV-Filter in den oben angegebenen Mengen in der Zubereitung vorliegen.

Die erfindungsgemäßen Zubereitungen können darüber hinaus weitere übliche hautschonende oder hautpflegende Wirkstoffe enthalten. Dies können prinzipiell alle den Fachmann bekannten Wirkstoffe sein.

Die erfindungsgemäßen Zubereitungen können darüber hinaus weitere übliche hautschonende oder hautpflegende Wirkstoffe enthalten. Dies können prinzipiell alle den Fachmann bekannten Wirkstoffe sein.

Dies können Chromon-Derivate sein. Dabei werden vorzugsweise bestimmte Chromen-2-on-Derivate, die sich als Wirkstoffe zur vorbeugenden Behandlung von menschlicher Haut und menschlicher Haare gegen Alterungsprozesse und schädigende Umwelteinflüssen eignen, unter der Bezeichnung Chromon-Derivate verstanden. Sie zeigen gleichzeitig ein niedriges Irritationspotential für die Haut, beeinflussen die Wasserbindung in der Haut positiv, erhalten oder erhöhen die Elastizität der Haut und fördern somit eine Glättung der Haut. Diese Verbindungen entsprechen vorzugsweise der Formel IV

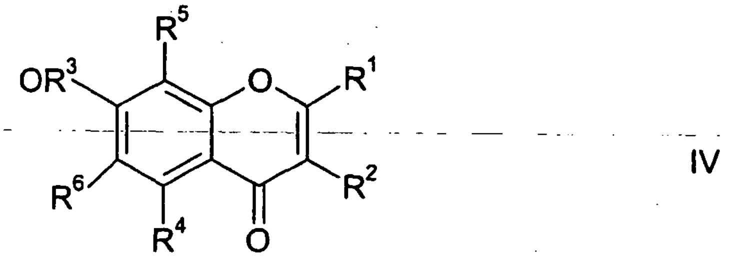

IV

wobei

$R^1$ und $R^2$ gleich oder verschieden sein können und ausgewählt sind aus

- H, -C(=O)-$R^7$, -C(=O)-$OR^7$,
- geradkettigen oder verzweigten $C_1$- bis $C_{20}$-Alkylgruppen,
- geradkettigen oder verzweigten $C_3$- bis $C_{20}$-Alkenylgruppen, geradkettigen oder verzweigten $C_1$- bis $C_{20}$-Hydroxyalkylgruppen, wobei die Hydroxygruppe an ein primäres oder sekundäres Kohlenstoffatom der

Kette gebunden sein kann und weiter die Alkylkette auch durch Sauerstoff unterbrochen sein kann, und/oder

- $C_3$- bis $C_{10}$-Cycloalkylgruppen und/oder $C_3$- bis $C_{12}$-Cycloalkenylgruppen, wobei die Ringe jeweils auch durch $-(CH_2)_n$-Gruppen mit n = 1 bis 3 überbrückt sein können,

  $R^3$ steht für H oder geradkettige oder verzweigte $C_1$- bis $C_{20}$-Alkylgruppen,

  $R^4$ steht für H oder $OR^8$,

$R^5$ und $R^6$ gleich oder verschieden sein können und ausgewählt sind aus

- -H, -OH,
- geradkettigen oder verzweigten $C_1$- bis $C_{20}$-Alkylgruppen,
- geradkettigen oder verzweigten $C_3$- bis $C_{20}$-Alkenylgruppen,
- geradkettigen oder verzweigten $C_1$- bis $C_{20}$-Hydroxyalkylgruppen, wobei die Hydroxygruppe an ein primäres oder sekundäres Kohlenstoffatom der Kette gebunden sein kann und weiter die Alkylkette auch durch Sauerstoff unterbrochen sein kann und

$R^7$ steht für H, geradkettige oder verzweigte $C_1$- bis $C_{20}$-Alkylgruppen, eine Polyhydroxy-Verbindung, wie vorzugsweise einen Ascorbinsäurerest oder glycosidische Reste und

$R^8$ steht für H oder geradkettige oder verzweigte $C_1$- bis $C_{20}$-Alkylgruppen, wobei mindestens 2 der Substituenten $R^1$, $R^2$, $R^4$-$R^6$ verschieden von H sind oder mindestens ein Substituent aus $R^1$ und $R^2$ für -C(=O)-$R^7$ oder - C (=O)-O$R^7$ steht.

Der Anteil an einer oder mehreren Verbindungen ausgewählt Cromon-Derivaten in der erfindungsgemässen Zubereitung beträgt vorzugsweise von 0,001 bis 5 Gew.%, besonders bevorzugt von 0,01 bis 2 Gew.% bezogen auf die gesamte Zubereitung.

Weiter kann es bevorzugt sein, wenn die erfindungsgemäße Zubereitung mindestens ein Repellent enthält, wobei das Repellent vorzugsweise ausgewählt aus N,N-Diethyl-3-methylbenzamid, 3-(Acetyl-butyl-amino)-propionsäure Ethylester, Dimethylphthalat, Butopyronoxyl, 2,3,4,5-bis-(2-Butylen)-tetrahydro-2-furaldehyd, N,N-Caprylsäurediethylamid, N,N-Diethylbenzamid, o-Chlor-N,N-diethylbenzamid, Dimethylcarbat, Di-n-propylisocinchomeronat, 2-Ethylhexan-1,3-diol, N-Octyl-bicyclohepetendiecarboximid, Piperonyl-butoxid, 1-(2-Methylpropyloxycarbonyl)-2-(hydroxyethyl)-piperidin oder Mischungen davon, wobei es insbesondere bevorzugt ausgewählt ist aus N,N-Diethyl-3-methylbenzamid, 3-(Acetyl-butyl-amino)-propionsäure-ethylester 1-(2-Methylpropyloxycarbonyl)-2-(hydroxyethyl)-piperidin oder Mischungen davon.

Bei den erfindungsgemäßen Zubereitungen, die Repellentien enthalten, handelt es sich dabei vorzugsweise um Insektenabwehrmittel. Insektenabwehrmittel werden in Form von Lösungen, Gelen, Stiften, Rollern, Pump-Sprays und Aerosol-Sprays angeboten, wobei Lösungen und Sprays den Hauptteil der im Handel erhältlichen Produkte bilden. Basis für diese beiden Produktformen sind meist alkoholische bzw. wässrig-alkoholische Lösungen unter Zusatz fettender Substanzen und leichter Parfümierung.

Besonders bevorzugte Wirkstoffe sind Pyrimidincarbonsäuren und/oder Aryloxime.

Pyrimidincarbonsäuren kommen in halophilen Mikroorganismen vor und spielen bei der Osmoregulation dieser Organismen eine Rolle *(*E. A. Galinski et al., Eur. J. Biochem., 149 (1985) Seite 135-139). Dabei sind unter den Pyrimidincarbonsäuren insbesondere Ectoin ((S)-1,4,5,6-Tetrahydro-2-methyl-4-pyrimidincarbonsäure) und Hydroxyectoin ((S,S)-1,4,5,6-Tetrahydro-5-hydroxy-2-methyl-4-pyrimidincarbonsäure und deren Derivate zu nennen. Diese Verbindungen stabilisieren Enzyme und andere Biomoleküle in wässrigen Lösungen und organischen Lösungsmitteln. Weiter stabilisieren sie insbesondere Enzyme gegen denaturierende Bedingungen, wie Salze, extreme pH-Werte, Tenside, Harnstoff, Guanidiniumchlorid und andere Verbindungen.

Ectoin und Ectoin-Derivate wie Hydroxyectoin können vorteilhaft in Arzneimitteln verwendet werden. Insbesondere kann Hydroxyectoin zur Herstellung eines Arzneimittels zur Behandlung von Hauterkrankungen eingesetzt werden. Andere Einsatzgebiete des Hydroxyectoins und anderer Ectoin-Derivate liegen typischerweise in Gebieten in denen z.B. Trehalose als Zusatzstoff verwendet wird. So können Ectoin-Derivate, wie Hydroxyectoin, als Schutzstoff in getrockneten Hefe- und Bakterienzellen Verwendung finden. Auch pharmazeutische Produkte wie nicht glykosylierte, pharmazeutische wirksame Peptide und Proteine z.B. t-PA können mit Ectoin oder seinen Derivaten geschützt werden.

Unter den kosmetischen Anwendungen ist insbesondere die Verwendung von Ectoin und Ectoin-Derivaten zur Pflege von gealterter, trockener oder gereizter Haut zu nennen. So wird in der europäischen Patentanmeldung EP-A-0671 161 insbesondere beschrieben, dass Ectoin und Hydroxyectoin in kosmetischen Zubereitungen wie Pudern, Seifen, tensidhaltigen Reinigungsprodukten, Lippenstiften, Rouge, Make-Ups, Pflegecremes und Sonnenschutzpräparaten eingesetzt werden.

Dabei wird vorzugsweise eine Pyrimidincarbonsäure gemäß der unten stehenden Formel V eingesetzt,

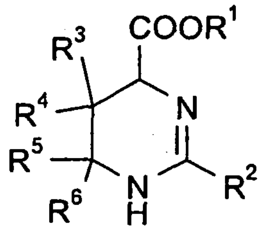

V

worin $R^1$ ein Rest H oder C1-8-Alkyl, $R^2$ ein Rest H oder C1-4-Alkyl und $R^3$, $R^4$, $R^5$ sowie $R^6$ jeweils unabhängig voneinander ein Rest aus der Gruppe H, OH, $NH_2$ und C1-4-Alkyl sind. Bevorzugt werden Pyrimidincarbonsäuren eingesetzt, bei denen $R^2$ eine Methyl- oder eine Ethylgruppe ist und $R^1$ bzw. $R^5$ und $R^6$ H sind. Insbesondere bevorzugt werden die Pyrimidincarbonsäuren Ectoin ((S)-1,4,5,6-Tetrahydro-2-methyl-4-pyrimidin-carbonsäure) und Hydroxyectoin ((S, S)-1,4,5,6-Tetrahydro-5-hydroxy-2-methyl-4-pyrimidin-carbonsäure) eingesetzt. Dabei enthalten die erfindungsgemäßen Zubereitungen derartige Pyrimidincarbonsäuren vorzugsweise in Mengen bis zu 15 Gew.-%. Vorzugsweise werden die Pyrimidincarbonsäuren dabei in Verhältnissen von 100:1 bis 1:100 zu den Verbindungen der Formel I eingesetzt, wobei Verhältnisse im Bereich 1:10 bis 10:1 besonders bevorzugt sind.

Unter den Aryloximen wird vorzugsweise 2-Hydroxy-5-methyllaurophenonoxim, welches auch als HMLO, LPO oder F5 bezeichnet wird, eingesetzt. Seine Eignung zum Einsatz in kosmetischen Mitteln ist beispielsweise aus der Deutschen Offenlegungsschrift DE-A-4116123 bekannt. Zubereitungen, die 2-Hydroxy-5-methyllauro-phenonoxim enthalten, sind demnach zur Behandlung von Hauterkrankungen, die mit Entzündungen einhergehen, geeignet. Es ist bekannt, dass derartige Zubereitungen z.B. zur Therapie der Psioriasis, unterschiedlicher Ekzemformen, irritativer und toxischer Dermatitis, UV-Dermatitis sowie weiterer allergischer und/oder entzündlicher Erkrankungen der Haut und der Hautanhangsgebilde verwendet werden können. Erfindungsgemäße Zubereitungen, die neben der Verbindung der Formel I zusätzlich eine Aryloxim, vorzugsweise 2-Hydroxy-5-methyllaurophenonoxim enthalten, zeigen überraschende antiinflammatorische Eignung. Dabei enthalten die Zubereitungen vorzugsweise 0,01 bis 10 Gew.-% des Aryloxims, wobei es insbesondere bevorzugt ist, wenn die Zubereitung 0,05 bis 5 Gew-% Aryloxim enthält.

In einer weiteren ebenfalls bevorzugten Ausführungsform der vorliegenden Erfindung enthält die erfindungsgemäße Zubereitung mindestens einen Selbstbräuner.

Als vorteilhafte Selbstbräuner können unter anderem eingesetzt werden:

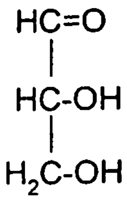
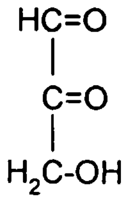
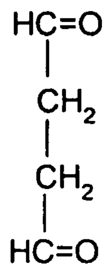
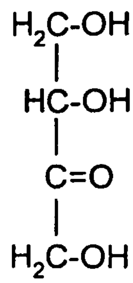

Glycerolaldehyd Hydroxymethylglyoxal γ-Dialdehyd Erythrulose

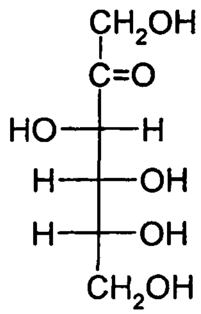

6-Aldo-D-Fructose

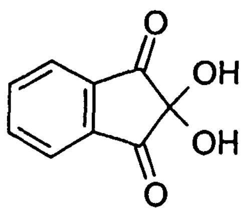

Ninhydrin

Ferner ist das 5-Hydroxy-1,4-naphtochinon (Juglon) zu nennen, das aus den Schalen frischer Walnüsse extrahiert wird

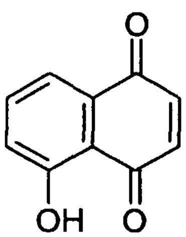

5-Hydroxy-1,4-naphtochinon (Juglon)
sowie das in den Henna-Blättern vorkommende 2-Hydroxy-1,4-naphtochinon (Lawson).
2-Hydroxy-1,4-naphtochinon (Lawson)

Ganz besonders bevorzugt ist das 1,3-Dihydroxyaceton (DHA), ein im menschlichen Körper vorkommender dreiwertiger Zucker und dessen Derivate.

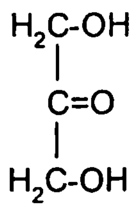

1,3-Dihydroxyaceton (DHA)

Ferner können die erfindungsgemäßen Zubereitungen auch Farbstoffe und Farbpigmente enthalten. Die Farbstoffe und -pigmente können aus der entsprechenden Positivliste der Kosmetikverordnung bzw. der EG-Liste kosmetischer Färbemittel ausgewählt werden. In den meisten Fällen sind sie mit den für Lebensmittel zugelassenen Farbstoffen identisch. Vorteilhafte Farbpigmente sind beispielsweise Titandioxid, Glimmer, Eisenoxide (z. B. $Fe_2O_3$, $Fe_3O_4$, FeO(OH)) und/oder Zinnoxid. Vorteilhafte Farbstoffe sind beispielsweise Carmin, Berliner Blau, Chromoxidgrün, Ultramerinblau und/oder Manganviolett. Es ist insbesondere vorteilhaft, die Farbstoffe und/oder Farbpigmente aus der folgenden Liste zu wählen. Die Colour Index Nummern (CIN) sind dem Rowe Colour Index, 3. Auflage, Society of Dyers and Colourists, Bradford, England, 1971 entnommen.

| **Chemische oder sonstige Bezeichnung** | **CIN** | **Farbe** |
|---|---|---|
| Pigment Green | 10006 | grün |
| Acid Green 1 | 10020 | Grün |
| 2,4-Dinitrohydroxynaphthalin-7-sulfonsäure | 10316 | Gelb |
| Pigment Yellow 1 | 11680 | Gelb |
| Pigment Yellow 3 | 11710 | Gelb |
| Pigment Orange 1 | 11725 | Orange |
| 2,4-Dihydroxyazobenzol | 11920 | Orange |
| Solvent Red 3 | 12010 | Rot |
| 1-(2'-Chlor-4'-nitro-1'-phenylazo)-2-hydroxynaphthalin | 12085 | Rot |
| Pigment Red 3 | 12120 | Rot |
| Ceresrot; Sudanrot; Fettrot G | 12150 | Rot |
| Pigment Red 112 | 12370 | Rot |
| Pigment Red 7 | 12420 | Rot |
| Pigment Brown 1 | 12480 | Braun |
| 4-(2'-Methoxy-5'sulfonsäurediethylamid-1'-phenylazo)-3-hydroxy-5"-chloro-2",4"-dimethoxy2-naphthoesäureanilid | 12490 | Rot |
| Disperse Yellow 16 | 12700 | Gelb |
| 1-(4-Sulfo-1-phenylazo)-4-amino-benzol-5-sulfosäure | 13015 | Gelb |
| 2,4-Dihydroxy-azobenzol-4'-sulfosäure | 14270 | Orange |
| 2-(2,4-Dimethylphenylazo-5-sulfosäure)-1-hydroxynaphthalin-4-sulfosäure | 14700 | Rot |
| 2-(4-Sulfo-1-naphthylazo)-1-naphthol-4-sulfosäure | 14720 | Rot |
| 2-(6-Sulfo-2,4-xylylazo)-1-naphthol-5-sulfosäure | 14815 | Rot |
| 1-(4'-Sulfophenylazo)-2-hydroxynaphthalin | 15510 | Orange |
| 1-(2-Sulfosäure-4-chlor-5-carbonsäure-1-phenylazo)-2-hydroxynaphthalin | 15525 | Rot |
| 1-(3-Methyl-phenylazo-4-sulfosäure)-2-hydroxynaphthalin | 15580 | Rot |
| 1-(4',(8')-Sulfosäurenaphthylazo)-2-hydroxynaphthalin | 15620 | Rot |

(fortgesetzt)

| Chemische oder sonstige Bezeichnung | CIN | Farbe |
|---|---|---|
| 2-Hydroxy-1,2'-azonaphthalin-1'-sulfosäure | 15630 | Rot |
| 3-Hydroxy-4-phenylazo-2-naphthylcarbonsäure | 15800 | Rot |
| 1-(2-Sulfo-4-methyl-1-phenylazo)-2-naphthylcarbonsäure | 15850 | rot |
| 1-(2-Sulfo-4-methyl-5-chlor-1-phenylazo)-2-hydroxy-naphthalin-3-carbonsäure | 15865 | Rot |
| 1-(2-Sulfo-1-naphthylazo)-2-hydroxynaphthalin-3-carbonsäure | 15880 | Rot |
| 1-(3-Sulfo-1-phenylazo)-2-naphthol-6-sulfosäure | 15980 | Orange |
| 1-(4-Sulfo-1-phenylazo)-2-naphthol-6-sulfosäure | 15985 | Gelb |
| Allura Red | 16035 | Rot |
| 1-(4-Sulfo-1-naphthylazo)-2-naphthol-3,6-disulfosäure | 16185 | Rot |
| Acid Orange 10 | 16230 | Orange |
| 1-(4-Sulfo-1-naphthylazo)-2-naphthol-6,8-disulfosäure | 16255 | Rot |
| 1-(4-Sulfo-1-naphthylazo)-2-naphthol-3,6,8-trisulfosäure | 16290 | Rot |
| 8-Amino-2-phenylazo-1-naphthol-3,6-disulfosäure | 17200 | Rot |
| Acid Red 1 | 18050 | Rot |
| Acid Red 155 | 18130 | Rot |
| Acid Yellow 121 | 18690 | Gelb |
| Acid Red 180 | 18736 | Rot |
| Acid Yellow 11 | 18820 | Gelb |
| Acid Yellow 17 | 18965 | Gelb |
| 4-(4-Sulfo-1-phenylazo)-1-(4-sulfophenyl)-5-hydroxy-pyrazolon-3-carbonsäure | 19140 | Gelb |
| Pigment Yellow 16 | 20040 | Gelb |
| 2,6-(4'-Sulfo-2",4"-dimethyl)-bis-phenylazo)1,3-dihydroxybenzol | 20170 | Orange |
| Acid Black 1 | 20470 | Schwarz |
| Pigment Yellow 13 | 21100 | Gelb |
| Pigment Yellow 83 | 21108 | Gelb |
| Solvent Yellow | 21230 | Gelb |
| Acid Red 163 | 24790 | Rot |
| Acid Red 73 | 27290 | Rot |
| 2-[4'-(4"-Sulfo-1"-phenylazo)-7'-sulfo-1'-naphthylazo]-1-hydroxy-7-aminonaphthalin-3,6-disulfosäure | 27755 | schwarz |
| 4-[4"-Sulfo-1"-phenylazo)-7'-sulfo-1'-naphthylazo]-1-hydroxy-8-acetyl-aminonaphthalin-3,5-disulfosäure | 28440 | Schwarz |
| Direct Orange 34, 39, 44, 46, 60 | 40215 | Orange |
| Food Yellow | 40800 | Orange |
| trans-ß-Apo-8'-Carotinaldehyd ($C_{30}$) | 40820 | Orange |
| trans-Apo-8'-Carotinsäure ($C_{30}$)-ethylester | 40850 | Orange |
| Canthaxanthin | 40850 | Orange |
| Acid Blue 1 | 42045 | Blau |
| 2,4-Disulfo-5-hydroxy-4'-4"-bis-(diethylamino)triphenyl-carbinol | 42051 | Blau |
| 4-[(-4-N-Ethyl-p-sulfobenzylamino)-phenyl-(4-hydroxy-2-sulfophenyl)-(methylen)-1-(N-ethylN-p-sulfobenzyl)-2,5-cyclohexadienimin] | 42053 | Grün |
| Acid Blue 7 | 42080 | Blau |
| (N-Ethyl-p-sulfobenzyl-amino)-phenyl-(2-sulfophenyl)-methylen-(N-ethyl-N-p-sulfo-benzyl) $\Delta^{2,5}$-cyclohexadienimin | 42090 | Blau |
| Acid Green 9 | 42100 | Grün |
| Diethyl-di-sulfobenzyl-di-4-amino-2-chlor-di-2-methyl-fuchsonimmonium | 42170 | Grün |
| Basic Violet 14 | 42510 | Violet |
| Basic Violet 2 | 42520 | Violet |

(fortgesetzt)

| Chemische oder sonstige Bezeichnung | CIN | Farbe |
|---|---|---|
| 2'-Methyl-4'-(N-ethyl-N-m-sulfobenzyl)-amino-4"-(N-diethyl)-amino-2-methyl-N-ethyllN-m-sulfobenzyl-fuchsonimmonium | 42735 | Blau |
| 4'-(N-Dimethyl)-amino-4"-(N-phenyl)-aminonaphtho-N-dimethylfuchsonimmonium | 44045 | Blau |
| 2-Hydroxy-3,6-disulfo-4,4'-bis-dimethylaminonaphthofuchsonimmonium | 44090 | Grün |
| Acid Red 52 | 45100 | Rot |
| 3-(2'-Methylphenylamino)-6-(2'-methyl-4'-sulfophenylamino)-9-(2"-carboxyphenyl)-xantheniumsalz | 45190 | Violet |
| Acid Red 50 | 45220 | Rot |
| Phenyl-2-oxyfluoron-2-carbonsäure | 45350 | gelb |
| 4,5-Dibromfluorescein | 45370 | Orange |
| 2,4,5,7-Tetrabromfluorescein | 45380 | Rot |
| Solvent Dye | 45396 | Orange |
| Acid Red 98 | 45405 | Rot |
| 3',4',5',6'-Tetrachlor-2,4,5,7-tetrabromfluorescein | 45410 | Rot |
| 4,5-Diiodfluorescein | 45425 | Rot |
| 2,4,5,7-Tetraiodfluorescein | 45430 | Rot |
| Chinophthalon | 47000 | Gelb |
| Chinophthalon-disulfosäure | 47005 | Gelb |
| Acid Violet 50 | 50325 | Violett |
| Acid Black 2 | 50420 | Schwarz |
| Pigment Violet 23 | 51319 | Violett |
| 1,2-Dioxyanthrachinon, Calcium-Sluminiumkomplex | 58000 | Rot |
| 3-Oxypyren-5,8,10-sulfosäure | 59040 | Grün |
| 1-Hydroxy-4-N-phenyl-aminoanthrachinon | 60724 | Violett |
| 1-Hydroxy-4-(4'-methylphenylamino)-anthrachinon | 60725 | Violett |
| Acid Violet 23 | 60730 | Violett |
| 1,4-Di(4'-methyl-phenylamino)-anthrachinon | 61565 | Grün |
| 1,4-Bis-(o-sulfo-p-toluidino)-anthrachinon | 61570 | Grün |
| Acid Blue 80 | 61585 | Blau |
| Acid Blue 62 | 62045 | Blau |
| N,N'-Dihydro-1,2,1',2'-anthrachinonazin | 69800 | Blau |
| Vat Blue 6; Pigment Blue 64 | 69825 | Blau |
| Vat Orange 7 | 71105 | orange |
| Indigo | 73000 | Blau |
| Indigo-disulfosäure | 73015 | Blau |
| 4,4'-Dimethyl-6,6'-dichlorthioindigo | 73360 | Rot |
| 5,5'Dichlor-7,7'-dimethylthioindigo | 73385 | violett |
| Quinacridone Violet 19 | 73900 | violett |
| Pigment Red 122 | 73915 | Rot |
| Pigment Blue 16 | 74100 | blau |
| Phthalocyanine | 74160 | blau |
| Direct Blue 86 | 74180 | blau |
| Chlorierte Phthalocyanine | 74260 | grün |
| Natural Yellow 6, 19; Natural Red 1 | 75100 | gelb |
| Bixin, Nor-Bixin | 75120 | orange |
| Lycopin | 75125 | gelb |
| trans-alpha-, bet- bzw. gamma-Carotin | 75130 | orange |
| Keto- und/oder Hydroxylderivate des Carotins | 75135 | gelb |

(fortgesetzt)

| Chemische oder sonstige Bezeichnung | CIN | Farbe |
|---|---|---|
| Guanin oder Perlglanzmittel | 75170 | weiß |
| 1,7-Bis-(4-hydroxy-3-methoxyphenyl)1,6-heptadien-3,5-dion | 75300 | gelb |
| Komplexsalz (Na, Al, Ca) der Karminsäure | 75470 | Rot |
| Chlorophyll a und b; Kupferverbindungen der Chlorophylle und Chlorophylline | 75810 | grün |
| Aluminium | 77000 | weiß |
| Tonerdehydrat | 77002 | weiß |
| Wasserhaltige Aluminiumsilikate | 77004 | weiß |
| Ultramarin | 77007 | blau |
| Pigment Red 101 und 102 | 77015 | Rot |
| Bariumsulfat | 77120 | weiß |
| Bismutoxychlorid und seine Gemische mit Glimmer | 77163 | weiß |
| Calciumcarbonat | 77220 | weiß |
| Calciumsulfat | 77231 | weiß |
| Kohlenstoff | 77266 | schwarz |
| Pigment Black 9 | 77267 | schwarz |
| Carbo medicinalis vegetabilis | 77268:1 | schwarz |
| Chromoxid | 77288 | grün |
| Chromoxid, wasserhaltig | 77278 | grün |
| Pigment Blue 28, Pigment Green 14 | 77346 | grün |
| Pigment Metal 2 | 77400 | braun |
| Gold | 77480 | braun |
| Eisenoxide und -hydoxide | 77489 | orange |
| Eisenoxid | 77491 | rot |
| Eisenoxidhydrat | 77492 | gelb |
| Eisenoxid | 77499 | schwarz |
| Mischungen aus Eisen(II)- und Eisen(III)-hexacyahoferrat | 77510 | blau |
| Pigment White 18 | 77713 | weiß |
| Mangananimoniumdiphosphat | 77742 | violett |
| Manganphosphat; $Mn_3(PO_4)_2 \cdot 7\ H_2O$ | 77745 | rot |
| Silber | 77820 | weiß |
| Titandioxid und seine Gemische mit Glimmer | 77891 | weiß |
| Zinkoxid | 77947 | weiß |
| 6,7-Dimethyl-9-(1'-D-ribityl)-isoalloxazin, Lactoflavin | | gelb |
| Zuckerkulör | | braun |
| Capsanthin, Capsorubin | | orange |
| Betanin | | rot |
| Benzopyryliumsalzem, Anthocyane | | rot |
| Aluminium-, Zink-, Magnesium- und Calciumstearat | | weiß |
| Bromthymolblau | | blau |

Es kann ferner günstig sein, als Farbstoff eine oder mehrerer Substanzen aus der folgenden Gruppe zu wählen:

2,4-Dihydroxyazobenzol, 1-(2'-Chlor-4'-nitro-1'phenylazo)-2-hydroxynaphthalin, Ceresrot, 2-(4-Sulfo-1-naphthylazo)-1-naphthol-4-sulfosäure, Calciumsalz der 2-Hydroxy-1,2'-azonaphthalin-1'-sulfonsäure, Calcium- und Bariumsalze der 1-(2-Sulfo-4-methyl-1-phenylazo)-2-naphthylcarbonsäure, Calciumsalz der 1-(2-Sulfo-1-naphthylazo)-2-hydroxynaphthalin-3-carbonsäure, Aluminiumsalz der 1-(4-Sulfo-1-phenylazo)-2-naphthol-6-sulfosäure, Aluminiumsalz der 1-(4-Sulfo-1-naphthylazo)-2-naphthol-3,6-disulfosäure, 1-(4-Sulfo-1-naphthylazo)-2-naphthol-6,8-disulfonsäure, Aluminiumsalz der 4-(4-Sulfo-1-phenylazo)-2-(4-sulfophenyl)-5-hydroxy-pyrazolon-3-carbonsäure, Aluminium- und Zirkoniumsalze von 4,5-Dibromfluorescein, Aluminium- und Zirkoniumsalze von 2,4,5,7-Tetrabromfluorescein, 3',4',5',6'-Tetrachlor-2,4,5,7-tetrabromfluorescein und sein Aluminiumsalz, Aluminiumsalz von 2,4,5,7-Tetraiodfluorescein, Aluminiumsalz der Chinophthalon-disulfosäure, Aluminiumsalz der Indigo-disulfosäure, rotes und schwarzes Eisenoxid (CIN: 77 491 (rot) und 77 499 (schwarz)), Eisenoxidhydrat (CIN: 77492), Manganammoniumdiphosphat und Titandioxid.

Ferner vorteilhaft sind öllösliche Naturfarbstoffe, wie z. B. Paprikaextrakt, ß-Carotin oder Cochenille.

Vorteilhaft im Sinne der vorliegenden Erfindung sind ferner Gelcrèmes mit einem Gehalt an Perlglanzpigmenten. Bevorzugt sind insbesondere die im folgenden aufgelisteten Arten von Perlglanzpigmenten:

1. Natürliche Perlglanzpigmente, wie z. B.

    6. "Fischsilber" (Guanin/Hypoxanthin-Mischkristalle aus Fischschuppen) und
    7. "Perlmutt" (vermahlene Muschelschalen)

2. Monokristalline Perlglanzpigmente wie z. B. Bismuthoxychlorid (BiOCl)
3. Schicht-Substrat Pigmente: z. B. Glimmer / Metalloxid

Basis für Perlglanzpigmente sind beispielsweise pulverförmige Pigmente oder Ricinusöldispersionen von Bismutoxychlorid und/oder Titandioxid sowie Bismutoxychlorid und/oder Titandioxid auf Glimmer. Insbesondere vorteilhaft ist z. B. das unter der CIN 77163 aufgelistete Glanzpigment.

Vorteilhaft sind ferner beispielsweise die folgenden Perlglanzpigmentarten auf Basis von Glimmer/Metalloxid:

| **Gruppe** | **Belegung/Schichtdicke** | **Farbe** |
|---|---|---|
| **Silberweiße Perlglanzpigmente** | $TiO_2$: 40-60 nm | silber |
| **Interferenzpigmente** | $TiO_2$: 60-80 nm | gelb |
| | $TiO_2$: 80-100 nm | rot |
| | $TiO_2$: 100-140 nm | blau |
| | $TiO_2$: 120-160 nm | grün |
| **Farbglanzpigmente** | $Fe_2O_3$ | bronze |
| | $Fe_2O_3$ | kupfer |
| | $Fe_2O_3$ | rot |
| | $Fe_2O_3$ | rotviolett |
| | $Fe_2O_3$ | rotgrün |
| | $Fe_2O_3$ | schwarz |
| **Kombinationspigmente** | $TiO_2$ / $Fe_2O_3$ | Goldtöne |
| | $TiO_2$ / $Cr_2O_3$ | grün |
| | $TiO_2$ / Berliner Blau | tiefblau |

Besonders bevorzugt sind z. B. die von der Firma Merck unter den Handelsnamen Timiron, Colorona oder Dichrona erhältlichen Perlglanzpigmente.

Die Liste der genannten Perlglanzpigmente soll selbstverständlich nicht limitierend sein. Im Sinne der vorliegenden Erfindung vorteilhafte Perlglanzpigmente sind auf zahlreichen, an sich bekannten Wegen erhältlich. Beispielsweise lassen sich auch andere Substrate außer Glimmer mit weiteren Metalloxiden beschichten, wie z. B. Silica und dergleichen mehr. Vorteilhaft sind z. B. mit $TiO_2$ und $Fe_2O_3$ beschichtete $SiO_2$-Partikel ("Ronaspheren"), die von der Firma Merck vertrieben werden und sich besonders für die optische Reduktion feiner Fältchen eignen.

Es kann darüber hinaus von Vorteil sein, gänzlich auf ein Substrat wie Glimmer zu verzichten. Besonders bevorzugt sind Perlglanzpigmente, welche unter der Verwendung von $SiO_2$ hergestellt werden. Solche Pigmente , die auch zusätzlich gonichromatische Effekte haben können, sind z. B. unter dem Handelsnamen Sicopearl Fantastico bei der Firma BASF erhältlich.

Weiterhin vorteilhaft können Pigmente der Firma Engelhard / Mearl auf Basis von Calcium Natrium Borosilikat, die mit Titandioxid beschichtet sind, eingesetzt werden. Diese sind unter dem Namen Reflecks erhältlich. Sie weisen durch ihre Partikelgröße von 40-80 μm zusätzlich zu der Farbe einen Glitzereffekt auf.

Besonders vorteilhaft sind ferner auch Effektpigmente, welche unter der Handelsbezeichnung Metasomes Standard / Glitter in verschiedenen Farben (yellow, red, green, blue) von der Firma Flora Tech erhältlich sind. Die Glitterpartikel liegen hierbei in Gemischen mit verschiedenen Hilfs- und Farbstoffen (wie beispielsweise den Farbstoffen mit den Colour Index (CI) Nummern 19140, 77007, 77289, 77491) vor.

Die Farbstoffe und Pigmente können sowohl einzeln als auch im Gemisch vorliegen sowie gegenseitig miteinander beschichtet sein, wobei durch unterschiedliche Beschichtungsdicken im allgemeinen verschiedene Farbeffekte hervorgerufen werden. Die Gesamtmenge der Farbstoffe und farbgebenden Pigmente wird vorteilhaft aus dem Bereich von z. B. 0,1 Gew.% bis 30 Gew.%, vorzugsweise von 0,5 bis 15 Gew.%, insbesondere von 1,0 bis 10 Gew.% gewählt, jeweils bezogen auf das Gesamtgewicht der Zubereitungen.

Alle Verbindungen oder Komponenten, die in den Zubereitungen verwendet werden können, sind entweder bekannt und käuflich erwerbbar oder können nach bekannten Verfahren synthetisiert werden.

Die eine oder die mehreren Verbindungen der Formel I können in der üblichen Weise in kosmetische oder dermatologische Zubereitungen eingearbeitet werden. Geeignet sind Zubereitungen für eine äußerliche Anwendung, beispielsweise als Creme, Lotion, Gel, oder als Lösung, die auf die Haut aufgesprüht werden kann. Für eine innerliche Anwendung sind Darreichungsformeln wie Kapseln, Dragees, Pulver, Tabletten-Lösungen oder Lösungen geeignet.

Als Anwendungsform der erfindungsgemäßen Zubereitungen seien z.B. genannt: Lösungen, Suspensionen, Emulsionen, PIT-Emulsionen, Pasten, Salben, Gele, Cremes, Lotionen, Puder, Seifen, tensidhaltige Reinigungspräparate, Öle, Aerosole und Sprays. Weitere Anwendungsformen sind z.B. Sticks, Shampoos und Duschbäder. Der Zubereitung können beliebige übliche Trägerstoffe, Hilfsstoffe und gegebenenfalls weitere Wirkstoffe zugesetzt werden.

Vorzuziehende Hilfsstoffe stammen aus der Gruppe der Konservierungsstoffe, Antioxidantien, Stabilisatoren, Lösungsvermittler, Vitamine, Färbemittel, Geruchsverbesserer.

Salben, Pasten, Cremes und Gele können die üblichen Trägerstoffe enthalten, z.B. tierische und pflanzliche Fette, Wachse, Paraffine, Stärke, Traganth, Cellulosederivate, Polyethylenglykole, Silicone, Bentonite, Kieselsäure, Talkum und Zinkoxid oder Gemische dieser Stoffe.

Puder und Sprays können die üblichen Trägerstoffe enthalten, z.B. Milchzucker, Talkum, Kieselsäure, Aluminiumhydroxid, Calciumsilikat und Polyamid-Pulver oder Gemische dieser Stoffe. Sprays können zusätzlich die üblichen Treibmittel, z.B. Chlorfluorkohlenwasserstoffe, Propan/Butan oder Dimethylether, enthalten.

Lösungen und Emulsionen können die üblichen Trägerstoffe wie Lösungsmittel, Lösungsvermittler und Emulgatoren, z.B. Wasser, Ethanol, Isopropanol, Ethylcarbonat, Ethlyacetat, Benzylalkohol, Benzylbenzoat, Propylenglykol, 1,3-Butylglykol, Öle, insbesondere Baumwollsaatöl, Erdnussöl, Maiskeimöl, Olivenöl, Rizinusöl und Sesamöl, Glycerinfettsäureester, Polyethylenglykole und Fettsäureester des Sorbitans oder Gemische dieser Stoffe enthalten.

Suspensionen können die üblichen Trägerstoffe wie flüssige Verdünnungsmittel, z.B. Wasser, Ethanol oder Propylenglykol, Suspendiermittel, z.B. ethoxylierte Isostearylalkohole, Polyoxyethylensorbitester und Polyoxyethylensorbitanester, mikrokristalline Cellulose, Aluminiummetahydroxid, Bentonit, Agar-Agar und Traganth oder Gemische dieser Stoffe enthalten.

Seifen können die üblichen Trägerstoffe wie Alkalisalze von Fettsäuren, Salze von Fettsäurehalbestern, Fettsäureeiweißhydrolysaten, Isothionate, Lanolin, Fettalkohol, Pflanzenöle, Pflanzenextrakte, Glycerin, Zucker oder Gemische dieser Stoffe enthalten.

Tensidhaltige Reinigungsprodukte können die üblichen Trägerstoffe wie Salze von Fettalkoholsulfaten, Fettalkoholethersulfaten, Sulfobernsteinsäurehalbestern, Fettsäureeiweißhydrolysaten, Isothionate, Imidazoliniumderivate, Methyltaurate, Sarkosinate, Fettsäureamidethersulfate, Alkylamidobetaine, Fettalkohole, Fettsäureglyceride, Fettsäurediethanolamide, pflanzliche und synthetische Öle, Lanolinderivate, ethoxylierte Glycerinfettsäureester oder Gemische dieser Stoffe enthalten.

Gesichts- und Körperöle können die üblichen Trägerstoffe wie synthetische Öle wie Fettsäureester, Fettalkohole, Silikonöle, natürliche Öle wie Pflanzenöle und ölige Pflanzenauszüge, Paraffinöle, Lanolinöle oder Gemische dieser Stoffe enthalten.

Weitere typische kosmetische Anwendungsformen sind auch Lippenstifte, Lippenpflegestifte, Mascara, Eyeliner, Lidschatten, Rouge, Puder-, Emulsions- und Wachs-Make up sowie Sonnenschutz-, Prä-Sun- und After-Sun-Präparate.

Zu den bevorzugten erfindungsgemäßen Zubereitungsformen gehören insbesondere Emulsionen.

Erfindungsgemäße Emulsionen sind vorteilhaft und enthalten z. B. die genannten Fette, Öle, Wachse und anderen Fettkörper, sowie Wasser und einen Emulgator, wie er üblicherweise für einen solchen Typ der Zubereitung verwendet wird.

Die Lipidphase kann vorteilhaft gewählt werden aus folgender Substanzgruppe:

- Mineralöle, Mineralwachse
- Öle, wie Triglyceride der Caprin- oder der Caprylsäure, ferner natürliche Öle wie z. B. Rizinusöl;
- Fette, Wachse und andere natürliche und synthetische Fettkörper, vorzugsweise Ester von Fettsäuren mit Alkoholen

niedriger C-Zahl, z.B. mit Isopropanol, Propylenglykol oder Glycerin, oder Ester von Fett-Ikoholen mit Alkansäuren niedriger C-Zahl oder mit Fettsäuren;

- Silikonöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigtem und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäure und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexaldecylstearat, 2-Octyldodecylpalmitat, Oleyloleat, Oleylerucat, Erucyloleat, Erucylerucat sowie synthetische, halbsynthetische und natürliche Gemische solcher Ester, z. B. Jojobaöl.

Ferner kann die Ölphase vorteilhaft gewählt werden aus der Gruppe der verzweigten und unverzweigten Kohlenwasserstoffe und -wachse, der Silikonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12-18 C-Atomen. Die Fettsäuretriglyceride können beispielsweise vorteilhaft gewählt werden aus der Gruppe der synthetischen, halbsynthetischen und natürlichen Öle, z. B. Olivenöl, Sonnenblumenöl, Sojaöl, Erdnussöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, $C_{12-15}$-Alkylbenzoat, Capryl-Caprinsäure-triglycerid, Dicaprylether.

Besonders vorteilhaft sind Mischungen aus $C_{12-15}$-Alkylbenzoat und 2-Ethylhexylisostearat, Mischungen aus $C_{12-15}$-Alkylbenzoat und Isotridecylisononanoat sowie Mischungen aus $C_{12-15}$-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann auch die Ölphase ferner einen Gehalt an cyclischen oder linearan Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die wässrige Phase der erfindungsgemäßen Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z. B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Insbesondere werden Gemisch der vorstehend genannten Lösemittel verwendet. Bei alkoholischen Lösemitteln kann Wasser ein weiterer Bestandteil sein.

Erfindungsgemäße Emulsionen sind vorteilhaft und enthalten z. B. die genannten Fette, Öle, Wachse und anderen Fettkörper, sowie Wasser und einen Emulgator, wie er üblicherweise für einen solchen Typ der Formuierung verwendet wird.

In einer bevorzugten Ausführungsform enthalten die erfindungsgemäßen Zubereitungen hydrophile Tenside.

Die hydrophilen Tenside werden bevorzugt gewählt aus der Gruppe der Alkylglucoside, der Acyllactylate, der Betaine sowie der Cocoamphoacetate.

Die Alkylglucoside werden ihrerseits vorteilhaft gewählt aus der Gruppe der Alkylglucoside, welche sich durch die Strukturformel

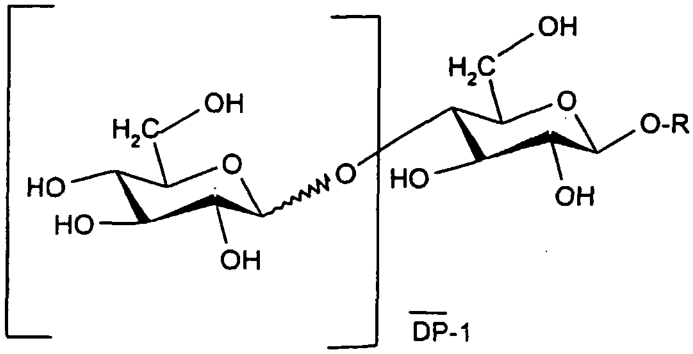

auszeichnen, wobei R einen verzweigten oder unverzweigten Alkylrest mit 4 bis 24 Kohlenstoffatomen darstellt und wobei $\overline{DP}$ einen mittleren Glucosylierungsgrad von bis zu 2 bedeutet.

Der Wert $\overline{DP}$ repräsentiert den Glucosidierungsgrad der erfindungsgemäß verwendeten Alkylglucoside und ist definiert als

$$\overline{DP} = \frac{p_1}{100} \cdot 1 + \frac{p_2}{100} \cdot 2 + \frac{p_3}{100} \cdot 3 + ... = \Sigma \frac{p_i}{100} \cdot i$$

Dabei stellen $p_1$, $p_2$, $p_3$ ... bzw. $p_i$ den Anteil der einfach, zweifach dreifach ... i-fach glucosylierten Produkte in Gewichtsprozenten dar. Erfindungemäß vorteilhaft werden Produkte mit Glucosylierungsgraden von 1-2, insbesondere vorteilhaft von 1, 1 bis 1,5, ganz besonders vorteilhaft von 1,2-1,4, insbesondere von 1,3 gewählt.

Der Wert DP trägt den Umstande Rechnung, dass Alkylglucoside herstellungsedingt in der Regel Gemische aus Mono- und Oligoglucosiden darstellen. Erfindungsgemäß vorteilhaft ist ein relativ hoher Gehalt an Monoglucosiden, typischerweise in der Größenordnung von 40-70 Gew.-%.

Erfindungsgemäß besonders vorteilhaft verwendete Alkylglylcoside werden gewählt aus der Gruppe Octylglucopyranosid, Nonylglucopyranosid, Decylglucopyranosid, Undecylglucopyranosid, Dodecylglucopyranosid, Tetradecylglucopyranosid und Hexadecylglucopyranosid.

Es ist ebenfalls von Vorteil, natürliche oder synthetische Roh- und Hilfsstoffe bzw. Gemische einzusetzen, welche sich durch einen wirksamen Gehalt an den erfindungsgemäß verwendeten Wirkstoffen auszeichnen, beispielsweise Plantaren® 1200 (Henkel KGaA), Oramix® NS 10 (Seppic).

Die Acyllactylate werden ihrerseits vorteilhaft gewählt aus der Gruppe der Substanzen, welche sich durch die Strukturformel

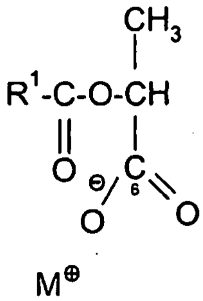

auszeichnen, wobei $R^1$ einen verzweigten oder unverzweigten Alkylrest mit 1 bis 30 Kohlenstoffatomen bedeutet und $M^+$ aus der Gruppe der Alkaliionen sowie der Gruppe der mit einer oder mehreren Alkyl- und/oder mit einer oder mehreren Hydroxyalkylresten substituierten Ammoniumionen gewählt wird bzw. dem halben Äquivalent eines Erdalkalions entspricht.

Vorteilhaft ist beispielsweise Natriumisostearyllactylat, beispielsweise das Produkt Pathionic® ISL von der Gesellschaft American Ingredients Company.

Die Betaine werden vorteilhaft gewählt aus der Gruppe der Substanzen, welche sich durch die Strukturformel

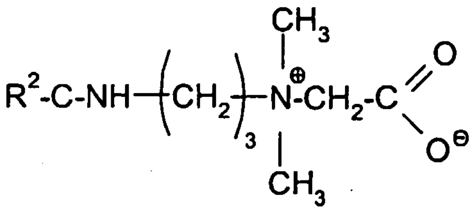

auszeichnen, wobei $R^2$ einen verzweigten oder unverzeigten Alkylrest mit 1 bis 30 Kohlenstoffatomen bedeutet.

Insbesondere vorteilhaft bedeutet $R^2$ einen verzweigten oder unverzweigten Alkylrest mit 6 bis 12 Kohlenstoffatomen.

Vorteilhaft ist beispielsweise Capramidopropylbetain, beispielsweise das Produkt Tego® Betain 810 von der Gesellschaft Th. Goldschmidt AG.

Als erfindungsgemäß vorteilhaftes Cocoamphoacetat wird beispielsweise Natriumcocoamphoacetat gewählt, wie es unter der Bezeichnung Miranol® Ultra C32 von der Gesellschaft Miranol Chemical Corp. erhältlich ist.

Die erfindungsgemäßen Zubereitungen sind vorteilhaft dadurch gekennzeichnet, dass das oder die hydrophilen Tenside in Konzentrationen von 0,01-20 Gew.-% bevorzugt 0,05-10 Gew.-%, besonders bevorzugt 0,1-5 Gew.-%, jeweils bezogen auf das Gesamtgewicht der Zusammensetzung, vorliegt oder vorliegen.

Zu Anwendung werden die erfindungsgemäßen kosmetischen und dermatologischen Zubereitungen in der für Kosmetika üblichen Weise aufdie Haut und/oder die Haare in ausreichender Menge aufgebracht.

Erfindungsgemäße kosmetische und dermatologische Zubereitungen können in verschiedenen Formen vorliegen. So können sie z. B. eine Lösung, eine wasserfreie Zubereitung, eine Emulsion oder Mikroemulsion vom Typ Wasser-in-Öl (W/O) oder vom Typ Öl-in-Wasser (O/W), eine multiple Emulsion, beispielsweise vom Typ Waser-in-Öl-in-Wasser (W/O/W), ein Gel, einen festen Stift, eine Salbe oder auch ein Aerosol darstellen. Es ist auch vorteilhaft, Ectoine in verkapselter Form darzureichen, z. B. in Kollagenmatrices und anderen üblichen Verkapselungsmaterialien, z. B. als Celluloseverkapselungen, in Gelatine, Wachsmatrices oder liposomal verkapselt. Insbesondere Wachsmatrices wie sie in der DE-OS 43 08 282 beschrieben werden, haben sich als günstig herausgestellt. Bevorzugt werden Emulsionen. O/W-Emulsinen werden besonders bevorzugt. Emulsionen, W/O-Emulsionen und O/W-Emulsionen sind in üblicher Weise erhältlich.

Als Emulgatoren können beispielsweise die bekannten W/O- und O/W-Emulgatoren verwendet werden. Es ist vorteilhaft, weitere übliche Co-emulgatoren in den erfindungsgemäßen bevorzugten O/W-Emulsionen zu verwenden.

Erfindungsgemäß vorteilhaft werden als Co-Emulgatoren beispielsweise O/W-Emulgatoren gewählt, vornehmlich aus der Gruppe der Substanzen mit HLB-Werten von 11-16, ganz besonders vorteilhaft mit HLB-Werten von 14,5-15,5, sofern die O/W-Emulgatoren gesättigte Reste R und R' aufweisen. Weisen die O/W-Emulgatoren ungesättigte Reste R und/oder R' auf, oder liegen Isoalkylderivate vor, so kann der bevorzugte HLB-Wert solcher Emulgatoren auch niedriger oder darüber liegen.

Es ist von Vorteil, die Fettalkoholethoxylate aus der Gruppe der ethoxylierten Stearylalkhole, Cetylalkohole, Cetylstearylalkohole (Cetearylalkohole) zu wählen. Insbesondere bevorzugt sind: Polyethylenglycol(13)stearylether (Steareth-13), Polyethylenglycol(14)stearylether (Steareth-14), Polyethylenglycol(15)stearylether (Steareth-15), Polyethylenglycol(16)stearylether (Steareth-16), Polyethylenglycol(17)-stearylether (Steareth-17),Polyethylenglycol(18) stearylether (Steareth-18), Polyethylenglycol(19)stearylether (Steareth-19), Polyethylenglycol(20)-stearylether (Steareth-20), Polyethylenglycol(12)isostearylether (Isosteareth-12), Polyethylenglycol(13)isostearylether (Isosteareth-13), Polyethylenglycol(14)isostearylether (Isosteareth-14), Polyethylenglycol(15)isostearylether (Isosteareth-15), Polyethylenglycol(16)-isostearylether (Isosteareth-16), Polyethylenglycol(17)isostearylether (Isosteareth-17), Polyethylenglycol(18)isostearylether (Isosteareth-18), Polyethylenglycol(19)isostearylether (Isosteareth-19), Polyethylenglycol(20)isostearylether (Isosteareth-20), Polyethylenglycol(13)cetylether (Ceteth-13), Polyethylenglycol(14)cetylether (Ceteth-14), Polyethylenglycol(15)cetylether (Ceteth-15), Polyethylenglycol(16)cetylether (Ceteth-16), Polyethylenglycol(17)cetylether (Ceteth-17), Polyethylenglycol(18)-cetylether (Ceteth-18), Polyethylenglycol(19)cetylether (Ceteth-19), Polyethylen-glycol(20)cetylether (Ceteth-20), Polyethylenglycol(13)isocetylether (Isoceteth-13), Polyethylenglycol(14)isocetylether (Isoceteth-14), Polyethylenglycol(15)isocetylether (Isoceteth-15), Polyethylenglycol(16)isocetylether (Isoceteth-16), Polyethylenglycol(17)-isocetylether (Isoceteth-17), Polyethylenglycol(18)isocetylether (Isoceteth-18), Polyethylenglycol (19)isocetylether (Isoceteth-19), Polyethylenglycol(20)isocetylether (Isoceteth-20), Polyethylenglycol(12)oleylether (Oleth-12), Polyethylenglycol(13)oleylether (Oleth-13), Polyethylenglycol(14)oleylether (Oleth-14), Polyethylenglycol (15)oleylether (Oleth-15), Polyethylenglycol(12)laurylether (Laureth-12), Polyethylenglycol(12)-isolaurylether (Isolaureth-12), Polyethylenglycol(13)cetylstearylether (Ceteareth-13), Polyethylenglycol(14)cetylstearylether (Ceteareth-14), Polyethylenglycol(15)cetylstearylether (Ceteareth-15), Polyethylenglycol(16)cetylstearylether (Ceteareth-16), Polyethylenglycol(17)-cetylstearylether (Ceteareth-17), Polyethylenglycol(18)cetylstearylether (Ceteareth-18), Polyethylenglycol (19)cetylstearylether (Ceteareth-19), Polyethylenglycol(20)cetylstearylether (Ceteareth-20).

Es ist ferner von Vorteil, die Fettsäureethoxylate ausfolgender Gruppe zu wählen:

Polyethylenglycol(20)stearat, Polyethylenglycol(21 )stearat, Polyethylenglycol(22)stearat, Polyethylenglycol(23)stearat, Polyethylenglycol(24)stearat, Polyethylenglycol(25)stearat, Polyethylenglycol(12)isostearat, Polyethylenglycol(13)isostearat, Polyethylenglycol(14)isostearat, Polyethylenglycol(15)isostearat, Polyethylenglycol(16)isostearat, Polyethylenglycol(17)isostearat, Polyethylenglycol(18)isostearat, Polyethylenglycol(19)isostearat, Polyethylenglycol(20)isostearat, Polyethylenglycol(21)isostearat, Polyethylenglycol(22)isostearat, Polyethylenglycol(23)isostearat, Polyethylenglycol(24)isostearat, Polyethylenglycol(25)isostearat, Polyethylenglycol(12)oleat, Polyethylenglycol(13)oleat, Polyethylenglycol(14)oleat, Polyethylenglycol(1 5)oleat, Polyethylenglycol(16)oleat, Polyethylenglycol(17)oleat, Polyethylenglycol(18)oleat, Polyethylenglycol(19)oleat, Polyethylenglycol(20)oleat,

Als ethoxylierte Alkylethercarbonsäure bzw. deren Salz kann vorteilhaft das Natriumlaureth-11-carboxylat verwendet werden. Als Alkylethersulfat kann Natrium Laureth1-4sulfat vorteilhaft verwendet werden. Als ethoxyliertes Cholesterinderivat kann vorteilhaft Polyethylenglycol(30)Cholesterylether verwendet werden. Auch Polyethylenglycol(25) Sojasterol hat sich bewährt. Als ethoxylierte Triglyceride können vorteilhaft die Polyethylenglycol(60) Evening Primrose Glycerides verwendet werden (Evening Primrose = Nachtkerze).

Weiterhin ist von Vorteil, die Polyethylenglycolglycerinfettsäureester aus der Gruppe Polyethylenglycol(20) glyceryllaurat, Polyethylenglycol(21)glyceryllaurat, Polyethylenglycol(22)glyceryllaurat, Polyethylenglycol(23)glyceryllaurat, Polyethylenglycol(6)glycerylcaprat/cprinat, Polyethylenglycol-(20)glyceryloleat, Polyethylenglycol(20)glycerylisostearat, Polyethylenglycol(18)glyceryloleat(cocoat zu wählen.

Es ist ebenfalls günstig, die Sorbitanester aus der Gruppe Polyethylenglycol(20)sorbitanmonolaurat, Polyethylenglycol(20)sorbitanmonostearat, Polyethylenglycol(20)sorbitanmonoisostearat, Polyethylenglycol(20)sorbitanmonopalmitat, Polyethylenglycol(20)sorbitanmonooleat zu wählen.

Als fakultative, dennoch erfindungsgemäß gegebenenfalls vorteilhafte W/O-Emulgatoren können eingesetzt werden:

Fettalkohole mit 8 bis 30 Kohlenstoffatomen, Monoglycerinester gesättigter und/oder ungesättigter, verzweigter und/oder unverzweigter Alkancarbonsäuren einer Kettenlänge von 8 bis 24, insbesondere 12-18 C-Atome, Diglycerinester gesättigter und/oder ungesättigter, verzweigter und/oder unverzweigter Alkancarbonsäuren einer Kettenlänge von 8 bis 24, insbesondere 12-18 C-Atomen, Monoglycerinether gesättigter und/oder ungesättigter, verzweigter und/oder unverzweigter Alkohole einer Kettenlänge von 8 bis 24, insbesondere 12-18 C-Atomen, Diglycerinether gesättigter und/ oder ungesättigter, verzweigter und/oder unverzweigter Alkhole einer Kettenlänge von 8 bis 24, insbesondere 12-18 C-Atomen, Propylenglycolester gesättigter und/oder ungesättigter, verzweigter und/oder unverzweigter Alkancarbonsäuren einer Kettenlänge von 8 bis 24, insbesondere 12-18 C-Atomen sowie Sorbitanester gesättigter und/oder ungesättigter, verzweigter und/oder unverzweigter Alkancarbonsäuren einer Kettenlänge von 8 bis 24, insbesondere 12-18 C-Atomen.

Insbesondere vorteilhafte W/O-Emulgatoren sind Glycerylmonostearat, Glycerylmonoisostearat, Glycerylmonomyristat, Glycerylmonooleat, Diglycerylmonostearat, Diglycerylmonoisostearat, Propylenglycolmonostearat, Propylenglycolmonoisostearat, Propylenglycolmonocaprylat, Propylenglycolmonolaurat, Sorbitanmonoisostearat, Sorbitanmonolaurat, Sorbitanmonocaprylat, Sorbitanmonoisooleat, Saccharosedistearat, Cetylalkohol, Stearylalkohol, Arachidylalkohol, Behenylalkohol, Isobehenylalkohol, Selachylalkohol, Chimylalkohol, Polyethylenglycol(2)stearylether (Steareth-2), Glycerylmonolaurat, Glycerylmonocaprinat, Glycerylmonocaprylat.

Erfindungsgemäß bevorzugte Zubereitungen eignen sich besonders zum Schutz menschlicher Haut gegen Alterungsprozesse sowie vor oxidativem Stress, d.h. gegen Schädigungen durch Radikale, wie sie z.B. durch Sonneneinstrahlung, Wärme oder andere Einflüsse erzeugt werden. Dabei liegt sie in verschiedenen, für diese Anwendung üblicherweise verwendeten Darreichungsformen vor. So kann sie insbesondere als Lotion oder Emulsion, wie als Creme oder Milch (O/W, W/O, O/W/O, W/O/W), in Form ölig-alkoholischer, ölig-wässriger oder wässrigalkoholischer Gele bzw. Lösungen, als feste Stifte vorliegen oder als Aerosol konfektioniert sein.

Die Zubereitung kann kosmetische Adjuvantien enthalten, welche in dieser Art von Zubereitungen üblicherweise verwendet werden, wie z.B. Verdickungsmittel, weichmachende Mittel, Befeuchtungsmittel, grenzflächenaktive Mittel, Emulgatoren, Konservierungsmittel, Mittel gegen Schaumbildung, Parfums, Wachse, Lanolin, Treibmittel, Farbstoffe und/oder Pigmente, welche das Mittel selbst oder die Haut färben, und andere in der Kosmetik gewöhnlich verwendete Ingredienzien.

Man kann als Dispersions- bzw. Solubilisierungsmittel ein Öl, Wachs oder sonstigen Fettkörper, einen niedrigen Monoalkohol oder ein niedriges Polyol oder Mischungen davon verwenden. Zu den besonders bevorzugten Monoalkoholen oder Polyolen zählen Ethanol, i-Propanol, Propylenglykol, Glycerin und Sorbit.

Eine bevorzugte Ausführungsform der Erfindung ist eine Emulsion, welche als Schutzcreme oder -Milch vorliegt und außer der oder den Verbindungen der Formel I bzw. Formel II beispielsweise Fettalkohole, Fettsäuren, Fettsäureester, insbesondere Triglyceride von Fettsäuren, Lanolin, natürliche und synthetische Öle oder Wachse und Emulgatoren in Anwesenheit von Wasser enthält.

Weitere bevorzugte Ausführungsformen stellen ölige Lotionen auf Basis von natürlichen oder synthetischen Ölen und Wachsen, Lanolin, Fettsäureestern, insbesondere Triglyceriden von Fettsäuren, oder öligalkoholische Lotionen auf Basis eines Niedrigalkohols, wie Ethanol, oder eines Glycerols, wie Propylenglykol, und/oder eines Polyols, wie Glycerin, und Ölen, Wachsen und Fettsäureestern, wie Triglyceriden von Fettsäuren, dar.

Die erfindungsgemäße Zubereitung kann auch als alkoholisches Gel vorliegen, welches einen oder mehrere Niedrigalkohole oder -polyole, wie Ethanol, Propylenglykol oder Glycerin, und ein Verdickungsmittel, wie Kieselerde umfaßt. Die ölig-alkoholischen Gele enthalten außerdem natürliches oder synthetisches Öl oder Wachs.

Die festen Stifte bestehen aus natürlichen oder synthetischen Wachsen und Ölen, Fettalkoholen, Fettsäuren, Fettsäureestern, Lanolin und anderen Fettkörpern.

Ist eine Zubereitung als Aerosol konfektioniert, verwendet man in der Regel die üblichen Treibmittel, wie Alkane, Fluoralkane und Chlorfluoralkane.

Die kosmetische Zubereitung kann auch zum Schutz der Haare gegen fotochemische Schäden verwendet werden, um Veränderungen von Farbnuancen, ein Entfärben oder Schäden mechanischer Art zu verhindern. In diesem Fall erfolgt geeignet eine Konfektionierung als Shampoo, Lotion, Gel oder Emulsion zum Ausspülen, wobei die jeweilige Zubereitung vor oder nach dem Shamponieren, vor oder nach dem Färben oder Entfärben bzw. vor oder nach der Dauerwelle aufgetragen wird. Es kann auch eine Zubereitung als Lotion oder Gel zum Frisieren und Behandeln, als Lotion oder Gel zum Bürsten oder Legen einer Wasserwelle, als Haarlack, Dauerwellenmittel, Färbe- oder Entfärbemittel der Haare gewählt werden. Die Zubereitung mit Lichtschutzeigenschaften kann außer der oder den Verbindungen der Formel I bzw. Formel II verschiedene, in diesem Mitteltyp verwendete Adjuvantien enthalten, wie Grenzflächen aktive Mittel, Verdickungsmittel, Polymere, weichmachende Mittel, Konservierungsmittel, Schaumstabilisatoren, Elektrolyte, organische Lösungsmittel, Silikonderivate, Öle, Wachse, Antifettmittel, Farbstoffe und/oder Pigmente, die das Mittel selbst oder die Haare färben oder andere für die Haarpflege üblicherweise verwendete Ingredienzien.

Weitere Gegenstände der vorliegenden Erfindung sind ein Verfahren zur Herstellung einer Zubereitung, welches dadurch gekennzeichnet ist, dass mindestens eine Verbindung der Formel I bzw. Formel II mit Resten wie oben beschrieben mit einem kosmetisch oder dermatologisch oder für Nahrungsmittel geeigneten Träger vermischt wird, und die Verwendung einer Verbindung der Formel I bzw. Formel 11 zur Herstellung einer Zubereitung.

Die erfindungsgemäßen Zubereitungen können dabei mit Hilfe von Techniken hergestellt werden, die dem Fachmann wohl bekannt sind.

Das Vermischen kann ein Lösen, Emulgieren oder Dispergieren der Verbindung gemäß Formel I bzw. Formel II in dem Träger zur Folge haben.

Es wurde auch festgestellt, dass Verbindungen der Formel I bzw. Formel II stabilisierend auf die Zubereitung wirken können. Bei der Verwendung in entsprechenden Produkten bleiben diese daher auch länger stabil und verändern ihr Aussehen nicht. Insbesondere bleibt auch bei längendauernder Anwendung bzw. längerer Lagerung die Wirksamkeit der Inhaltsstoffe, z.B. Vitamine, erhalten. Dies ist unter anderem besonders vorteilhaft bei Zusammensetzungen zum Schutz der Haut gegen die Einwirkung von UV-Strahlen, da diese Kosmetika besonders hohen Belastungen durch die UV-Strahlung ausgesetzt sind.

Weitere Gegenstände der vorliegenden Erfindung sind ein Verfahren zur Herstellung einer Zubereitung, welches dadurch gekennzeichnet ist, dass mindestens eine Verbindung der Formel I mit Resten wie oben beschrieben mit einem kosmetisch oder dermatologisch geeigneten Träger vermischt wird, und die Verwendung einer Verbindung der Formel I zur Herstellung einer Zubereitung mit Lichtschutzeigenschaften.

Die erfindungsgemäßen Zubereitungen können dabei mit Hilfe von Techniken hergestellt werden, die dem Fachmann wohl bekannt sind.

Das Vermischen kann ein Lösen, Emulgieren oder Dispergieren der Verbindung gemäß Formel I in dem Träger zur Folge haben.

In einem erfindungsgemäß bevorzugten Verfahren wird die Verbindung nach Formel II hergestellt durch Umsetzung einer 2-Hydroxyacetophenon-Verbindung mit einer Lithiumverbindung und anschließend mit einer Ketoverbindung. Die Herstellung dieser Verbindungen ist dabei in EP1400579 A2 bzw. WO2002060889 A1 beschrieben, deren diesbezügliche Offenbarung ausdrücklich auch zum Offenbarungsgehalt der vorliegenden Anmeldung gehört.

Die Komplex-Verbindungen der Formel I lassen sich herstellen, indem Verbindungen der Formel II mit Cyclodextrinen in Lösung, vorzugsweise bei erhöhter Temperatur, umgesetzt werden. Ein entsprechendes Verfahren ist ein weiterer Gegenstand der vorliegenden Erfindung.

Es hat sich gezeigt, dass in bevorzugten Ausführungsformen der vorliegenden Erfindung Komplexe mit etwa 1 oder 2 mol Cyclodextrin pro mol Flavonoid der Formel II die erfindungsgemäßen Anforderungen in besonderer Weise erfüllen. Daher ist es erfindungsgemäß bevorzugt, wenn in Formel I o gleich 1 ist und p in dem Bereich 1 bis 3, vorzugsweise wenn p in dem Bereich 1,7 bis 2,1 ist.

Entsprechende Verbindungen können hergestellt werden, wenn das Cyclodextrin im Überschuß oder genau im Molverhältnis 1:1 bzw. 2 : 1 bezogen auf das Flavonoid eingesetzt wird.

Es wurde auch festgestellt, dass Verbindungen der Formel I stabilisierend auf die Zubereitung wirken können. Bei der Verwendung in entsprechenden Produkten bleiben diese daher auch länger stabil und verändern ihr Aussehen nicht. Insbesondere bleibt auch bei längerdauernder Anwendung bzw. längerer Lagerung die Wirksamkeit der Inhaltsstoffe, z.B. Vitamine, erhalten. Dies ist unter anderem besonders vorteilhaft bei Zusammensetzungen zum Schutz der Haut gegen die Einwirkung von UV-Strahlen, da diese Kosmetika besonders hohen Belastungen durch die UV-Strahlung ausgesetzt sind.

Die positiven Wirkungen von Verbindungen der Formel I ergeben deren besondere Eignung zur Verwendung in kosmetischen oder pharmazeutischen Zubereitungen.

Ebenso positiv sind die Eigenschaften von Verbindungen mit der Formel I zu werten für eine Verwendung in Nahrungsmitteln oder als Nahrungsergänzungsmittel oder als "functional food". Die weiteren zu Nahrungsmitteln ausgeführten Erläuterungen gelten sinngemäß auch für Nahrungsergänzungsmittel und für "functional food".

Die Nahrungsmittel, die nach der vorliegenden Erfindung mit einer oder mehreren Verbindungen der Formel I angereichert werden können, umfassen alle Materialien, die für den Verzehr durch Tiere oder für den Verzehr durch Menschen geeignet sind, beispielsweise Vitamine und Provitamine davon, Fette, Mineralien oder Aminosäuren ". (Die Nahrungsmittel können fest sein aber auch flüssig, also als Getränk vorliegen). Weitere Gegenstände der vorliegenden Erfindung sind dementsprechend die Verwendung einer Verbindung nach Formel I als Nahrungsmittelzusatz für die human- oder Tierernährung sowie Zubereitungen, die Nahrungsmittel oder Nahrungsergänzungsmittel sind und entsprechende Träger enthalten.

Nahrungsmittel, die nach der vorliegenden Erfindung mit einer oder mehreren Verbindungen der Formel I angereichert werden können, sind beispielsweise auch Nahrungsmittel, die aus einer einzigen natürlichen Quelle stammen, wie z.B. Zucker, ungesüßter Saft, Nektar oder Püree von einer einzigen Pflanzenspezies, wie z.B. ungesüßter Apfelsaft (z.B. auch eine Mischung verschiedener Sorten Apfelsaft), Grapefruitsaft, Orangensaft, Apfelkompott, Aprikosennektar, Tomatensaft, Tomatensoße, Tomatenpüree usw. Weitere Beispiele für Nahrungsmittel, die nach der vorliegenden Erfindung mit einer oder mehreren Verbindungen der Formel I angereichert werden können, sind Korn oder Getreide einer einzigen Pflanzenspezies und Materialien, die aus derartigen Pflanzenspezies hergestellt werden, wie z.B. Getreidesirup, Roggenmehl, Weizenmehl oder Haferkleie. Auch Mischungen von derartigen Nahrungsmitteln sind geeignet, um nach der vorliegenden Erfindung mit einer oder mehreren Verbindungen der Formel I angereichert zu werden, beispielsweise Multivitaminpräparate, Mineralstoffmischungen oder gezuckerter Saft. Als weitere Beispiele für Nahrungsmittel, die nach der vorliegenden Erfindung mit einer oder mehreren Verbindungen der Formel I angereichert werden können, seien Nahrungsmittelzubereitungen, beispielsweise zubereitete Cerealien, Gebäck, Mischgetränke, speziell für Kinder zubereitete Nahrungsmittel, wie Joghurt, Diätnahrungsmittel, kalorienarme Nahrungsmittel oder Tierfutter, genannt.

Die Nahrungsmittel, die nach der vorliegenden Erfindung mit einer oder mehreren Verbindungen der Formel I angereichert werden können, umfassen somit alle genießbaren Kombinationen von Kohlehydraten, Lipiden, Proteinen, anorganischen Elementen, Spurenelementen, Vitaminen, Wasser oder aktiven Metaboliten von Pflanzen und Tieren.

Die Nahrungsmittel, die nach der vorliegenden Erfindung mit einer oder mehreren Verbindungen der Formel I angereichert werden können, werden vorzugsweise oral angewendet, z.B. in Form von Speisen, Pillen, Tabletten, Kapseln, Pulver, Sirup, Lösungen oder Suspensionen.

Die mit einer oder mehreren Verbindungen der Formel I angereicherten erfindungsgemäßen Nahrungsmittel können mit Hilfe von Techniken hergestellt werden, die dem Fachmann wohl bekannt sind.

Durch ihre Wirkung als Antioxidationsmittel bzw. als Radikalfänger eignen sich Verbindungen der Formel I auch als Arzneimittelinhaltsstoff. Sie wirken dabei unterstützend oder substituierend zu natürlichen Mechanismen, welche Radikale im Körper abfangen. Die Verbindungen der Formel I können in ihrer Wirkung teilweise mit Radikalfängern wie Vitamin C verglichen werden. Verbindungen der Formel I können beispielsweise zur vorbeugenden Behandlungen von Entzündungen und Allergien der Haut sowie in bestimmten Fällen zur Verhütung bestimmter Krebsarten verwendet werden. Insbesondere eignen sich Verbindungen der Formel I zur Herstellung eines Arzneimittels zur Behandlung von Entzündungen, Allergien und Irritationen, insbesondere der Haut. Ferner können Arzneimittel hergestellt werden in einer Wirkung als Venentonikum, als Mittel zur Erhöhung der Festigkeit von Blutkapillaren, als Hemmstoff für Cuperose, als Hemmstoff chemischer, physikalischer oder aktinischer Erytheme, als Mittel zur Behandlung empfindlicher Haut, als Dekongestionsmittel, als Entwässerungsmittel, als Mittel zum Schlankmachen, als Antifaltenmittel, als Stimulatoren der Synthese von Komponenten der extrazellulären Matrix, als stärkendes Mittel zur Verbesserung der Häutelastizität und als Antialterungsmittel. Weiter zeigen in diesem Zusammenhang bevorzugte Verbindungen der Formel I antiallergische

und antiinflammatorische und antiirritative Wirkungen. Sie eignen sich daher zur Herstellung von Arzneimitteln zur Behandlung von Entzündungen oder allergischen Reaktionen.

Im folgenden wird die Erfindung anhand von Beispielen näher erläutert. die Erfindung ist im gesamten beanspruchten Bereich ausführbar und nicht auf die hier genannten Beispiele beschränkt.

## Beispiele

### Beispiel A - Herstellung eines 7,8-Dihydroxyflavon - beta-Cyclodextrin-Komplexes

1 g 7,8-Dihydroxyflavon (3,93 mmol) wird in 200 mL Methanol gelöst. 6 g 2-Hydroxypropyl-beta-cyclodextrin (3,93 mmol) wird in der Lösung gelöst. Die Lösung wird eine Nacht gerührt. Das Methanol wird aus der Lösung abdestilliert. Der Rückstand wird im Vakuum bis zur Trockne eingeengt und der verbleibende Feststoff bei 40°C im Vakuum nachgetrocknet. Der Feststoff wird mit 100 mL Wasser gewaschen und erneut getrocknet. Es werden 5,4 g gelber Komplex erhalten. Laut HPLC liegen hier 40 Moleküle Cyclodextrin pro Molekül Flavonoid vor. Löslichkeit des 7,8-Dihydroxyflavon-Cyclodextrin Komplexes: In 1 L Wasser wird Komplex enthaltend 5 g Flavonoid gelöst ohne Sättigung zu erreichen.

### Variante zu Beispiel A:

500 mg 7,8-Dihydroxyflavon (1,97 mmol) wird in 100 mL Methanol gelöst. 30 mg 2-Hydroxypropyl-beta-cyclodextrin (0,020 mmol) wird in der Lösung gelöst. Die Lösung wird eine Nacht gerührt. Das Methanol wird aus der Lösung abdestilliert. Der Rückstand wird im Vakuum bis zur Trockne eingeengt und der verbleibende Feststoff bei 40°C im Vakuum nachgetrocknet. Der Feststoff wird mit 100 mL Wasser gewaschen und erneut getrocknet. Es werden 20 mg gelber Komplex erhalten. Laut HPLC liegen hier 8 Moleküle Cyclodextrin pro-Molekül Flavonoid vor. Löslichkeit des 7,8-Dihydroxyflavon-Cyclodextrin Komplexes: In 1 L Wasser wird Komplex enthaltend 5 g Flavonoid gelöst ohne Sättigung zu erreichen

### Beispiel B - Herstellung eines 7,8-Dihydroxyflavon - gamma-Cyclodextrin-Komplexes

(3,1g, 4.72mmol) Hydroxypropyl-gamma-cyclodextrin (Fa. Aldrich; 2'-Hydroxypropyl-cyclooctaamylose; CAS.-Nr. 128446-34-4) werden in 70 ml Wasser vorgelegt und auf 50°C erwärmt. 0,3 g 7,8-Dihydroxyflavon werden in 60 ml Ethanol gelöst und in die vorgelegte Lösung zugetropft. Die Lösung wird eine Nacht bei 50°C gerührt. Das Ethanol wird aus der Lösung abdestilliert. Der Rückstand wird im Vakuum bis zur Trockne eingeengt und der verbleibende Feststoff bei 40°C und 200 mbar über Nacht nachgetrocknet.

50mg des 7,8-Dihydroxyflavon wurden komplexiert wiedergefunden (Ausbeute: 17%).

Löslichkeit des 7,8-Dihydroxyflavon-gamma-Cyclodextrin Komplexes:

In 1 ml Wasser wird 0,3 g Komplex gelöst, ohne Sättigung zu erreichen. Das entspricht einer Löslichkeit bezogen auf reines 7,8-Dihydroxyhydroxyflavon von mindestens 12 g/l.

Analog Beispiel A bzw. B werden Cyclodextrin-Komplexe von

- 6,3',4'-Trihydroxyflavon,
- 5,6,7-Trihydroxyflavon
- 7,8,3',4'-Tetrahydroxyflavon
- 7,8-Dihydroxy-3',4'-dimethoxyflavone
- 6,7-Dihydroxyflavone
- 6,7-Dihydroxy-3',4',5'-trimethoxyflavone

erhalten.

### Beispiel C: Zubereitungen

Im folgenden werden beispielhaft Rezepturen für kosmetische Zubereitungen angegeben, die erfindungsgemäße Verbindungen enthalten. In den folgenden Beispielrezepturen wird das angegebene Flavon jeweils als Hydroxypropyl-gamma-cyclodextrin-Komplex gemäß Beispiel B bzw. Hydroxypropyl-beta-cyclodextrin-Komplex gemäß Beispiel A eingesetzt. Im übrigen sind die INCI-Bezeichnungen der handelsüblichen Verbindungen angegeben.

UV-Pearl , OMC steht für die Zubereitung mit der INCI-Bezeichnung: Water (for EU: Aqua), Ethylhexyl Methoxycinnamate, Silica, PVP, Chlorphenesin, BHT; diese Zubereitung ist im handel unter der Bezeichnung Eusolex®UV Pearl™OMC von der Merck KGaA, Darmstadt erhältlich.

Die anderen in den Tabellen angegebenen UV-Pearl sind jeweils analog zusammengesetzt, wobei OMC gegen die

angegebenen UV-Filter ausgetauscht wurde.

Tabelle 1 W/O-Emulsionen (Zahlen in Gew.-%)

| | 1-1 | 1-2 | 1-3 | 1-4 | 1-5 | 1-6 | 1-7 | 1-8 | 1-9 | 1-10 |
|---|---|---|---|---|---|---|---|---|---|---|
| Titanium dioxide | | 2 | 5 | | | | | | | 3 |
| 6,3',4'-Trihydroxyflavon | 5 | 3 | 2 | 1 | 2 | | | | 1 | 1 |
| 5,6,7-Trihydroxyflavon | | | | | | 1 | 2 | 1 | | |
| Zinc oxide | | | | | | | | 5 | 2 | |
| UV-Pearl , OMC | 30 | 15 | 15 | 15 | 15 | 15 | 15 | 15 | 15 | 15 |
| Polyglyceryl-3-Dimerate | 3 | 3 | 3 | 3 | 3 | 3 | 3 | 3 | 3 | 3 |
| Cera Alba | 0,3 | 0,3 | 0,3 | 0,3 | 0,3 | 0,3 | 0,3 | 0,3 | 0,3 | 0,3 |
| Hydrogenated Castor Oil | 0,2 | 0,2 | 0,2 | 0,2 | 0,2 | 0,2 | 0,2 | 0,2 | 0,2 | 0,2 |
| Paraffinium Liquidum | 7 | 7 | 7 | 7 | 7 | 7 | 7 | 7 | 7 | 7 |
| Caprylic/Capric Triglyceride | 7 | 7 | 7 | 7 | 7 | 7 | 7 | 7 | 7 | 7 |
| Hexyl Laurate | 4 | 4 | 4 | 4 | 4 | 4 | 4 | 4 | 4 | 4 |
| PVP/Eicosene Copolymer | 2 | 2 | 2 | 2 | 2 | 2 | 2 | 2 | 2 | 2 |
| Propylene Glycol | 4 | 4 | 4 | 4 | 4 | 4 | 4 | 4 | 4 | 4 |
| Magnesium Sulfate | 0,6 | 0,6 | 0,6 | 0,6 | 0,6 | 0,6 | 0,6 | 0,6 | 0,6 | 0,6 |
| Tocopherol | 0,5 | 0,5 | 0,5 | 0,5 | 0,5 | 0,5 | 0,5 | 0,5 | 0,5 | 0,5 |
| Tocopheryl Acetate | 0,5 | 0,5 | 0,5 | 0,5 | 0,5 | 0,5 | 0,5 | 0,5 | 0,5 | 0,5 |
| Cyclomethicone | 0,5 | 0,5 | 0,5 | 0,5 | 0,5 | 0,5 | 0,5 | 0,5 | 0,5 | 0,5 |
| Propylparabene | 0,05 | 0,05 | 0,05 | 0,05 | 0,05 | 0,05 | 0,05 | 0,05 | 0,05 | 0,05 |
| Methylparabene | 0,15 | 0,15 | 0,15 | 0,15 | 0,15 | 0,15 | 0,15 | 0,15 | 0,15 | 0,15 |
| Water | ad 100 | ad 100 | ad 100 | ad 100 | ad 100 | ad 100 | ad 100 | ad 100 | ad 100 | ad 100 |

Tabelle 1 (Fortsetzung)

| | 1-11 | 1-12 | 1-13 | 1-14 | 1-15 | 1-16 | 1-17 | 1-18 |
|---|---|---|---|---|---|---|---|---|
| Titanium dioxide | 3 | | 2 | | 3 | | 2 | 5 |
| Benzylidene malonate polysiloxane | | 1 | 0,5 | | | | | |
| Methylene Bis-Benztriazolyl Tetramethylbutylphenol | 1 | 1 | 0,5 | | | | | |
| 5,6,7-Trihydroxyflavon | 5 | 3 | 2 | 5 | 1 | 3 | 7 | 2 |
| Polyglyceryl-3-Dimerate | 3 | 3 | 3 | 3 | | | | |
| Cera Alba | 0,3 | 0,3 | 0,3 | 0,3 | 2 | 2 | 2 | 2 |
| Hydrogenated Castor Oil | 0,2 | 0,2 | 0,2 | 0,2 | | | | |
| Paraffinium Liquidum | 7 | 7 | 7 | 7 | | | | |
| Caprylic/Capric Triglyceride | 7 | 7 | 7 | 7 | | | | |
| Hexyl Laurate | 4 | 4 | 4 | 4 | | | | |
| PVP/Eicosene Copolymer | 2 | 2 | 2 | 2 | | | | |
| Propylene Glycol | 4 | 4 | 4 | 4 | | | | |
| Magnesium Sulfate | 0,6 | 0,6 | 0,6 | 0,6 | | | | |
| Tocopherol | 0,5 | 0,5 | 0,5 | 0,5 | | | | |
| Tocopheryl Acetate | 0,5 | 0,5 | 0,5 | 0,5 | 1 | 1 | 1 | 1 |
| Cyclomethicone | 0,5 | 0,5 | 0,5 | 0,5 | | | | |
| Propylparabene | 0,05 | 0,05 | 0,05 | 0,05 | 0,05 | 0,05 | 0,05 | 0,05 |
| Methylparabene | 0,15 | 0,15 | 0,15 | 0,15 | 0,15 | 0,15 | 0,15 | 0,15 |
| Dicocoyl Pentyerythrityl Citrate (and) Sorbitan Sesquioleate (and) Cera Alba (and) Aluminium Stearate | | | | | 6 | 6 | 6 | 6 |
| PEG-7 Hydrogenated Castor Oil | | | | | 1 | 1 | 1 | 1 |
| Zinc Stearate | | | | | 2 | 2 | 2 | 2 |
| Oleyl Erucate | | | | | 6 | 6 | 6 | 6 |
| Decyl Oleate | | | | | 6 | 6 | 6 | 6 |
| Dimethicone | | | | | 5 | 5 | 5 | 5 |
| Tromethamine | | | | | 1 | 1 | 1 | 1 |
| Glycerin | | | | | 5 | 5 | 5 | 5 |
| Allantoin | | | | | 0,2 | 0,2 | 0,2 | 0,2 |
| water | ad 100 | ad 100 | ad 100 | ad 100 | ad 100 | ad 100 | ad 100 | ad 100 |

Tabelle 1 (Fortsetzung)

| | 1-19 | 1-20 | 1-21 | 1-22 | 1-23 | 1-24 | 1-25 | 1-26 | 1-27 | 1-28 | 1-29 |
|---|---|---|---|---|---|---|---|---|---|---|---|
| Titanium dioxide | | 2 | 5 | | | | | | | 3 | 3 |
| Benzylidene malonate polysiloxane | | | | 1 | | | | | 1 | 1 | |
| Methylene Bis-Benztriazolyl Tetramethylbutylphenol | | | | | | 1 | 2 | 1 | | | 1 |
| Zinc oxide | | | | | | | | 5 | 2 | | |
| 6,3',4'-Trihydroxyflavon | 5 | 5 | 5 | 5 | 7 | 5 | 5 | 5 | 5 | 5 | 8 |
| UV-Pearl , OCR | | 10 | | | | | | | | | 5 |
| UV-Pearl, EthylhexylDimethylPABA | | | 10 | | | | | | | | |
| UV-Pearl, Homosalate | | | | 10 | | | | | | | |
| UV-Pearl, Ethylhexyl salicylate | | | | | 10 | | | | | | |
| UV-Pearl , OMC, BP-3 | | | | | | 10 | | | | | |
| UV-Pearl , OCR, BP-3 | | | | | | | 10 | | | | |
| UV-Pearl, Ethylhexyl Dimethyl PABA, BP-3 | | | | | | | | 10 | | | |
| UV-Pearl, Homosalate, BP-3 | | | | | | | | | 10 | | |
| UV-Pearl, Ethylhexyl salicylate, BP-3 | | | | | | | | | | 10 | |
| BMDBM | | | | | | | | | | | 2 |
| UV-Pearl OMC, 4-Methylbenzylidene Camphor | 25 | | | | | | | | | | |
| Polyglyceryl-3-Dimerate | 3 | 3 | 3 | 3 | 3 | 3 | 3 | 3 | 3 | 3 | 3 |
| Cera Alba | 0,3 | 0,3 | 0,3 | 0,3 | 0,3 | 0,3 | 0,3 | 0,3 | 0,3 | 0,3 | 0,3 |
| Hydrogenated Castor Oil | 0,2 | 0,2 | 0,2 | 0,2 | 0,2 | 0,2 | 0,2 | 0,2 | 0,2 | 0,2 | 0,2 |
| Paraffinium Liquidum | 7 | 7 | 7 | 7 | 7 | 7 | 7 | 7 | 7 | 7 | 7 |
| Caprylic/Capric Triglyceride | 7 | 7 | 7 | 7 | 7 | 7 | 7 | 7 | 7 | 7 | 7 |
| Hexyl Laurate | 4 | 4 | 4 | 4 | 4 | 4 | 4 | 4 | 4 | 4 | 4 |
| PVP/Eicosene Copolymer | 2 | 2 | 2 | 2 | 2 | 2 | 2 | 2 | 2 | 2 | 2 |
| Propylene Glycol | 4 | 4 | 4 | 4 | 4 | 4 | 4 | 4 | 4 | 4 | 4 |
| Magnesium Sulfate | 0,6 | 0,6 | 0,6 | 0,6 | 0,6 | 0,6 | 0,6 | 0,6 | 0,6 | 0,6 | 0,6 |
| Tocopherol | 0,5 | 0,5 | 0,5 | 0,5 | 0,5 | 0,5 | 0,5 | 0,5 | 0,5 | 0,5 | 0,5 |
| Tocopheryl Acetate | 0,5 | 0,5 | 0,5 | 0,5 | 0,5 | 0,5 | 0,5 | 0,5 | 0,5 | 0,5 | 0,5 |
| Cyclomethicone | 0,5 | 0,5 | 0,5 | 0,5 | 0,5 | 0,5 | 0,5 | 0,5 | 0,5 | 0,5 | 0,5 |
| Propylparabene | 0,05 | 0,05 | 0,05 | 0,05 | 0,05 | 0,05 | 0,05 | 0,05 | 0,05 | 0,05 | 0,05 |
| Methylparabene | 0,15 | 0,15 | 0,15 | 0,15 | 0,15 | 0,15 | 0,15 | 0,15 | 0,15 | 0,15 | 0,15 |
| Water | ad 100 | | | | | | | | | | |

Tabelle 2: O/W-Emulsionen, Zahlen in Gew.-%

| | 2-1 | 2-2 | 2-3 | 2-4 | 2-5 | 2-6 | 2-7 | 2-8 | 2-9 | 2-10 |
|---|---|---|---|---|---|---|---|---|---|---|
| Titanium dioxide | | 2 | 5 | | | | | | | 3 |
| Methylene Bis-Benztriazolyl Tetramethylbutylphenol | | | | | | 1 | 2 | 1 | | |
| 7,8,3',4'-Tetrahydroxyflavon | 1 | 3 | | 2 | 2 | 5 | | 5 | 2 | 1 |
| 6,3',4'-Trihydroxyflavon | 5 | 5 | 5 | 5 | 5 | 5 | 5 | 5 | 5 | 5 |
| 5,6,7-Trihydroxyflavon | 1 | 5 | 4 | | 6 | | 7 | | 2 | 1 |
| 4-Methylbenzyliden Camphor | 2 | | 3 | | 4 | | 3 | | 2 | |
| BMDBM | 1 | 3 | | 3 | 3 | | 3 | 3 | 3 | |
| Stearyl Alcohol (and) Steareth-7 (and) Steareth-10 | 3 | 3 | 3 | 3 | 3 | 3 | 3 | 3 | 3 | 3 |
| Glyceryl Stearate (and) Ceteth-20 | 3 | 3 | 3 | 3 | 3 | 3 | 3 | 3 | 3 | 3 |
| Glyceryl Stearate | 3 | 3 | 3 | 3 | 3 | 3 | 3 | 3 | 3 | 3 |
| Microwax | 1 | 1 | 1 | 1 | 1 | 1 | 1 | 1 | 1 | 1 |
| Cetearyl Octanoate | 11,5 | 11,5 | 11,5 | 11,5 | 11,5 | 11,5 | 11,5 | 11,5 | 11,5 | 11,5 |
| Caprylic/Capric Triglyceride | 6 | 6 | 6 | 6 | 6 | 6 | 6 | 6 | 6 | 6 |
| Oleyl Oleate | 6 | 6 | 6 | 6 | 6 | 6 | 6 | 6 | 6 | 6 |
| Propylene Glycol | 4 | 4 | 4 | 4 | 4 | 4 | 4 | 4 | 4 | 4 |
| Glyceryl Stearate SE | | | | | | | | | | |
| Stearic Acid | | | | | | | | | | |
| Persea Gratissima | | | | | | | | | | |
| Propylparabene | 0,05 | 0,05 | 0,05 | 0,05 | 0,05 | 0,05 | 0,05 | 0,05 | 0,05 | 0,05 |
| Methylparabene | 0,15 | 0,15 | 0,15 | 0,15 | 0,15 | 0,15 | 0,15 | 0,15 | 0,15 | 0,15 |
| Tromethamine | | | 1,8 | | | | | | | |
| Glycerin | | | | | | | | | | |
| Water | ad 100 | ad 100 | ad 100 | ad 100 | ad 100 | ad 100 | ad 100 | ad 100 | ad 100 | ad 100 |
| | | | | | | | | | | |

Tabelle 2 (Fortsetzung)

| | 2-11 | 2-12 | 2-13 | 2-14 | 2-15 | 2-16 | 2-17 | 2-18 |
|---|---|---|---|---|---|---|---|---|
| Titanium dioxide | 3 | | 2 | | | | 2 | 5 |
| Benzylidene malonate polysiloxane | | 1 | 0,5 | | | | | |
| Methylene Bis-Benztriazolyl Tetramethylbutylphenol | 1 | 1 | 0,5 | | | | | |
| 7,8,3',4'-Tetrahydroxyflavon | 1 | 3 | | 2 | | 5 | | 5 |
| 5,6,7-Trihydroxyflavon | 5 | 5 | 5 | 5 | 5 | 5 | 5 | 5 |
| 6,3',4'-Trihydroxyflavon | 1 | 5 | 4 | | 6 | | 7 | |
| Zinc oxide | | | 2 | | | | | |
| UV-Pearl , OMC | 15 | 15 | 15 | 30 | 30 | 30 | 15 | 15 |
| 4-Methylbenzyliden Camphor | | | | 3 | | | | |
| BMDBM | | | | 1 | | | | |
| Phenylbenzimidazole Sulfonic Acid | | | | | 4 | | | |
| Stearyl Alcohol (and) Steareth-7 (and) Steareth-10 | 3 | 3 | 3 | 3 | | | | |
| Glyceryl Stearate (and) Ceteth-20 | 3 | 3 | 3 | 3 | | | | |
| Glyceryl Stearate | 3 | 3 | 3 | 3 | | | | |
| Microwax | 1 | 1 | 1 | 1 | | | | |
| Cetearyl Octanoate | 11,5 | 11,5 | 11,5 | 11,5 | | | | |
| Caprylic/Capric Triglyceride | 6 | 6 | 6 | 6 | 14 | 14 | 14 | 14 |
| Oleyl Oleate | 6 | 6 | 6 | 6 | | | | |
| Propylene Glycol | 4 | 4 | 4 | 4 | | | | |
| Glyceryl Stearate SE | | | | | 6 | 6 | 6 | 6 |
| Stearic Acid | | | | | 2 | 2 | 2 | 2 |
| Persea Gratissima | | | | | 8 | 8 | 8 | 8 |
| Propylparabene | 0,05 | 0,05 | 0,05 | 0,05 | 0,05 | 0,05 | 0,05 | 0,05 |
| Methylparabene | 0,15 | 0,15 | 0,15 | 0,15 | 0,15 | 0,15 | 0,15 | 0,15 |
| Tromethamine | | | | | 1,8 | | | |
| Glycerin | | | | | 3 | 3 | 3 | 3 |
| Water | ad 100 | ad 100 | ad 100 | ad 100 | ad 100 | ad 100 | ad 100 | ad 100 |

Tabelle 2 (Fortsetzung)

| | 2-19 | 2-20 | 2-21 | 2-22 | 2-23 | 2-24 | 2-25 | 2-26 | 2-27 | 2-28 |
|---|---|---|---|---|---|---|---|---|---|---|
| Titanium dioxide | | | | | | | 3 | 3 | | 2 |
| Benzylidene malonate polysiloxane | 1 | 2 | | | | 1 | 1 | | 1 | 0,5 |
| 7,8,3',4'-Tetrahydroxyflavon | | | | 1 | 2 | | | | 1 | 1 |
| 5,6,7-Trihydroxyflavon | 1 | 3 | | 2 | | 5 | | 5 | 2 | |
| 6,3',4'-Trihydroxyflavon | 5 | 5 | 5 | 5 | 5 | 5 | 5 | 5 | 5 | 5 |
| Methylene Bis-Benztriazolyl Tetramethylbutylphenol | | | 1 | 2 | 1 | | | 1 | 1 | 0,5 |
| Zinc oxide | | | | | 5 | 2 | | | | 2 |
| UV-Pearl , OMC | 15 | 15 | 15 | 15 | 15 | 15 | 15 | 15 | 15 | 15 |
| Caprylic/Capric Triglyceride | 14 | 14 | 14 | 14 | 14 | 14 | 14 | 14 | 14 | 14 |
| Oleyl Oleate | | | | | | | | | | |
| Propylene Glycol | | | | | | | | | | |
| Glyceryl Stearate SE | 6 | 6 | 6 | 6 | 6 | 6 | 6 | 6 | 6 | 6 |
| Stearic Acid | 2 | 2 | 2 | 2 | 2 | 2 | 2 | 2 | 2 | 2 |
| Persea Gratissima | 8 | 8 | 8 | 8 | 8 | 8 | 8 | 8 | 8 | 8 |
| Propylparabene | 0,05 | 0,05 | 0,05 | 0,05 | 0,05 | 0,05 | 0,05 | 0,05 | 0,05 | 0,05 |
| Methylparabene | 0,15 | 0,15 | 0,15 | 0,15 | 0,15 | 0,15 | 0,15 | 0,15 | 0,15 | 0,15 |
| Glyceryl Stearate, Ceteareth-20, Ceteareth-10, Cetearyl Alcohol, Cetyl Palmitate | | | | | | | | | | |
| Ceteareth-30 | | | | | | | | | | |
| Dicaprylyl Ether | | | | | | | | | | |
| Hexyldecanol, Hexyldexyllaurate | | | | | | | | | | |
| Cocoglycerides | | | | | | | | | | |
| Tromethamine | | | | | | | | | | |
| Glycerin | 3 | 3 | 3 | 3 | 3 | 3 | 3 | 3 | 3 | 3 |
| Water | ad 100 | ad 100 | ad 100 | ad 100 | ad 100 | ad 100 | ad 100 | ad 100 | ad 100 | ad 100 |

Tabelle 3: Gele, Zahlen in Gew.-%

| | 3-1 | 3-2 | 3-3 | 3-4 | 3-5 | 3-6 | 3-7 | 3-8 | 3-9 | 3-10 |
|---|---|---|---|---|---|---|---|---|---|---|
| A = aqueaous gel | | | | | | | | | | |
| Titanium dioxide | | 2 | 5 | | | | | | | 3 |
| 5,6,7-Trihydroxyflavon | | | | 1 | 2 | | | | 1 | 1 |
| 7,8,3',4'-Tetrahydroxyflavon | 1 | 3 | | 2 | | 5 | | 5 | 2 | |
| 6,3',4'-Trihydroxyflavon | 5 | 5 | 5 | 5 | 5 | 5 | 5 | 5 | 5 | 5 |
| Benzylidene malonate polysiloxane | | | 1 | 1 | 2 | | | | 1 | 1 |
| Methylene Bis-Benztriazolyl Tetramethylbutylphenol | | 1 | | | | 1 | 2 | 1 | | |
| Zinc oxide | | | | 2 | | | | 5 | 2 | |
| UV-Pearl , Ethylhexyl Mehtoxycinnamat | 30 | 15 | 15 | 15 | 15 | 15 | 15 | 15 | 15 | 15 |
| 4-Methylbenzyliden Camphor | | | | | 2 | | | | | |
| Butylmethoxydibenzoylmethane | | 1 | | | | | | | | |
| Phenylbenzimidazole Sulfonic Acid | | | 4 | | | | | | | |
| Prunus Dulcis | 5 | 5 | 5 | 5 | 5 | 5 | 5 | 5 | 5 | 5 |
| Tocopheryl Acetate | 0,5 | 0,5 | 0,5 | 0,5 | 0,5 | 0,5 | 0,5 | 0,5 | 0,5 | 0,5 |
| Caprylic/Capric Triglyceride | 3 | 3 | 3 | 3 | 3 | 3 | 3 | 3 | 3 | 3 |
| Octyldodecanol | 2 | 2 | 2 | 2 | 2 | 2 | 2 | 2 | 2 | 2 |
| Decyl Oleate | 2 | 2 | 2 | 2 | 2 | 2 | 2 | 2 | 2 | 2 |
| PEG-8 (and) Tocopherol (and) Ascorbyl Palmitate (and) Ascorbic Acid (and) Citric Acid | 0,05 | 0,05 | 0,05 | 0,05 | 0,05 | 0,05 | 0,05 | 0,05 | 0,05 | 0,05 |
| Sorbitol | 4 | 4 | 4 | 4 | 4 | 4 | 4 | 4 | 4 | 4 |
| Polyacrylamide (and) C13-14 Isoparaffin (and) Laureth-7 | 3 | 3 | 3 | 3 | 3 | 3 | 3 | 3 | 3 | 3 |
| Propylparabene | 0,05 | 0,05 | 0,05 | 0,05 | 0,05 | 0,05 | 0,05 | 0,05 | 0,05 | 0,05 |
| Methylparabene | 0,15 | 0,15 | 0,15 | 0,15 | 0,15 | 0,15 | 0,15 | 0,15 | 0,15 | 0,15 |
| Tromethamine | | | 1,8 | | | | | | | |
| Water | ad 100 | ad 100 | ad 100 | ad 100 | ad 100 | ad 100 | ad 100 | ad 100 | ad 100 | ad 100 |

Tabelle 3 (Fortsetzung)

| | 3-11 | 3-12 | 3-13 | 3-14 | 3-15 | 3-16 | 3-17 | 3-18 |
|---|---|---|---|---|---|---|---|---|
| a = aqueous gel | | | | a | a | a | a | a |
| Titanium dioxide | 3 | | 2 | | | | | |
| Benzylidene malonate polysiloxane | | 1 | 0,5 | 1 | 2 | | | |
| Methylene Bis-Benztriazolyl Tetramethylbutylphenol | 1 | 1 | 0,5 | | | 1 | 2 | 1 |
| 7,8,3',4'-Tetrahydroxyflavon | 1 | 3 | | 2 | 2 | 5 | | 5 |
| 5,6,7-Trihydroxyflavon | 5 | 5 | 5 | 5 | 5 | 5 | 5 | 5 |
| 6,3',4'-Trihydroxyflavon | 1 | 5 | 4 | | 6 | | 7 | |
| Zinc oxide | | | 2 | | | | | |
| UV-Pearl , Ethylhexyl Mehtoxycinnamat | 15 | 15 | 15 | 15 | 15 | 15 | 15 | 15 |
| Prunus Dulcis | 5 | 5 | 5 | | | | | |
| Tocopheryl Acetate | 0,5 | 0,5 | 0,5 | | | | | |
| Caprylic/Capric Triglyceride | 3 | 3 | 3 | | | | | |
| Octyldodecanol | 2 | 2 | 2 | | | | | |
| Decyl Oleate | 2 | 2 | 2 | | | | | |
| PEG-8 (and) Tocopherol (and) Ascorbyl Palmitate (and) Ascorbic Acid (and) Citric Acid | 0,05 | 0,05 | 0,05 | | | | | |
| Sorbitol | 4 | 4 | 4 | 5 | 5 | 5 | 5 | 5 |
| Polyacrylamide (and) C13-14 Isoparaffin (and) Laureth-7 | 3 | 3 | 3 | | | | | |
| Carbomer | | | | 1,5 | 1,5 | 1,5 | 1,5 | 1,5 |
| Propylparabene | 0,05 | 0,05 | 0,05 | | | | | |
| Methylparabene | 0,15 | 0,15 | 0,15 | 0,15 | 0,15 | 0,15 | 0,15 | 0,15 |
| Allantoin | | | | 0,2 | 0,2 | 0,2 | 0,2 | 0,2 |
| Tromethamine | | | | 2,4 | 2,4 | 2,4 | 2,4 | 2,4 |
| Water | ad 100 | ad 100 | ad 100 | ad 100 | ad 100 | ad 100 | ad 100 | ad 100 |

Tabelle 3 (Fortsetzung)

| | 3-19 | 3-20 | 3-21 | 3-22 | 3-23 | 3-24 | 3-25 | 3-26 | 3-27 | 3-28 |
|---|---|---|---|---|---|---|---|---|---|---|
| 7,8,3',4'-Tetrahydroxyflavon | | | | 1 | 2 | | | | 1 | 1 |
| 5,6,7-Trihydroxyflavon | 1 | 3 | | 2 | | 5 | | 5 | 2 | |
| 6,3',4'-Trihydroxyflavon | 5 | 5 | 5 | 5 | 5 | 5 | 5 | 5 | 5 | 5 |
| UV-Pearl , OMC | 30 | 30 | 15 | 15 | 15 | 11 | 12 | 15 | 15 | 15 |
| Phenylbenzimidazole Sulfonic Acid | | 4 | 4 | | | | | | | |
| Sorbitol | 5 | 5 | 5 | 5 | 5 | 5 | 5 | 5 | 5 | 5 |
| Carbomer | 1,5 | 1,5 | 1,5 | 1,5 | 1,5 | 1,5 | 1,5 | 1,5 | 1,5 | 1,5 |
| Propylparabene | | | | | | | | | | |
| Methylparabene | 0,15 | 0,15 | 0,15 | 0,15 | 0,15 | 0,15 | 0,15 | 0,15 | 0,15 | 0,15 |
| Allantoin | 0,2 | 0,2 | 0,2 | 0,2 | 0,2 | 0,2 | 0,2 | 0,2 | 0,2 | 0,2 |
| Tromethamine | 2,4 | 4,2 | 4,2 | 2,4 | 2,4 | 2,4 | 2,4 | 2,4 | 2,4 | 2,4 |
| Water | ad 100 | ad 100 | ad 100 | ad 100 | ad 100 | ad 100 | ad 100 | ad 100 | ad 100 | ad 100 |

Tabelle 3 (Fortsetzung)

| | 3-29 | 3-30 | 3-31 | 3-32 | 3-33 | 3-34 | 3-35 | 3-36 |
|---|---|---|---|---|---|---|---|---|
| 4'-Methoxy-7-ß-glucosidflavon | | | | 1 | 2 | | | |
| 7,8,3',4'-Tetrahydroxyflavon | 1 | 3 | | 2 | | 5 | | 5 |
| 6,3',4'-Trihydroxyflavon | 5 | 5 | 5 | 5 | 5 | 5 | 5 | 5 |
| 5,6,7-Trihydroxyflavon | 1 | 5 | 4 | | 6 | | 7 | |
| UV-Pearl , OMC | 15 | 10 | | 10 | 10 | 10 | 15 | 10 |
| UV-Pearl , OCR | | | 10 | | | | | |
| UV-Pearl , OMC, Methylene Bis-Benztriazolyl Tetramethylbutylphenol | | 7 | | 6 | | | | |
| UV-Pearl, Ethylhexyl salicylate, BMDBM | | | 10 | | | | | |
| Disodium Phenyl Dibenzimidazole Tetrasulfonate | | 3 | | | | 3 | | 3 |
| Phenylbenzimidazole Sulfonic Acid | | 2 | | | 2 | 3 | | 3 |
| Prunus Dulcis | 5 | 5 | 5 | | | | | |
| Tocopheryl Acetate | 0,5 | 0,5 | 0,5 | | | | | |
| Caprylic/Capric Triglyceride | 3 | 3 | 3 | | | | | |
| Octyldodecanol | 2 | 2 | 2 | | | | | |
| Decyl Oleate | 2 | 2 | 2 | | | | | |
| PEG-8 (and) Tocopherol (and) Ascorbyl Palmitate (and) Ascorbic Acid (and) Citric Acid | 0,05 | 0,05 | 0,05 | | | | | |
| Sorbitol | 4 | 4 | 4 | 5 | 5 | 5 | 5 | 5 |
| Polyacrylamide (and) C13-14 Isoparaffin (and) Laureth-7 | 3 | 3 | 3 | | | | | |
| Carbomer | | | | 1,5 | 1,5 | 1,5 | 1,5 | 1,5 |
| Propylparabene | 0,05 | 0,05 | 0,05 | | | | | |
| Methylparabene | 0,15 | 0,15 | 0,15 | 0,15 | 0,15 | 0,15 | 0,15 | 0,15 |
| Allantoin | | | | 0,2 | 0,2 | 0,2 | 0,2 | 0,2 |
| Tromethamine | | | | 2,4 | 2,4 | 2,4 | 2,4 | 2,4 |
| Water | ad 100 | ad 100 | ad 100 | ad 100 | ad 100 | ad 100 | ad 100 | ad 100 |

**Patentansprüche**

**1.** Komplex-Verbindung der Formel I

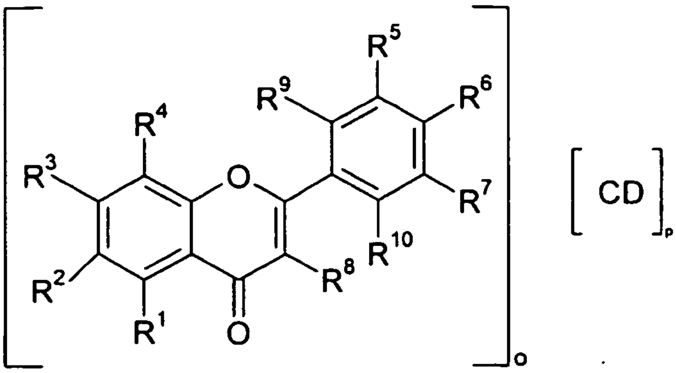

I

wobei $R^1$ bis $R^{10}$ gleich oder verschieden sein können und ausgewählt sind aus

- H
- $OR^{11}$
- geradkettigen oder verzweigten $C_1$- bis $C_{20}$-Alkylgruppen,
- geradkettigen oder verzweigten $C_3$- bis $C_{20}$-Alkenylgruppen,
- geradkettigen oder verzweigten $C_1$- bis $C_{20}$-Hydroxyalkylgruppen, wobei die Hydroxygruppe an ein primäres oder sekundäres Kohlenstoffatom der Kette gebunden sein kann und weiter die Alkylkette auch durch Sauerstoff unterbrochen sein kann, und/oder
- $C_3$- bis $C_{10}$-Cycloalkylgruppen und/oder $C_3$- bis $C_{12}$-Cycloalkenylgruppen, wobei die Ringe jeweils auch durch $-(CH_2)_n$-Gruppen mit n = 1 bis 3 überbrückt sein können,
- wobei alle $OR^{11}$ unabhängig voneinander stehen für

    - OH
    - geradkettige oder verzweigte $C_1$- bis $C_{20}$-Alkyloxygruppen,
    - geradkettigen oder verzweigten $C_3$- bis $C_{20}$-Alkenyloxygruppen,
    - geradkettigen oder verzweigten $C_1$- bis $C_{20}$-Hydroxyalkoxygruppen, wobei die Hydroxygruppe(n) an ein primäre oder sekundäre Kohlenstoffatome der Kette gebunden sein können und weiter die Alkylkette auch durch Sauerstoff unterbrochen sein kann, und/oder
    - $C_3$- bis $C_{10}$-Cycloalkyloxygruppen und/oder $C_3$- bis $C_{12}$-Cycloalkenyloxygruppen, wobei die Ringe jeweils auch durch $-(CH_2)_n$-Gruppen mit n = 1 bis 3 überbrückt sein können und/oder,
    - Mono- und/oder Oligoglycosylreste, wobei Mono- und Oligoglykosylreste für $R^8$ ausgeschlossen sind,

CD steht für ein Cyclodextrin-Molekül
o steht für die Zahl 1 und
p steht für eine Zahl aus dem Bereich 0,5 bis 50.

mit der Maßgabe, dass mindestens 2 Reste aus $R^1$ bis $R^7$ stehen für OH und dass im Molekül mindestens 1 Paar benachbarter Gruppen -OH vorliegt.

2. Verbindung der Formel I nach Anspruch 1, **dadurch gekennzeichnet, dass** mindestens 3 Reste aus $R^1$ bis $R^7$ stehen für OH und dass im Molekül mindestens 2 Paare benachbarter Gruppen -OH vorliegen, oder $R^2$, $R^5$ und $R^6$ für OH und die Reste $R^1$, $R^3$, $R^4$ und $R^{7-10}$ für H stehen.

3. Verbindung der Formel I nach mindestens einem der vorstehenden Ansprüche, **dadurch gekennzeichnet, dass** mindestens zwei benachbarte Reste der Reste $R^1$ bis $R^4$ stehen für OH und mindestens zwei benachbarte Reste der Reste $R^5$ bis $R^7$ stehen für OH.

4. Verbindung der Formel I nach mindestens einem der vorstehenden Ansprüche, **dadurch gekennzeichnet, dass** mindestens drei benachbarte Reste der Reste $R^1$ bis $R^4$ stehen für OH, wobei vorzugsweise die Reste $R^1$ bis $R^3$ für OH stehen.

5. Verbindung der Formel I nach mindestens einem der vorstehenden Ansprüche, **dadurch gekennzeichnet, dass** es sich bei dem Cyclodextrin CD um ein alpha-, beta-, oder gamma-Cyclodextrin, vorzugsweise ein gegebenenfalls

an ein oder mehreren Hydroxygruppen $C_{1-24}$-Alkyl- oder $C_{1-24}$-Hydroxyalky-substituiertes Cyclodextrin, insbesondere bevorzugt um ein Hydroxypropyl-cyclodextrin handelt.

6. Verfahren zur Herstellung von Komplex-Verbindungen nach mindestens einem der Ansprüche 1 bis 5, **dadurch gekennzeichnet, dass** Verbindungen gemäß Formel II

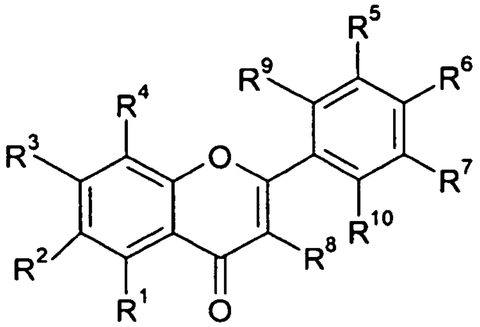

II

wobei $R^1$ bis $R^{10}$ gleich oder verschieden sein können und ausgewählt sind aus

- H
- $OR^{11}$
- geradkettigen oder verzweigten $C_1$- bis $C_{20}$-Alkylgruppen,
- geradkettigen oder verzweigten $C_3$- bis $C_{20}$-Alkenylgruppen,
- geradkettigen oder verzweigten $C_1$- bis $C_{20}$-Hydroxyalkylgruppen, wobei die Hydroxygruppe an ein primäres oder sekundäres Kohlenstoffatom der Kette gebunden sein kann und weiter die Alkylkette auch durch Sauerstoff unterbrochen sein kann, und/oder
- $C_3$- bis $C_{10}$-Cycloalkylgruppen und/oder $C_3$- bis $C_{12}$-Cycloalkenylgruppen, wobei die Ringe jeweils auch durch $-(CH_2)_n$-Gruppen mit n = 1 bis 3 überbrückt sein können,
- wobei alle $OR^{11}$ unabhängig voneinander stehen für

  - OH
  - geradkettige oder verzweigte $C_1$- bis $C_{20}$-Alkyloxygruppen,
  - geradkettigen oder verzweigten $C_3$- bis $C_{20}$-Alkenyloxygruppen,
  - geradkettigen oder verzweigten $C_1$- bis $C_{20}$-Hydroxyalkoxygruppen, wobei die Hydroxygruppe(n) an ein primäre oder sekundäre Kohlenstoffatome der Kette gebunden sein können und weiter die Alkylkette auch durch Sauerstoff unterbrochen sein kann, und/oder
  - $C_3$- bis $C_{10}$-Cycloalkyloxygruppen und/oder $C_3$- bis $C_{12}$-Cycloalkenyloxygruppen, wobei die Ringe jeweils auch durch $-(CH_2)_n$-Gruppen mit n = 1 bis 3 überbrückt sein können und/oder,
  - Mono- und/oder Oligoglycosylreste, wobei Mono- und Oligoglykosylreste für $R^3$ ausgeschlossen sind,

mit der Maßgabe, dass mindestens 2 Reste aus $R^1$ bis $R^7$ stehen für OH und dass im Molekül mindestens 1 Paar benachbarter Gruppen -OH vorliegt,
in Lösung mit Cyclodextrinen CD, vorzugsweise bei erhöhter Temperatur, umgesetzt werden.

7. Verfahren nach Anspruch 6, **dadurch gekennzeichnet, dass** das Cyclodextrin im Überschuss oder genau im Molverhältnis 1 : 1 oder 2 : 1 bezogen auf das Flavonoid der Formel II eingesetzt wird.

8. Zubereitung enthaltend einen geeigneten Träger, **dadurch gekennzeichnet, dass** die Zubereitung

- 0,005 bis 99 Gew.-% einer Komplex-Verbindung nach Formel I gemäß Anspruch 1 enthält oder die Zubereitung
- 0,002 bis 70 Gew.-% Cyclodextrin und
- 0,001 bis 60 Gew.-% mindestens einer Verbindung der Formel II gemäß Anspruch 6 oder ihrer topisch verträglicher Salze und/oder Derivate enthält.

9. Zubereitung nach Anspruch 8, **dadurch gekennzeichnet, dass** die eine oder die mehreren Verbindungen der Formel I in Mengen von 0,01 bis 20 Gew.%, vorzugsweise 0,05 bis 10 Gew.-% und insbesondere bevorzugt 0,1 bis 5 Gew.-% in der Zubereitung enthalten sind.

**10.** Zubereitung nach Anspruch 8, **dadurch gekennzeichnet, dass** der Gehalt an Cyclodextrinen in der Zubereitung 0,01-20,0 Gew.%, bevorzugt 0,05-10,0 Gew.%, besonders bevorzugt 0,1-5,0 Gew.%, jeweils bezogen auf das Gesamtgewicht der Zusammensetzung beträgt und der Gehalt an Verbindungen der Formel II in der Zubereitung von 0,01 bis 20 Gew.%, vorzugsweise 0,05 bis 10 Gew.% und insbesondere bevorzugt von 0,1 bis 5 Gew.% bezogen auf die gesamte Zubereitung, wobei der Anteil der Verbindungen der Formel II in der Zubereitung ganz außerordentlich bevorzugt im Bereich von 0,1 bis 2 Gew.% bezogen auf die gesamte Zubereitung liegt.

**11.** Zubereitung nach mindestens einem der vorhergehenden Ansprüche zum Schutz von Körperzellen gegen oxidativen Stress, insbesondere zur Verringerung der Hautalterung, **dadurch gekennzeichnet, dass** sie vorzugsweise ein oder mehrere weitere Antioxidantien und/oder Vitamine, vorzugsweise ausgewählt aus Vitamin-A-Palmitat, Vitamin C und dessen Derivaten, DL-$\alpha$-Tocopherol, Tocopherol-E-Acetat, Nicotinsäure, Pantothensäure und Biotin, enthält.

**12.** Zubereitung nach mindestens einem der vorhergehenden Ansprüche, wobei die Zubereitung neben der mindestens einen Verbindung nach Formel I einen oder mehrere UV-Filter enthält, die vorzugsweise ausgewählt sind aus der Gruppe, die 3-(4'-Methylbenzyliden)-dl-kampfer, 1-(4-tert-Butylphenyl)-3-(4-methoxyphenyl)propan-1,3-dion, 4-Isopropyldibenzoylmethan, 2-Hydroxy-4-methoxybenzophenon, Methoxyzimtsäureoctylester, 3,3,5-Trimethyl-cyclohexylsalicylat, 4-(Di-methylamino)benzoesäure2-ethylhexylester, 2-Cyano-3,3-diphenyl-acrylsäure-2-ethylhexylester, 2-Phenylbenzimidazol-5-sulfonsäure sowie ihre Kalium-, Natrium- und Triethanolaminsalze enthält.

**13.** Zubereitung nach mindestens einem der vorhergehenden Ansprüche, **dadurch gekennzeichnet, dass** die Zubereitung eine Nahrungsmittel oder ein Nahrungsergänzungsmittel ist und einen für Nahrungsmittel geeigneten Träger sowie gegebenenfalls weitere Nahrungsergänzungsstoffe enthält.

**14.** Verfahren zur Herstellung einer Zubereitung **dadurch gekennzeichnet, dass** eine Verbindung der Formel I mit Resten gemäß Anspruch 1 mit einem kosmetisch oder dermatologisch oder für Nahrungsmittel geeignetem Träger vermischt wird.

**15.** Verwendung einer Verbindung der Formel I, wobei die Variablen die in Anspruch 1 angegebene Bedeutung aufweisen, zur Herstellung einer Zubereitung mit antioxidanten Eigenschaften.

**16.** Verwendung einer Verbindung der Formel I, wobei die Variablen die in Anspruch 1 angegebene Bedeutung aufweisen, als Antioxidationsmittel mit langanhaltender Wirkung.

**17.** Verwendung einer Verbindung der Formel I, wobei die Variablen die in Anspruch 1 angegebene Bedeutung aufweisen, als Nahrungsmittelzusatz für die Human- oder Tierernährung.

## Claims

**1.** Complex compound of the formula I

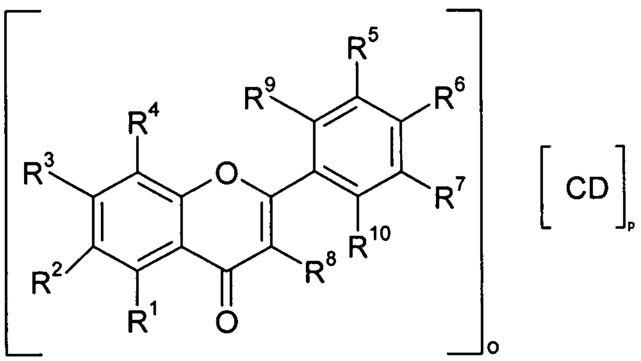

I

where $R^1$ to $R^{10}$ may be identical or different and are selected from

- H
- OR"

- straight-chain or branched $C_1$- to $C_{20}$-alkyl groups,
- straight-chain or branched $C_3$- to $C_{20}$-alkenyl groups,
- straight-chain or branched $C_1$- to $C_{20}$-hydroxyalkyl groups, where the hydroxyl group may be bonded to a primary or secondary carbon atom of the chain and furthermore the alkyl chain may also be interrupted by oxygen, and/or
- $C_3$- to $C_{10}$-cycloalkyl groups and/or $C_3$- to $C_{12}$-cycloalkenyl groups, where the rings may each also be bridged by $-(CH_2)_n-$ groups, where n = 1 to 3,
- where all $OR^{11}$, independently of one another, stand for

  - OH
  - straight-chain or branched $C_1$- to $C_{20}$-alkoxy groups,
  - straight-chain or branched $C_3$- to $C_{20}$-alkenyloxy groups,
  - straight-chain or branched $C_1$- to $C_{20}$-hydroxyalkoxy groups, where the hydroxyl group(s) may be bonded to a primary or secondary carbon atom of the chain and furthermore the alkyl chain may also be interrupted by oxygen, and/or
  - $C_3$- to $C_{10}$-cycloalkoxy groups and/or $C_3$- to $C_{12}$-cyclo-alkenyloxy groups, where the rings may each also be bridged by $-(CH_2)_n-$ groups, where n = 1 to 3, and/or
  - mono- and/or oligoglycosyl radicals, where mono- and oligoglycosyl radicals are excluded for $R^8$,

CD stands for a cyclodextrin molecule
o stands for the number 1 and
p stands for a number from the range 0.5 to 50,

with the proviso that at least 2 radicals from $R^1$ to $R^7$ stand for OH and that at least 1 pair of adjacent -OH groups is present in the molecule.

**2.** Compound of the formula I according to Claim 1, **characterised in that** at least 3 radicals from $R^1$ to $R^7$ stand for OH and **in that** at least 2 pairs of adjacent -OH groups are present in the molecule, or $R^2$, $R^5$ and $R^6$ stand for OH and the radicals $R^1$, $R^3$, $R^4$ and $R^{7-10}$ stand for H.

**3.** Compound of the formula I according to at least one of the preceding claims, **characterised in that** at least two adjacent radicals of the radicals $R^1$ to $R^4$ stand for OH and at least two adjacent radicals of the radicals $R^5$ to $R^7$ stand for OH.

**4.** Compound of the formula I according to at least one of the preceding claims, **characterised in that** at least three adjacent radicals of the radicals $R^1$ to $R^4$ stand for OH, where the radicals $R^1$ to $R^3$ preferably stand for OH.

**5.** Compound of the formula I according to at least one of the preceding claims, **characterised in that** the cyclodextrin CD is an alpha-, beta- or gamma-cyclodextrin, preferably a cyclodextrin which is optionally substituted by $C_{1-24}$-alkyl or $C_{1-24}$-hydroxyalkyl on one or more hydroxyl groups, particularly preferably a hydroxypropylcyclodextrin.

**6.** Process for the preparation of complex compounds according to at least one of Claims 1 to 5, **characterised in that** compounds of the formula II

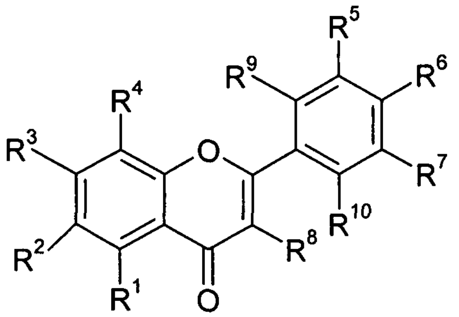

II

where $R^1$ to $R^{10}$ may be identical or different and are selected from

- H
- OR''
- straight-chain or branched $C_1$- to $C_{20}$-alkyl groups,
- straight-chain or branched $C_3$- to $C_{20}$-alkenyl groups,
- straight-chain or branched $C_1$- to $C_{20}$-hydroxyalkyl groups, where the hydroxyl group may be bonded to a primary or secondary carbon atom of the chain and furthermore the alkyl chain may also be interrupted by oxygen, and/or
- $C_3$- to $C_{10}$-cycloalkyl groups and/or $C_3$- to $C_{12}$-cycloalkenyl groups, where the rings may each also be bridged by $-(CH_2)_n-$ groups, where n = 1 to 3,
- where all $OR^{11}$, independently of one another, stand for

  - OH
  - straight-chain or branched $C_1$- to $C_{20}$-alkoxy groups,
  - straight-chain or branched $C_3$- to $C_{20}$-alkenyloxy groups,
  - straight-chain or branched $C_1$- to $C_{20}$-hydroxyalkoxy groups, where the hydroxyl group(s) may be bonded to a primary or secondary carbon atom of the chain and furthermore the alkyl chain may also be interrupted by oxygen, and/or
  - $C_3$- to $C_{10}$-cycloalkoxy groups and/or $C_3$- to $C_{12}$-cyclo-alkenyloxy groups, where the rings may each also be bridged by $-(CH_2)_n-$ groups, where n = 1 to 3, and/or
  - mono- and/or oligoglycosyl radicals, where mono- and oligoglycosyl radicals are excluded for $R^8$,

with the proviso that at least 2 radicals from $R^1$ to $R^7$ stand for OH and that at least 1 pair of adjacent -OH groups is present in the molecule,
are reacted with cyclodextrins CD in solution, preferably at elevated temperature.

7. Process according to Claim 6, **characterised in that** the cyclodextrin is employed in excess or precisely in the molar ratio 1 : 1 or 2 : 1 relative to the flavonoid of the formula II.

8. Composition comprising a suitable vehicle, **characterised in that** the composition comprises

   - 0.005 to 99% by weight of a complex compound of the formula I according to Claim 1 or the composition comprises
   - 0.002 to 70% by weight of cyclodextrin and
   - 0.001 to 60% by weight of at least one compound of the formula II according to Claim 6 or topically tolerated salts and/or derivatives thereof.

9. Composition according to Claim 8, **characterised in that** the one or more compounds of the formula I are present in the composition in amounts of 0.01 to 20% by weight, preferably 0.05 to 10% by weight and particularly preferably 0.1 to 5% by weight.

10. Composition according to Claim 8, **characterised in that** the content of cyclodextrins in the composition is 0.01-20.0% by weight, preferably 0.05-10.0% by weight, particularly preferably 0.1-5.0% by weight, in each case based on the total weight of the composition, and the content of compounds of the formula II in the composition is from 0.01 to 20% by weight, preferably 0.05 to 10% by weight and particularly preferably from 0.1 to 5% by weight, based on the composition as a whole, where the proportion of the compounds of the formula II in the composition is very extraordinarily preferably in the range from 0.1 to 2% by weight, based on the composition as a whole.

11. Composition according to at least one of the preceding claims for the protection of body cells against oxidative stress, in particular for reducing skin ageing, **characterised in that** it preferably comprises one or more further antioxidants and/or vitamins, preferably selected from vitamin A palmitate, vitamin C and derivatives thereof, DL-$\alpha$-tocopherol, tocopherol E acetate, nicotinic acid, pantothenic acid and biotin.

12. Composition according to at least one of the preceding claims, where the composition, besides the at least one compound of the formula I, comprises one or more UV filters, which are preferably selected from the group which comprises 3-(4'-methylbenzylidene)-dl-camphor, 1-(4-tert-butylphenyl)-3-(4-methoxyphenyl)propane-1,3-dione, 4-isopropyl-dibenzoylmethane, 2-hydroxy-4-methoxybenzophenone, octyl methoxycinnamate, 3,3,5-trimethylcyclohexyl salicylate, 2-ethylhexyl 4-(di-methylamino)benzoate, 2-ethylhexyl 2-cyano-3,3-diphenylacrylate, 2-phenylbenzimidazole-5-sulfonic acid and the potassium, sodium and triethanolamine salts thereof.

**13.** Composition according to at least one of the preceding claims, **characterised in that** the composition is a food or food supplement and comprises a vehicle which is suitable for foods and optionally further food supplements.

**14.** Process for the preparation of a composition, **characterised in that** a compound of the formula I containing radicals according to Claim 1 is mixed with a vehicle which is suitable cosmetically or dermatologically or for foods.

**15.** Use of a compound of the formula I, where the variables have the meaning indicated in Claim 1, for the preparation of a composition having antioxidant properties.

**16.** Use of a compound of the formula I, where the variables have the meaning indicated in Claim 1, as antioxidant having a long-lasting action.

**17.** Use of a compound of the formula I, where the variables have the meaning indicated in Claim 1, as food additive for human or animal nutrition.

**Revendications**

**1.** Composé complexe de la formule I

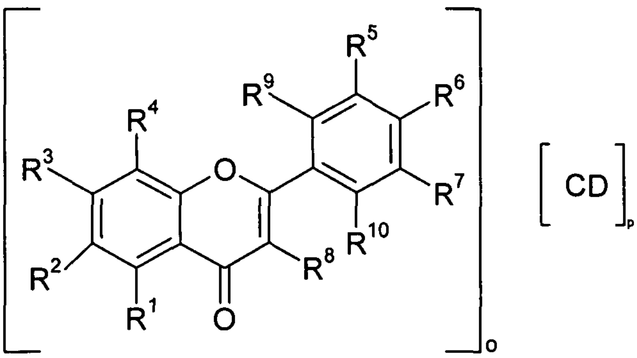

I

où $R^1$ à $R^{10}$ peuvent être identiques ou différents et sont choisis parmi

- H
- OR"
- des groupes $C_1$- à $C_{20}$-alkyle en chaîne droite ou ramifiés,
- des groupes $C_3$- à $C_{20}$-alkényle en chaîne droite ou ramifiés,
- des groupes $C_1$- à $C_{20}$-hydroxyalkyle en chaîne droite ou ramifiés, où le groupe hydroxyle peut être lié à un atome de carbone primaire ou secondaire de la chaîne et en outre, la chaîne alkyle peut également être interrompue par oxygène, et/ou
- des groupes $C_3$- à $C_{10}$-cycloalkyle et/ou des groupes $C_3$- à $C_{12}$-cycloalkényle, où les cycles peuvent également chacun être pontés par des groupes $-(CH_2)_n-$, où n = 1 à 3,
- où tous les $OR^{11}$, indépendamment les uns des autres, représentent

  - OH
  - des groupes $C_1$- à $C_{20}$-alcoxy en chaîne droite ou ramifiés,
  - des groupes $C_3$- à $C_{20}$-alkényloxy en chaîne droite ou ramifiés,
  - des groupes $C_1$- à $C_{20}$-hydroxyalcoxy en chaîne droite ou ramifiés, où le ou les groupe(s) hydroxyle peut/peuvent être lié(s) à un atome de carbone primaire ou secondaire de la chaîne et en outre, la chaîne alkyle peut également être interrompue par oxygène, et/ou
  - des groupes $C_3$- à $C_{10}$-cycloalcoxy et/ou des groupes $C_3$-à $C_{12}$-cycloalkényloxy, où les cycles peuvent également chacun être pontés par des groupes $-(CH_2)_n-$, où n = 1 à 3, et/ou
  - des radicaux mono- et/ou oligoglycosyle, où des radicaux mono- et oligoglycosyle sont exclus pour $R^8$,

CD représente une molécule de cyclodextrine,
o représente le nombre 1 et

p représente un nombre dans la plage de 0,5 à 50,

étant entendu qu'au moins 2 radicaux pris parmi $R^1$ à $R^7$ représentent OH et qu'au moins 1 paire de groupes -OH adjacents est présente dans la molécule.

2. Composé de la formule I selon la revendication 1, **caractérisé en ce qu'**au moins 3 radicaux pris parmi $R^1$ à $R^7$ représentent OH et **en ce qu'**au moins 2 paires de groupes -OH adjacents sont présentes dans la molécule, ou $R^2$, $R^5$ et $R^6$ représentent OH et les radicaux $R^1$, $R^3$, $R^4$ et $R^{7-10}$ représentent H.

3. Composé de la formule I selon au moins l'une des revendications précédentes, **caractérisé en ce qu'**au moins deux radicaux adjacents des radicaux $R^1$ à $R^4$ représentent OH et au moins deux radicaux adjacents des radicaux $R^5$ à $R^7$ représentent OH.

4. Composé de la formule I selon au moins l'une des revendications précédentes, **caractérisé en ce qu'**au moins trois radicaux adjacents des radicaux $R^1$ à $R^4$ représentent OH, où les radicaux $R^1$ à $R^3$ représentent de préférence OH.

5. Composé de la formule I selon au moins l'une des revendications précédentes, **caractérisé en ce que** la cyclodextrine CD est une alpha-, beta- ou gamma-cyclodextrine, de préférence une cyclodextrine qui est en option substituée par $C_{1-24}$-alkyle ou $C_{1-24}$-hydroxy-alkyle sur un ou plusieurs groupes hydroxyle, particulièrement de préférence une hydroxypropylcyclodextrine.

6. Procédé pour la préparation de composés complexes selon au moins l'une des revendications 1 à 5, **caractérisé en ce que** des composés de la formule II

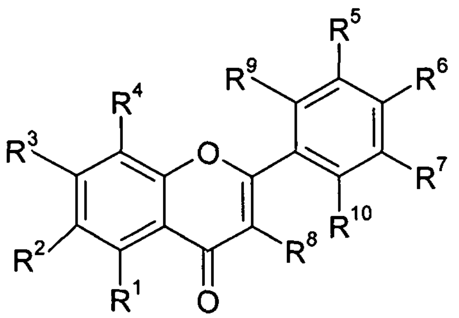

II

où $R^1$ à $R^{10}$ peuvent être identiques ou différents et sont choisis parmi

- H
- OR"
- des groupes $C_1$- à $C_{20}$-alkyle en chaîne droite ou ramifiés,
- des groupes $C_3$- à $C_{20}$-alkényle en chaîne droite ou ramifiés,
- des groupes $C_1$- à $C_{20}$-hydroxyalkyle en chaîne droite ou ramifiés, où le groupe hydroxyle peut être lié à un atome de carbone primaire ou secondaire de la chaîne et en outre, la chaîne alkyle peut également être interrompue par oxygène, et/ou
- des groupes $C_3$- à $C_{10}$-cycloalkyle et/ou des groupes $C_3$- à $C_{12}$-cycloalkényle, où les cycles peuvent également chacun être pontés par des groupes $-(CH_2)_n-$, où n = 1 à 3,
- où tous les $OR^{11}$, indépendamment les uns des autres, représentent

  - OH
  - des groupes $C_1$- à $C_{20}$-alcoxy en chaîne droite ou ramifiés,
  - des groupes $C_3$- à $C_{20}$-alkényloxy en chaîne droite ou ramifiés,
  - des groupes $C_1$- à $C_{20}$-hydroxyalcoxy en chaîne droite ou ramifiés, où le ou les groupe(s) hydroxyle peut/peuvent être lié(s) à un atome de carbone primaire ou secondaire de la chaîne et en outre, la chaîne alkyle peut également être interrompue par oxygène, et/ou
  - des groupes $C_3$- à $C_{10}$-cycloalcoxy et/ou des groupes $C_3$-à $C_{12}$-cycloalkényloxy, où les cycles peuvent également chacun être pontés par des groupes $-(CH_2)_n-$, où n = 1 à 3, et/ou

- des radicaux mono- et/ou oligoglycosyle, où des radicaux mono- et oligoglycosyle sont exclus pour $R^8$,

étant entendu qu'au moins 2 radicaux parmi $R^1$ à $R^7$ représentent OH et qu'au moins 1 paire de groupes -OH adjacents est présente dans la molécule,
sont amenés à réagir avec des cyclodextrines CD en solution, de préférence à température élevée.

**7.** Procédé selon la revendication 6, **caractérisé en ce que** la cyclodextrine est utilisée en excès ou précisément selon le rapport molaire de 1 : 1 ou de 2 : 1 par rapport au flavonoïde de la formule II.

**8.** Composition comprenant un vecteur approprié, **caractérisée en ce que** la composition comprend :

- de 0,005 % à 99 % en poids d'un composé complexe de la formule I selon la revendication 1 ou la composition comprend :
- de 0,002 % à 70 % en poids de cyclodextrine et
- de 0,001 % à 60% en poids d'au moins un composé de la formule II selon la revendication 6 ou de sels tolérés topiquement et/ou de leurs dérivés.

**9.** Composition selon la revendication 8, **caractérisée en ce que** les un ou plusieurs composés de la formule I sont présents dans la composition selon des quantités de 0,01 % à 20 % en poids, de préférence de 0,05 % à 10 % en poids et de façon particulièrement préférable, de 0,1 % à 5 % en poids.

**10.** Composition selon la revendication 8, **caractérisée en ce que** la teneur en cyclodextrines dans la composition est de 0,01-20,0 % en poids, de préférence de 0,05-10,0 % en poids et de façon particulièrement préférable de 0,1-5,0 % en poids, dans chaque cas sur la base du poids total de la composition, et la teneur en composés de la formule II dans la composition est de 0,01 % à 20 % en poids, de préférence de 0,05 % à 10 % en poids et de façon particulièrement préférable, de 0,1 % à 5 % en poids, sur la base de la composition prise dans sa globalité, où la proportion des composés de la formule II dans la composition est de façon très extraordinairement préférable dans la plage de 0,1 % à 2 % en poids, sur la base de la composition prise dans sa globalité.

**11.** Composition selon au moins l'une des revendications précédentes pour la protection de cellules du corps contre le stress oxydatif, en particulier pour réduire le vieillissement de la peau, **caractérisée en ce qu'**elle comprend de préférence un ou plusieurs autres antioxydants et/ou vitamines, de préférence choisis parmi le palmitate de vitamine A, la vitamine C et ses dérivés, le DL-$\alpha$-tocophérol, l'acétate de tocophérol E, l'acide nicotinique, l'acide pantothénique et la biotine.

**12.** Composition selon au moins l'une des revendications précédentes, où la composition, en plus de l'au moins un composé de la formule I, comprend un ou plusieurs filtres UV, lesquels sont de préférence choisis parmi le groupe qui comprend 3-(4'-méthylbenzylidène)-di-camphre, 1-(4-tert-butylphényl)-3-(4-méthoxyphényl)propane-1,3-dione, 4-isopropyldibenzoylméthane, 2-hydroxy-4-méthoxybenzophénone, octylméthoxycinnamate, 3,3,5-triméthylcyclohexylsalicylate, 2-éthylhexyl4-(diméthylamino)benzoate, 2-éthylhexyl2-cyano-3,3-diphénylacrylate, acide 2-phénylbenzimidazole-5-sulfonique et leurs sels de potassium, de sodium et de triéthanolamine.

**13.** Composition selon au moins l'une des revendications précédentes, **caractérisée en ce que** la composition est un aliment ou un complément alimentaire et comprend un vecteur qui convient pour les aliments et en option d'autres compléments alimentaires.

**14.** Procédé pour la préparation d'une composition, **caractérisé en ce qu'**un composé de la formule I contenant des radicaux selon la revendication 1 est mélangé avec un vecteur qui convient cosmétiquement ou dermatologiquement ou pour des aliments.

**15.** Utilisation d'un composé de la formule I, où les variables présentent la signification indiquée selon la revendication 1, pour la préparation d'une composition présentant des propriétés antioxydantes.

**16.** Utilisation d'un composé de la formule I, où les variables présentent la signification indiquée selon la revendication 1, en tant qu'antioxydant présentant une action de long terme.

**17.** Utilisation d'un composé de la formule I, où les variables présentent la signification indiquée selon la revendication 1, en tant qu'additif alimentaire pour la nutrition des êtres humains ou des animaux.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 19755504 A1
- WO 0200214 A
- WO 0061095 A
- EP 1400579 A
- DE 10162844 A1
- DE 10162842 A1
- DE 10162841 A1
- DE 10162840 A1
- DE 10048683 A1
- WO 2004030641 A
- EP 796624 A
- JP 059137499 B
- JP 7075536 A
- JP 5186344 A
- DE 10232595
- US 6242099 B1
- WO 0009652 A
- WO 0072806 A
- WO 0071084 A
- EP 0671161 A
- DE 4116123 A
- DE OS4308282 A
- EP 1400579 A2
- WO 2002060889 A1


### In der Beschreibung aufgeführte Nicht-Patentliteratur


- Laut CD Römpp Chemie Lexikon - Version 1.0. Georg Thieme Verlag, 1995
- **K. Lemanska ; H. Szymusiak ; B. Tyrakowska ; R. Zielinski.** I.M.C.M. Rietjens. *Current Topics in Biophysics,* 2000, vol. 24 (2), 101-108
- **C.A. Rice-Evans ; N.J. Miller ; G. Paganga.** *Trends in Plant Science,* 1997, vol. 2 (4), 152-159
- **K. Lemanska ; H. Szymusiak ; B. Tyrakowska ; R. Zielinski.** A.E.M.F. Soffers, I.M.C.M. Rietjens. *Free Radical Biology&Medicine,* 2001, vol. 31 (7), 869-881
- **K. Miyake ; H. Arima et al.** *Pharm. Dev. Techn.,* 2000, vol. 5 (3), 399-407
- **T. K. Nguyen ; H. Galons ; C. Chemtob.** *Congr. Int. Technol. Pharm., 6th,* 1992, vol. 5, 408-16408416
- **S. Dimova ; R. Mugabowindekwe et al.** *Int. J. Pharm.,* 2003, vol. 2638 (1-2), 95-103
- **R. Ficarra ; S. Tommasini et al.** *J. Pharm. Biomed. Analysis,* 2002, vol. 29 (6), 1005-1014
- **R.-L. Wang ; Yu Yang et al.** *Yingyong Huaxue,* 2002, vol. 19 (7), 702-704
- **K. Hostettmann ; M. Lederer ; A. Marston.** *Phytochemical Analysis,* 2000, vol. 1186, 380-382
- **C. Sanchez-Moreno ; J.A. Larrauri ; F. Saura-Calixto.** *J. Sci. Food Agric.,* 1998, vol. 76 (2), 270-276
- **E. A. Galinski et al.** *Eur. J. Biochem.,* 1985, vol. 149, 135-139
- Rowe Colour Index. Society of Dyers and Colourists, 1971